# EUROPEAN PATENT APPLICATION

(11) **EP 3 056 202 A1**
(43) Date of publication of application: **17.08.2016**
(21) Application number: 15155298.1
(22) Date of filing: 16.02.2015
(51) Int. Cl.: A61K 31/404, A61K 31/4178, A61K 31/439, A61K 31/4439, A61K 31/454, A61K 31/496, A61K 31/5377, C07D 453/00, C07D 209/20, A61P 35/00, A61P 31/12, C07D 403/12, C07D 401/14, C07D 401/06, C07D 401/12, C07D 403/06, C07D 405/06, C07D 409/06

(54) **BENZOPYRROLIDONE DERIVATIVES POSSESSING ANTIVIRAL AND ANTICANCER PROPERTIES**

(71) Applicant: Max-Planck-Gesellschaft zur Förderung der Wissenschaften e.V., 80539 München (DE); Vichem Chemie Ltd., 1022 Budapest (HU)
(72) Inventor: Kéri, György, 1021 Budapest (HU); Örfi, László, 1161 Budapest (HU); Horváth, Zoltán, 1035 Budapest (HU); Szokol, Bálint, Budapest 1022 (HU); Dobos, Judit, 1036 Budapest (HU); Nemes, Zoltán, 1089 Budapest (HU); Szàntay Kis, Csaba, 1094 Budapest (HU); Erös, Dánilel, 1146 Budapest (HU); Breza, Nóra, 1039 Budapest (HU); Baska, Ferenc, 1064 Budapest (HU); Karlas, Alexander, 10245 Berlin (DE); Gödert, Sigrid, 14612 Falkensee (DE); Meyer, Thomas F., 14612 Falkensee (DE)
(74) Representative: Arth, Hans-Lothar

(57) **Abstract**

The present invention relates to benzopyrrolidone derivatives and/or pharmaceutically acceptable salts thereof for the use in the treatment of infectious diseases or cancer, and pharmaceutical compositions containing at least one of said benzopyrrolidone derivatives and/or pharmaceutically acceptable salts thereof for the use in the treatment of infectious diseases or cancer.

SEQUENCE LISTING

<110>  Vichem Chemie Ltd. and Max-Planck-Gesellschaft zur
       Förderung der Wissenschaften e.V.

<120>  Biologically active heterocyclic compounds possessing antiviral
       and anticancer properties

<130>  VIC-P03759EP

<160>  2

<170>  PatentIn version 3.5

<210>  1
<211>  9
<212>  PRT
<213>  Artificial Sequence

<220>
<223>  substrate peptide Akt1activity assay


<220>
<221>  MISC_FEATURE
<222>  (1)..()
<223>  TAMRA

<400>  1

Gly Arg Thr Gly Arg Arg Asn Ser Ile
1               5


<210>  2
<211>  14
<212>  PRT
<213>  Artificial Sequence

<220>
<223>  substrate peptide for Clk1 activity assay


<220>
<221>  MISC_FEATURE
<222>  (1)..()
<223>  TAMRA

<400>  2

Ala Phe Arg Arg Glu Trp Ser Pro Gly Lys Glu Ala Lys Lys
1               5                   10

## Description

### Specification

The present invention relates to benzopyrrolidone derivatives and/or pharmaceutically acceptable salts thereof for use in the treatment of infection diseases, and pharmaceutical composition containing at least one of the said benzopyrrolidone derivatives and/or pharmaceutically acceptable salt thereof.

### Background of the invention

### Medical need for novel innovative drugs for influenza treatment

Despite vaccination and currently available antiviral drugs, influenza infections still make a huge impact on human health worldwide. In light of the risk posed by seasonal infections and also the recurring threat of influenza virus pandemics, there is an acute need to develop effective and lasting drugs. In Germany alone, the seasonal occurrence of influenza causes between 5,000 and 20,000 deaths per year (source: Robert Koch Institute). Worldwide, up to 500,000 humans die annually due to seasonal influenza or accompanying opportunistic infections (source: WHO). The recurring big influenza pandemics, however, present the greatest threat. The first great pandemic, the so-called "Spanish Flu", cost 40 million lives in the years 1918-1920, including a high percentage of healthy, middle-aged people. A similarly devastating pandemic could still be initiated by the recently arisen avian H5N1 influenza virus, if acquired mutations enable the virus to be transmitted from human-to-human. With mortality rates of approximately 60%, the H5N1 virus represents an unprecedented risk to human health. While the threat of a H5N1 pandemic still exists, the world is currently in the throes of yet another novel influenza strain, the pandemic (H1N1) 2009 virus. Although highly transmissible, the H1 N1 virus appears, currently, to be less hazardous, as mortality rates are still low (less than ∼1%) and comparable to seasonal epidemics. However, uncertainty exists about a possible acquisition of increased pathogenicity during its unavoidable adaptation process. Historical data from 1918 are giving special cause for concern: the first infections with the H1 N1 "Spanish Flu" virus in March 1918 led to relatively mild symptoms compared to the deadly second wave of infections that spread worldwide several months later.

The current panel of preventive and therapeutic measures against influenza virus infections rests on (i) active vaccination and (ii) the use of conventional anti-viral drugs. Both strategies have their intrinsic limitations owing to the high variability of influenza viruses. The genome of influenza viruses consists of eight genome segments that can be quickly re-assorted upon viral co-infections, a process called antigenic shift. If such viral recombinants are derived from animal reservoirs they may exhibit surface antigens that are unknown to the human immune system. Moreover, virus replication supports the generation of point mutations, giving rise to a steady 'antigenic drift' and viral escape. These two mechanisms of influenza virus variation necessitate the generation of new anti-influenza vaccines every yearly season. However, vaccine development is inherently afflicted with uncertainty about the emerging strain because the viral antigen has to be decided upon months before the virus is established in the human population. Similarly, in the case of a pandemic threat, as the new virus has to be isolated and amplified prior to vaccine generation, a lag time of several months is unavoidable. Since pandemic strains have the potential to spread rapidly across the globe, vaccination strategies necessitate the concurrent development and availability of effective complementary options to treat already infected individuals.

The fact that treatment (and immediate prophylaxis) of influenza virus infections is hampered by the occurrence of viral escape mutants is not just theoretical, but has been impressively demonstrated with the growing emergence of, e.g. Tamiflu®, resistant virus mutants of seasonal influenza strains as well as the new H5N1 and H1 N1 strains. Considering that, in the case of a severe pandemic threat, these conventional drugs would be given to millions of people; a global manifestation of drug resistance will be likely.

Existing anti-viral drugs are typically directed against *bona fide* viral targets, so-called direct antiviral targets, bearing the usual risk of generating therapeutic resistance. However, a largely ignored fact is that viral infection and replication intimately depend on the cellular factors provided by the host. Blocking human functions to prevent infection may appear - at a first glance - counter-intuitive. However, this very approach is taken on a regular and sometimes long-lasting basis to successfully treat thousands of other human diseases. Application of this widely used approach should thus also be feasible to treat viral infection, but this approach has not been systematically developed.

Recently, suitable human genes have been identified in the process of a genome-wide RNAi screen for human factors essential for influenza virus replication *in vitro (*Karlas et al., Nature, 2010*).* This high-throughput analysis revealed hundreds of human factors involved in the replication of the influenza virus. Subsequent validation yielded a panel of targets including many kinases (e.g. Clk1, Akt1 and Ack1; Karlas et al., Nature, 2010)) that fulfilled crucial *in vitro* requirements with respect to safety (no toxicity of RNAi knock-down) and efficacy (substantially diminished viral replication). These validated host cell targets were the basis for the subsequent drug discovery project.

### Anticancer application

Colorectal cancer is the third most commonly diagnosed cancer and the third leading cause of cancer death in both gender. Localized tumors may be curable with surgery, but those that have spread widely are usually not curable. Therefore, there is a need for more effective treatment regimes for advanced and metastatic disease. Besides cytotoxic chemotherapy patients may take advantage of so far limited targeted therapies (e. g. anti-EGFR therapy).

Many kinases have been found to be involved in the processes leading to tumor cell proliferation and survival. Benzopyrrolidone derivatives are well known kinase inhibitors (e.g. VEGFR, FGFR, PDGFR, c-KIT, FLT3, Met, Src and Aurora B). Several indolin-2-ones have been under preclinical or clinical study for cancer treatment. Benzopyrrolidone derivatives like sunitinib, nintedanib and toceranib have already been used as anticancer and anti-angiogenic agents.

### Brief description of the invention

It is object of the present invention to provide compounds and/or pharmaceutically acceptable salts thereof, which can be used as pharmaceutically active agents, especially for treatment of infection diseases, as well as compositions comprising at least one of these compounds and/or pharmaceutically acceptable salts thereof as pharmaceutically active ingredients.

This object is solved by providing compounds and/or their pharmaceutically acceptable salts according to the general formula (I) for use in the treatment of infectious diseases and pharmaceutical compositions comprising at least one of said compounds. Therefore the present invention relates to benzopyrrolidone derivatives of the general formula (I) for use in the treatment of infectious diseases, preferably in the treatment of viral infection such as influenza infection.

The objective of the present invention is solved by the teaching of the independent claims. Further advantageous features, aspects and details of the invention are evident from the dependent claims, the description, the figures, and the examples of the present application.

### Description of the invention

Thus, the present invention relates to compounds of general formula (I) 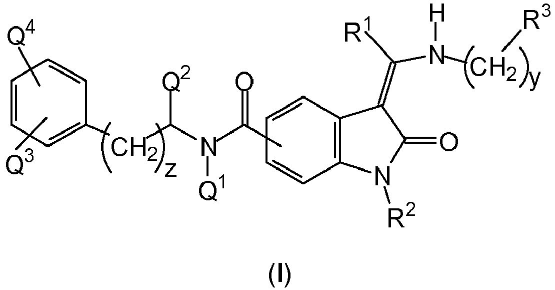 wherein,
y is an integer number selected from: 0, 1, and 2;
z is an integer number selected from: 0, 1, and 2;
Q¹ represents: -H or -CH₃;
Q² represents: -H, -CH₃ or -CH₂CH₃;
Q³ and Q⁴ are independently of each other selected from: -H, -F, -Cl, -CH₃, and -OCH₃;
R¹ represents: -CH₂CH₃, -CH(CH₃)₂, 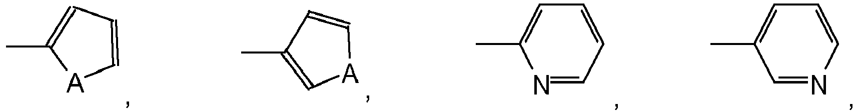 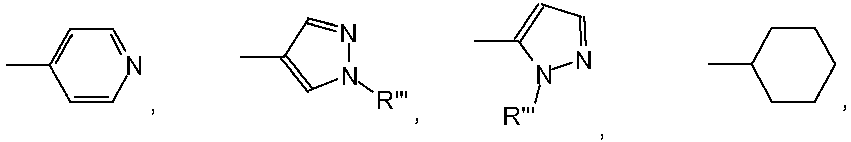 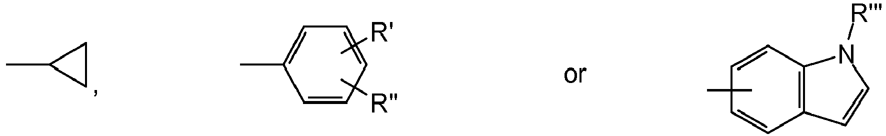
R' and R" are independently of each other selected from: -H, -F, -CH₃ and -OCH₃;
R"' represents: -H or -CH₃;
A represents -NH-, -O- or -S-;
R² represents: -H, -CH₃ or -C(=O)CH₃;
R³ represents: 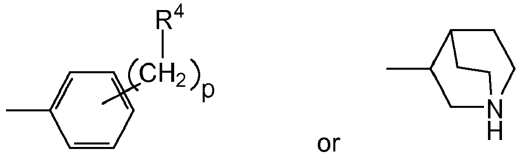
R⁴ represents: -R¹², -NR⁷R⁸, 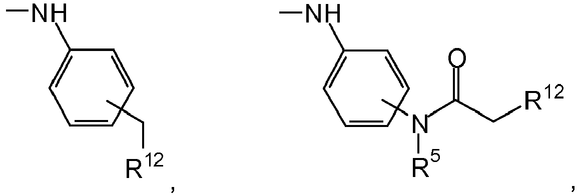 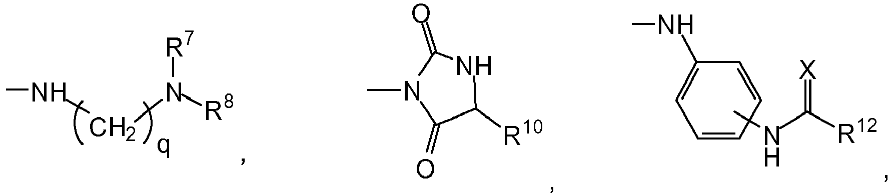 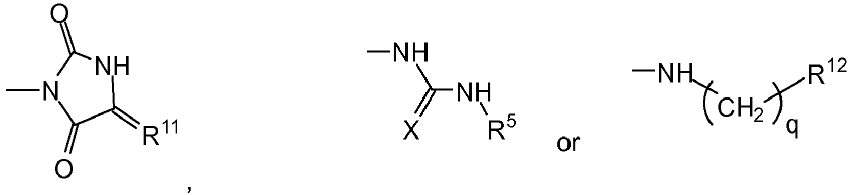
X represents: =O, =S;
R⁵ represents: -R⁶, 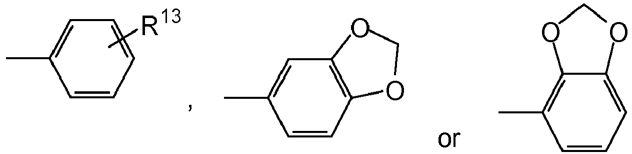
R⁶, R⁷,^{,} R⁸, R⁹ and R¹⁰ are independently of each other selected form : -H, -CH₃, -C₂H₅, -C₃H₇, -CH(CH₃)₂, -C(CH₃)₃-(CH₂)ₙ-CH₃, -(CH₂)ₙ-C(CH₃)₂, -CH[(CH₂)ₙCH₃]-(CH₂)ₘ-CH₃, -CH[(CH₂)ₙCH₃]-(CH₂)ₘ-C(CH₃)₂, -CH=CH₂, -(CH₂)ₙ-CH=CH-(CH₂)ₖ-CH₃, -CH≡CH, -(CH₂)ₙ-C≡C-(CH₂)ₖ-CH₃, -Ph, -(CH₂)ₙ-Ph, -(CH₂)ₙ-OH, -(CH₂)ₙ-NH₂, -(CH₂)ₙ-O-(CH₂)ₖ-CH₃, -(CH₂)ₙ-NH(CH₂)ₖ-CH₃, -(CH₂)ₙ-N(CH₃)₂, -(CH₂)ₙ-N(C₂H₅)₂ and -(CH₂)ₙ-N(CH₃)(C₂H₅);
R¹³ represents: -OH, -Cl, 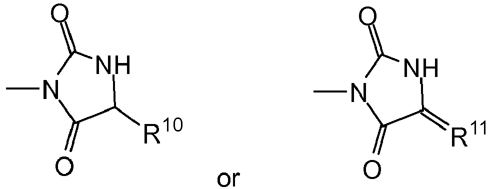
R¹¹ represents: =CH₂, =C(CH₃)₂, 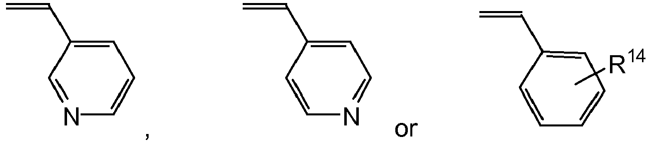
R¹⁴ represents: -H, -F, -Cl, -Br, -I, -OH or -OCH₃;
R¹² represents: 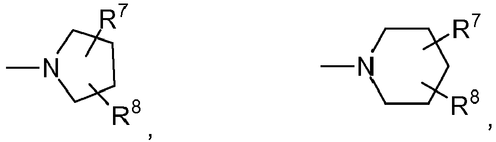 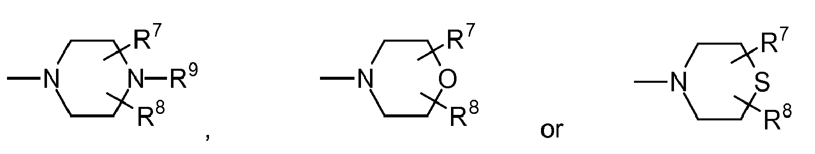
k and I are integer numbers and independently of each other selected from: 0, 1, 2, 3, 4 and 5;
m, n, and q are integer numbers and independently of each other selected from: 1, 2, 3, 4 and 5;
p is an integer number selected from: 0 and 1
and stereoisomeric forms, E/Z isomers, enantiomers, mixtures of enantiomers, anomers, deoxyforms, diastereomers, mixtures of diastereomers, prodrugs, tautomers, hydrates, solvates and racemates of the above mentioned compounds and pharmaceutically acceptable salts thereof, for the use in the treatment of infectious diseases and cancer.

The term 'prodrug' describes a precursor of the active ingredient containing a compound according to general formula (I) and further comprises groups which can be cleaved under physiological conditions or releases a compound according to general formula (I) under physiological conditions.

The expression 'tautomer' is defined as an organic compound that is interconvertible by a chemical reaction called tautomerization. Tautomerization can be catalyzed preferably by bases or acids or other suitable compounds. Compound according to claim 1, wherein z represents 0 and Q¹ is: -H.

The compounds of general formula (I) are basic and form salts with various organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, D-o-tolyltartaric acid, tartronic acid, a-toluic acid, (o, m, p)-toluic acid, naphthylamine sulfonic acid, amino acids such as glycone, alanine, valine, leucine, isoleucine, serine, threonine, phenylalanine, tyrosine, tryptophane, lysine, arginine, histidine, asparaginic acid, glutamic acid, asparagines, glutamine, cysteine, methionine, proline, 4-hydroxyproline, N,N,N-trimethyllysine, 3-methylhistidine, 5-hydroxylysine, O-phosphoserine, g-carboxyglutamate, e-N-acetyllysine, w-N-methylarginine, citrulline and ornithine, and other mineral or carboxylic acids well known to those skilled in the art. The salts are prepared by contacting the free base form with a sufficient amount of the desired acid to produce a salt in the conventional manner.

The free base forms may be regenerated by treating the salt with a suitable dilute aqueous base solution such as dilute aqueous sodium hydroxide, potassium carbonate, ammonia and sodium bicarbonate. The free base forms differ from their corresponding salt forms somewhat in certain physical properties, such as solubility in polar solvents, but the salts are otherwise equivalent to their corresponding free base forms for purposes of this invention.

A preferred embodiment of this invention is directed to compounds of general formula **(II)** 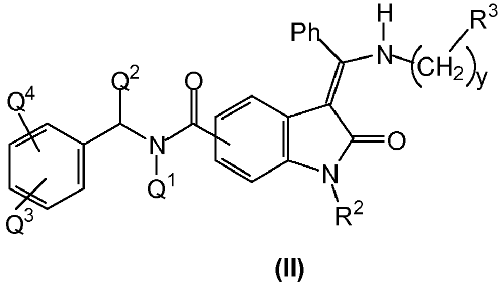 wherein the residues R², R³, Q¹ - Q⁴, and y have the meanings as defined herein. The amide moiety is preferably bound at the 5-position of the indol backbone.

A preferred embodiment of this invention is directed to compounds of general formula **(III)** 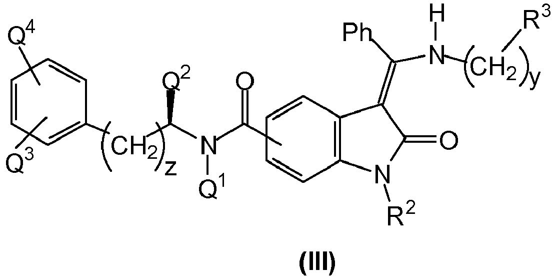 wherein the residues R², R³, Q¹ - Q⁴, y and z have the meanings as defined herein. The amide moiety is preferably bound at the 5-position of the indol backbone.

A preferred embodiment of this invention is directed to compounds of general formula **(IV)** 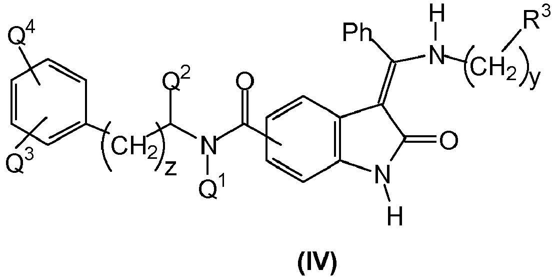 wherein the residues R³, Q¹ - Q⁴, y and z have the meanings as defined herein. The amide moiety preferably is bound at the 5-position of the indol backbone.

A preferred embodiment of this invention is directed to compounds of general formula **(V)** 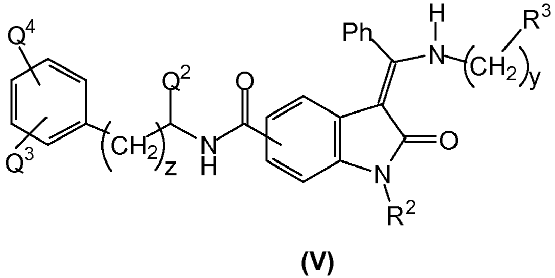 wherein the residues R², R³, Q² - Q⁴, y and z have the meanings as defined herein. The amide moiety is preferably bound at the 5-position of the indol backbone.

A preferred embodiment of this invention is directed to compounds of general formula **(VI)** 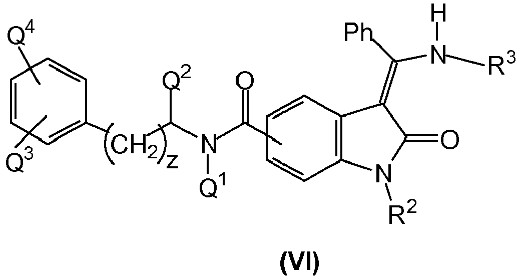 wherein the residues R², R³, Q¹ - Q⁴, and z have the meanings as defined herein. The amide moiety is preferably bound at the 5-position of the indol backbone.

A preferred embodiment of this invention is directed to compounds of general formula **(VII)** 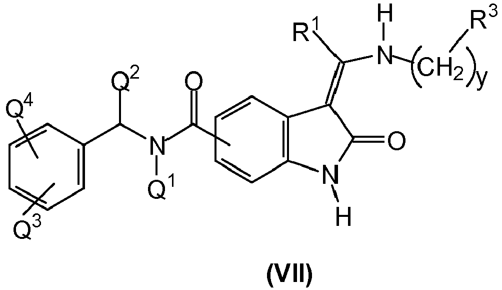 wherein the residues R¹, R³, Q¹ - Q⁴, and y have the meanings as defined herein. The amide moiety is preferably bound at the 5-position of the indol backbone.

A preferred embodiment of this invention is directed to compounds of general formula **(VIII)** 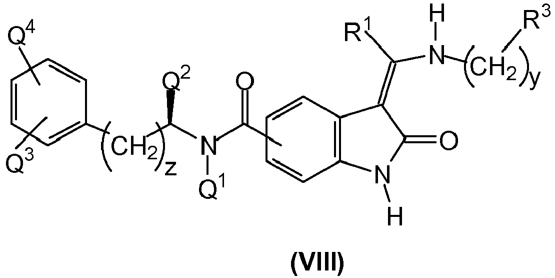 wherein the residues R¹, R³, Q¹ - Q⁴, y and z have the meanings as defined herein. The amide moiety is preferably bound at the 5-position of the indol backbone.

A preferred embodiment of this invention is directed to compounds of general formula **(IX)** 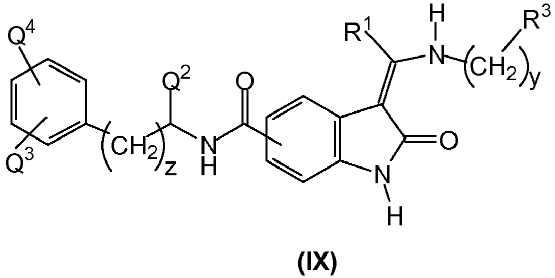 wherein the residues R¹, R³, Q² - Q⁴, y and z have the meanings as defined herein. The amide moiety is preferably bound at the 5-position of the indol backbone.

A preferred embodiment of this invention is directed to compounds of general formula **(X)** 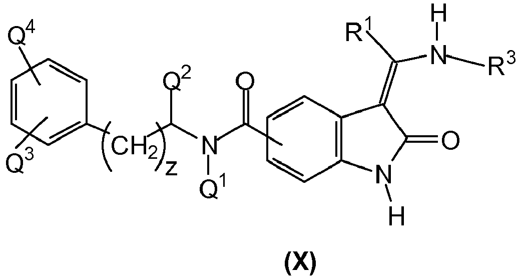 wherein the residues R¹, R³, Q¹ - Q⁴, and z have the meanings as defined herein. The amide moiety is preferably bound at the 5-position of the indol backbone.

A preferred embodiment of this invention is directed to compounds of general formula **(XI)** 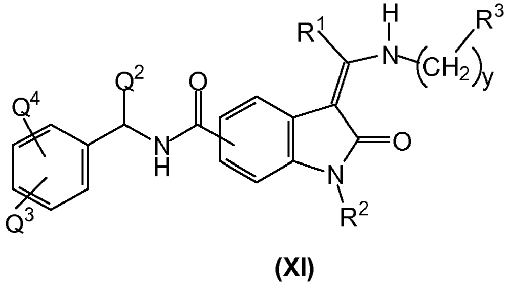 wherein the residues R¹, R³, Q² - Q⁴, and y have the meanings as defined herein. The amide moiety is preferably bound at the 5-position of the indol backbone.

A preferred embodiment of this invention is directed to compounds of general formula **(XII)** 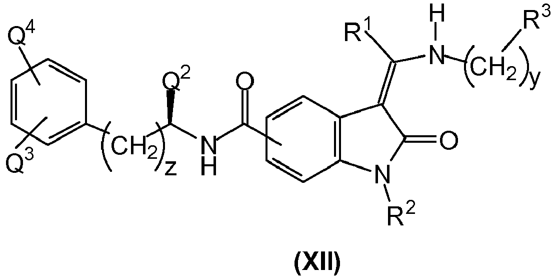 wherein the residues R¹ - R³, Q² - Q⁴, y and z have the meanings as defined herein. The amide moiety is preferably bound at the 5-position of the indol backbone.

A preferred embodiment of this invention is directed to compounds of general formula **(XIII)** 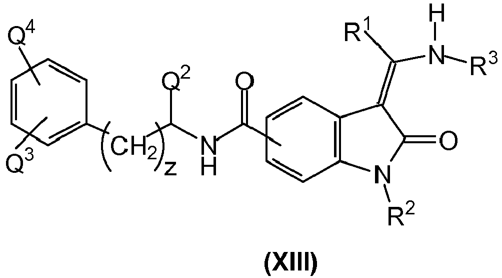 wherein the residues R¹ - R³, Q² - Q⁴, and z have the meanings as defined herein. The amide moiety is preferably bound at the 5-position of the indol backbone.

A preferred embodiment of this invention is directed to compounds of general formula **(XIV)** 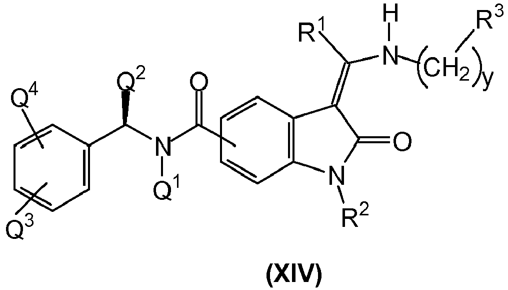 wherein the residues R¹ - R³, Q¹ - Q⁴, and y have the meanings as defined herein. The amide moiety is preferably bound at the 5-position of the indol backbone.

A preferred embodiment of this invention is directed to compounds of general formula **(XV)** 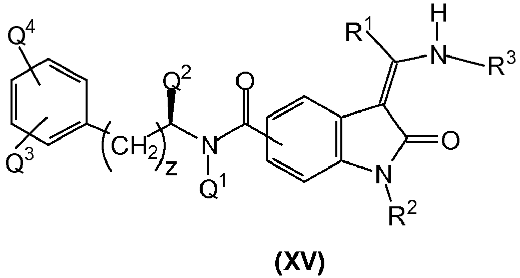 wherein the residues R¹ - R³, Q¹ - Q⁴, and z have the meanings as defined herein. The amide moiety is preferably bound at the 5-position of the indol backbone.

A preferred embodiment of this invention is directed to compounds of general formula **(XVI)** 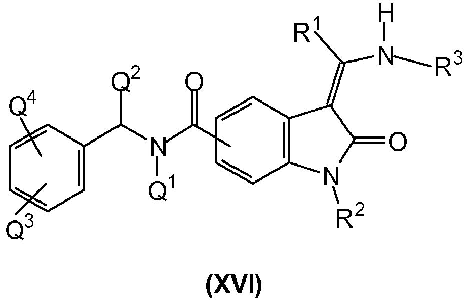 wherein the residues R¹ - R³, and Q¹ - Q⁴ have the meanings as defined herein. The amide moiety is preferably bound at the 5-position of the indol backbone.

A preferred embodiment of this invention is directed to compounds of general formula **(XVII)** 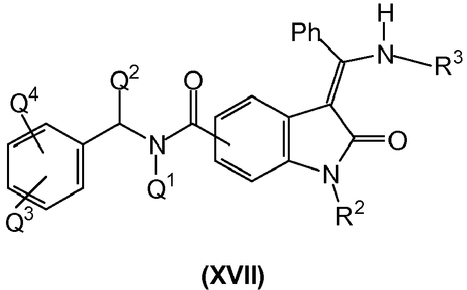 wherein the residues R², R³ and Q¹ - Q⁴ have the meanings as defined herein. The amide moiety is preferably bound at the 5-position of the indol backbone.

A preferred embodiment of this invention is directed to compounds of general formula **(XVIII)** 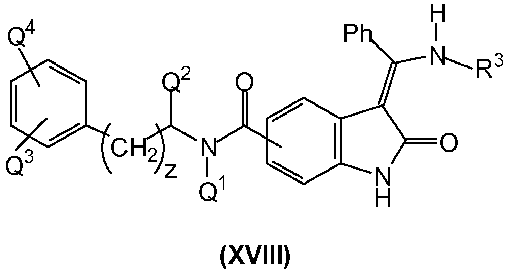 wherein the residues R³, Q¹ - Q⁴, and z have the meanings as defined herein. The amide moiety is preferably bound at the 5-position of the indol backbone.

A preferred embodiment of this invention is directed to compounds of general formula **(XIX)** 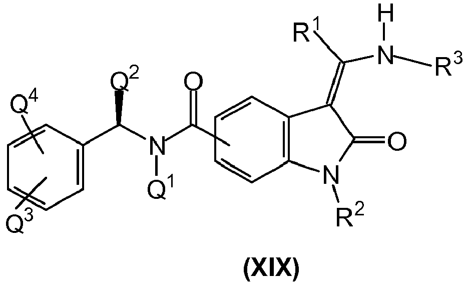 wherein the residues R¹ - R³ and Q¹ - Q⁴ have the meanings as defined herein. The amide moiety is preferably bound at the 5-position of the indol backbone.

A preferred embodiment of this invention is directed to compounds of general formula **(XX)** 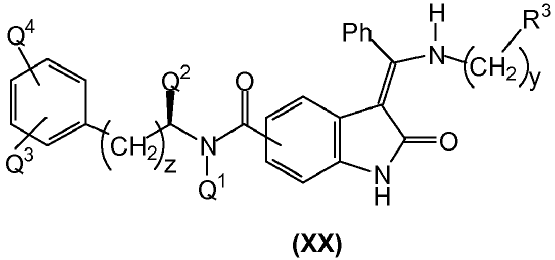 wherein the residues R³, Q¹ - Q⁴, y and z have the meanings as defined herein. The amide moiety is preferably bound at the 5-position of the indol backbone.

A preferred embodiment of this invention is directed to compounds of general formula **(XXI)** 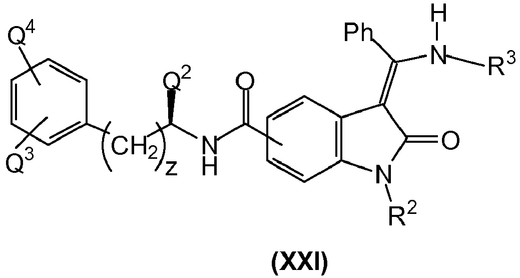 wherein the residues R², R³, Q¹ - Q⁴, and z have the meanings as defined herein. The amide moiety is preferably bound at the 5-position of the indol backbone.

A preferred embodiment of this invention is directed to compounds of general formula **(XXII)** 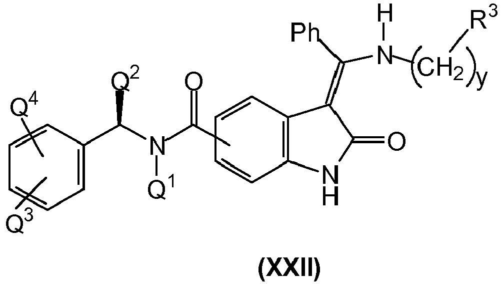 wherein the residues R³, Q¹ - Q⁴, and y have the meanings as defined herein. The amide moiety is preferably preferably bound at the 5-position of the indol backbone.

A preferred embodiment of this invention is directed to compounds of general formula **(XXIII)** 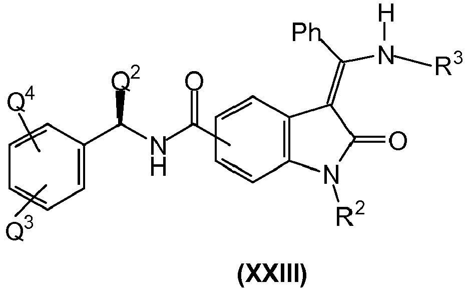 wherein the residues R², R³ and Q² - Q⁴ have the meanings as defined herein. The amide moiety is preferably preferably bound at the 5-position of the indol backbone.

A preferred embodiment of this invention is directed to compounds of general formula **(XXIV)** 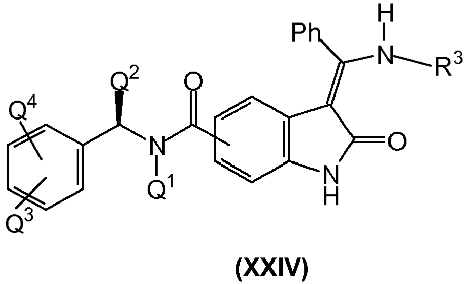 wherein the residues R³ and Q¹ - Q⁴ have the meanings as defined herein. The amide moiety is preferably bound at the 5-position of the indol backbone.

A preferred embodiment of this invention is directed to compounds of general formula **(XXV)** 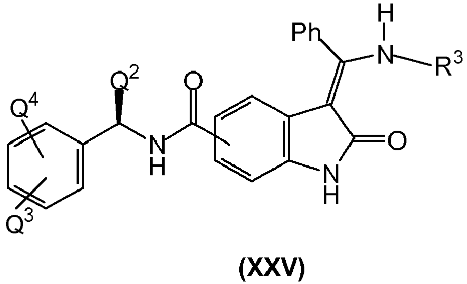 wherein the residues R³ and Q² - Q⁴ have the meanings as defined herein. The amide moiety is preferably bound at the 5-position of the indol backbone.

A preferred embodiment of this invention is directed to compounds of general formula **(XXVI)** 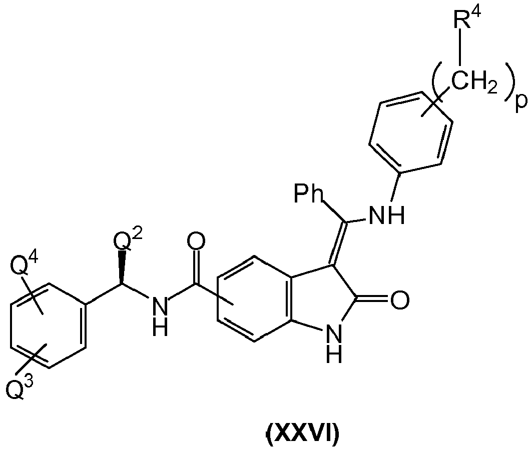 wherein the residues R⁴, Q² - Q⁴, and p have the meanings as defined herein. The amide moiety is preferably bound at the 5-position of the indol backbone.

A preferred embodiment of this invention is directed to compounds of general formula **(XXVII)** 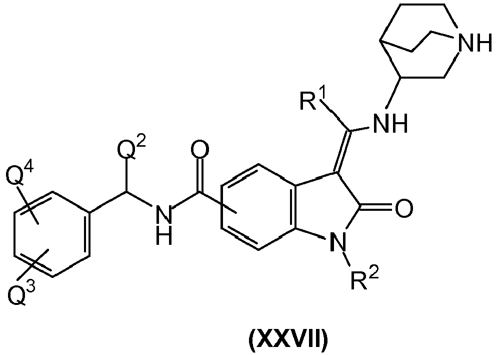 wherein the residues R¹, R², and Q² - Q⁴ have the meanings as defined herein. The amide moiety is preferably bound at the 5-position of the indol backbone.

Another preferred embodiment of the present invention relates to compounds of the general formulas (VII)-(XVI), (XIX) and (XXVII), wherein R¹ represents: -CH₂CH₃, -CH(CH₃)₂, 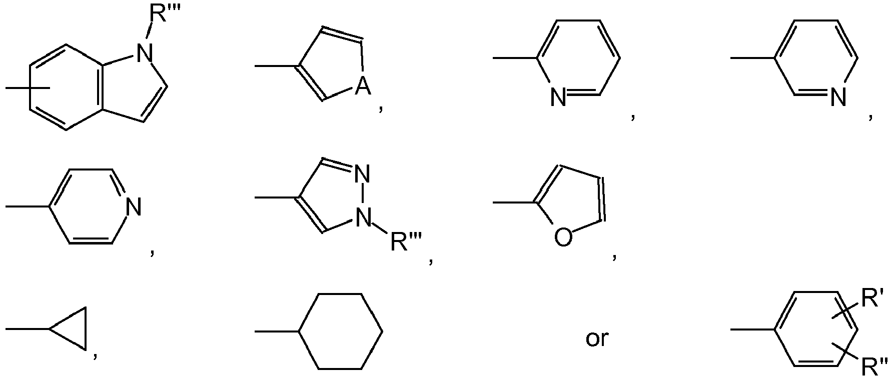 wherein R' and R" are independently of each other selected from: -H, -F, -CH₃ and -OCH₃; R"' represents: -CH₃; A represents: -O- or -S-.

Furthermore, a preferred embodiment refers to inventive compounds of general formulas (I)-(III), (V), (VI), (IX), (XII)-(XVII), (XIX), (XXI), and (XXVII), wherein R² represents: -H or -COCH₃.

Moreover, a preferred embodiment according to the invention comprises compounds of general formulas (I)-(XXV), wherein
R³ represents: 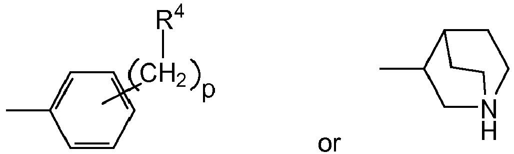 R⁴ represents: -R¹², -NH₂, -N(CH₃)₂,

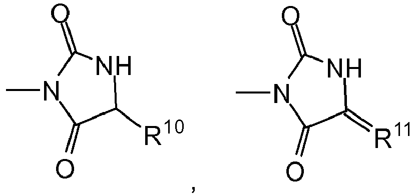

-N(C₂H₅)₂,

or 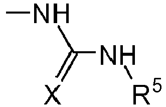 X represents: =O, =S;
R⁵ represents: -R⁶, 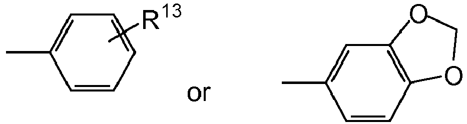 R⁶ is selected form : -H, -CH₃, -C₂H₅, -C₃H₇, -CH(CH₃)₂, -C(CH₃)₃-(CH₂)ₙ-CH₃, -(CH₂)ₙ-C(CH₃)₂, -CH[(CH₂)ₙCH₃]-(CH₂)ₘ-CH₃, -CH[(CH₂)ₙCH₃]-(CH₂)ₘ-C(CH₃)₂, -CH=CH₂, -(CH₂)ₙ-CH=CH-(CH₂)ₖ-CH₃, -CH≡CH, -(CH₂)ₙ-C≡C-(CH₂)ₖ-CH₃, -Ph, -(CH₂)ₙ-Ph, -(CH₂)ₙ-OH, -(CH₂)ₙ-NH₂, -(CH₂)ₙ-O-(CH₂)ₖ-CH₃, -(CH₂)ₙ-NH(CH₂)ₖ-CH₃, -(CH₂)ₙ-N(CH₃)₂ , -(CH₂)ₙ-N(C₂H₅)₂ and -(CH₂)ₙ-N(CH₃)(C₂H₅); preferably from : -H, -CH₃, -C₂H₅, -C₃H₇, -CH(CH₃)₂, -C(CH₃)₃-(CH₂)ₙ-CH₃, -(CH₂)ₙ-C(CH₃)₂, -CH=CH₂, -(CH₂)ₙ-CH=CH-(CH₂)ₖ-CH₃, -CH≡CH, -Ph, -(CH₂)ₙ-Ph, -(CH₂)ₙ-OH, -(CH₂)ₙ-NH₂, -(CH₂)ₙ-O-(CH₂)ₖ-CH₃, -(CH₂)ₙ-N(CH₃)₂, and -(CH₂)ₙ-N(C₂H₅)₂; more preferably from : -H, -CH₃, -C₂H₅, -C₃H₇, -CH(CH₃)₂, -CH=CH₂,-CH≡CH, -Ph, -CH₂-Ph, -CH₂-OH, and -CH₂-NH₂;
R13 represents: -OH, -Cl, 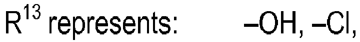 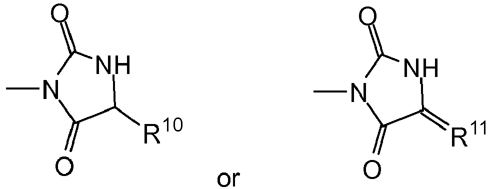 R¹¹ represents: 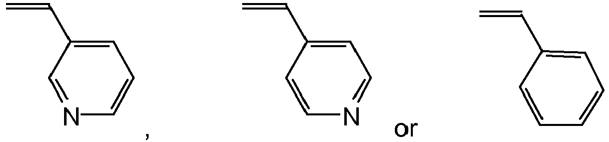 R¹² represents: 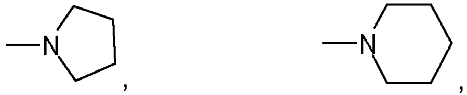 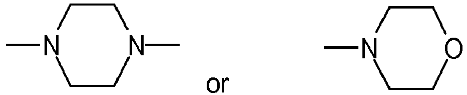 R¹⁰ has the meanings as disclosed herein and preferably R¹⁰ represents hydrogen;
k, m and n have the meanings as disclosed herein; and
p is an integer number selected from: 0 and 1.

A further preferred embodiment according to the invention refers to the compounds of the general formulas (I)-(XXVII), wherein Q³ is in para-position and Q⁴ in meta-position of the benzene ring. Q³ represents: -H and Q⁴ is selected from: -H, -F, -Cl, -CH₃, or Q⁴ represents: -H and Q³ is selected from: -H, -F, -Cl, or -CH₃.

A preferred embodiment of this invention is directed to compounds of general formulas (III), (VIII), (XII), (XIV), (XV), and (XIX)-(XVII), wherein Q² is selected from: -CH₃ and -C₂H₅.

Furthermore, a preferred embodiment refers to inventive compounds of general formulas (I), (III)-(VI), (VII)-(XIII), (XV), (XVIII), (XX), and (XXI), wherein z represents 0.

A preferred embodiment of the invention comprises the compounds according to the general formula **(I)** as depicted in the following Table 1

**Table 1:**

| No. | Compound |
|---|---|
| 12 | (3Z)-2-oxo-3-(phenyl{[3-(piperidin-1-ylmethyl)phenyl]amino} methylene)-*N*-[(1*R*)-1-phenylpropyl]indoline-5-carboxamide |
| 13 | (3*Z*)-2-oxo-*N*-[(1*R*)-1-phenylethyl]-3-(phenyl{[3-(piperidin-1-ylmethyl)phenyl] amino}methylene)indoline-5-carboxamide |
| 14 | (3*Z*)-2-oxo-3-(phenyl{[3-(piperidin-1-ylmethyl)phenyl]amino}methylene)-*N-*[(1*S*)-1-phenylpropyl]indoline-5-carboxamide |
| 15 | (3*Z*)-*N*-[(1*S*)-1-(4-chlorophenyl)ethyl]-2-oxo-3-(phenyl{[3-(piperidin-1-ylmethyl) phenyl]amino}methylene)indoline-5-carboxamide |
| 16 | (3*Z*)-*N*-[(1*R*)-1-(4-chlorophenyl)ethyl]-2-oxo-3-(phenyl{[3-(piperidin-1-ylmethyl)phenyl]amino}methylene)indoline-5-carboxamide |
| 17 | (3*Z*)-*N*-[(1*R*)-1-(4-fluorophenyl)ethyl]-2-oxo-3-(phenyl{[3-(piperidin-1-ylmethyl)phenyl]amino}methylene)indoline-5-carboxamide |
| 18 | (3*Z*)-2-oxo-3-(phenyl{[4-(piperidin-1-ylmethyl)phenyl]amino}methylene)-*N-*[(1*R*)-1-phenylpropyl]indoline-5-carboxamide |
| 19 | (3*Z*)-*N*-methyl-2-oxo-*N*-[(1*R*)-1-phenylethyl]-3-(phenyl{[3-(piperidin-1-ylmethyl)phenyl]amino}methylene)indoline-5-carboxamide |
| 20 | (3*Z*)-*N*-benzyl-2-oxo-3-(phenyl{[4-(piperidin-1-ylmethyl)phenyl]amino} methylene)indoline-5-carboxamide |
| 21 | (3*Z*)-*N*-[(1*R*)-1-(4-methoxyphenyl)ethyl]-2-oxo-3-(phenyl{[4-(piperidin-1-ylmethyl)phenyl]amino}methylene)indoline-5-carboxamide |
| 22 | (3*Z*)-*N*-[(1*R*)-1-(3-methoxyphenyl)ethyl]-2-oxo-3-(phenyl{[4-(piperidin-1-ylmethyl)phenyl]amino}methylene)indoline-5-carboxamide |
| 23 | (3*Z*)-*N*-[(1*R*)-1-(4-fluorophenyl)ethyl]-2-oxo-3-(phenyl{[4-(piperidin-1-ylmethyl)phenyl]amino}methylene)indoline-5-carboxamide |
| 24 | (3*Z*)-*N*-[(1*R*)-1-(3-methoxyphenyl)ethyl]-2-oxo-3-(phenyl{[3-(piperidin-1-ylmethyl)phenyl]amino}methylene)indoline-5-carboxamide |
| 25 | (3*Z*)-2-oxo-*N*-[(1*S*)-1-phenylethyl]-3-(phenyl{[3-(piperidin-1-ylmethyl)phenyl]amino}methylene)indoline-5-carboxamide |
| 26 | (3*Z*)-*N*-[(1*S*)-1-(4-fluorophenyl)ethyl]-2-oxo-3-(phenyl{[3-(piperidin-1-ylmethyl)phenyl]amino}methylene)indoline-5-carboxamide |
| 27 | (3*Z*)-*N*-[(1*R*)-1-(4-methylphenyl)ethyl]-2-oxo-3-(phenyl{[3-(piperidin-1-ylmethyl)phenyl]amino}methylene)indoline-5-carboxamide |
| 28 | (3*Z*)-*N*-[(1*S*)-1-(4-methylphenyl)ethyl]-2-oxo-3-(phenyl{[3-(piperidin-1-ylmethyl)phenyl]amino}methylene)indoline-5-carboxamide |
| 29 | (3*Z*)-*N*-[(1*R*)-1-(4-methoxyphenyl)ethyl]-2-oxo-3-(phenyl{[3-(piperidin-1-ylmethyl)phenyl]amino}methylene)indoline-5-carboxamide |
| 30 | (3*Z*)-2-oxo-*N*-[(1*S*)-1-phenylethyl]-3-(phenyl{[4-(piperidin-1-ylmethyl)phenyl]amino}methylene)indoline-5-carboxamide |
| 31 | (3*Z*)-2-oxo-*N*-[(1*R*)-1-phenylethyl]-3-(phenyl{[4-(piperidin-1-ylmethyl)phenyl]amino}methylene)indoline-5-carboxamide |
| 32 | (3*Z*)-2-oxo-3-(phenyl{[4-(piperidin-1-ylmethyl)phenyl]amino}methylene)-*N-*[(1*S*)-1-phenylpropyl]indoline-5-carboxamide |
| 33 | (3*Z*)-*N*-[(1*S*)-1-(4-methoxyphenyl)ethyl]-2-oxo-3-(phenyl{[4-(piperidin-1-ylmethyl)phenyl]amino}methylene)indoline-5-carboxamide |
| 34 | (3*Z*)-*N*-[(1*S*)-1-(3-methoxyphenyl)ethyl]-2-oxo-3-(phenyl{[4-(piperidin-1-ylmethyl)phenyl]amino}methylene)indoline-5-carboxamide |
| 35 | (3*Z*)-*N*-[(1*R*)-1-(3-methoxyphenyl)ethyl]-3-[({4-[(4-methylpiperazin-1-yl)methyl]phenyl}amino)(phenyl)methylene]-2-oxoindoline-5-carboxamide |
| 36 | (3*Z*)-*N*-[(1*R*)-1-(3-chlorophenyl)ethyl]-3-[({4-[(4-methylpiperazin-1-yl)methyl]phenyl}amino)(phenyl)methylene]-2-oxoindoline-5-carboxamide |
| 37 | (3*Z*)-3-[({4-[(4-methylpiperazin-1-yl)methyl]phenyl}amino)(phenyl)methylene]-2-oxo-*N*-[(1*R*)-1-phenylpropyl]indoline-5-carboxamide |
| 38 | (3Z)-3-[({4-[(4-methylpiperazin-1-yl)methyl]phenyl}amino)(phenyl)methylene]-2-oxo-*N*-[(1*R*)-1-phenylethyl]indoline-5-carboxamide |
| 39 | (3*Z*)-*N*-(3,4-dimethoxybenzyl)-3-[({4-[(4-methylpiperazin-1-yl)methyl]phenyl}amino)(phenyl)methylene]-2-oxoindoline-5-carboxamide |
| 40 | (3*Z*)-*N*-(3-methoxybenzyl)-3-[({4-[(4-methylpiperazin-1-yl)methyl]phenyl}amino)(phenyl)methylene]-2-oxoindoline-5-carboxamide |
| 41 | (3*Z*)-2-oxo-*N*-[(1*R*)-1-phenylethyl]-3-(phenyl{[4-(piperidin-1-ylmethyl)phenyl]amino}methylene)indoline-6-carboxamide |
| 42 | (3*Z*)-2-oxo-3-(phenyl{[4-(piperidin-1-ylmethyl)phenyl]amino}methylene)-*N-*[(1*R*)-1-phenylpropyl]indoline-6-carboxamide |
| 43 | (3*Z*)-3-[({3-[(4-methylpiperazin-1-yl)methyl]phenyl}amino)(phenyl)methylene]-2-oxo-*N*-[(1*R*)-1-phenylethyl]indoline-5-carboxamide |
| 44 | (3*Z*)-3-[({3-[(4-methylpiperazin-1-yl)methyl]phenyl}amino)(phenyl)methylene]-2-oxo-*N*-[(1*R*)-1-phenylpropyl]indoline-5-carboxamide |
| 45 | (3*Z*)-*N*-benzyl-3-[({3-[(4-methylpiperazin-1-yl)methyl]phenyl}amino)(phenyl)methylene]-2-oxoindoline-5-carboxamide |
| 46 | (3*Z*)-*N*-[(1*R*)-1-(3-chlorophenyl)ethyl]-3-[({3-[(4-methylpiperazin-1-yl)methyl]phenyl}amino)(phenyl)methylene]-2-oxoindoline-5-carboxamide |
| 47 | (3*Z*)-*N*-[(1*R*)-1-(3-methoxyphenyl)ethyl]-3-[({3-[(4-methylpiperazin-1-yl)methyl]phenyl}amino)(phenyl)methylene]-2-oxoindoline-5-carboxamide |
| 48 | (3*Z*)-*N*-(3-methoxybenzyl)-3-[({3-[(4-methylpiperazin-1-yl)methyl]phenyl}amino) (phenyl)methylene]-2-oxoindoline-5-carboxamide |
| 49 | (3*Z*)-*N*-(3,4-dimethoxybenzyl)-3-[({3-[(4-methylpiperazin-1-yl)methyl]phenyl}amino)(phenyl)methylene]-2-oxoindoline-5-carboxamid |
| 135 | (3*Z*)-3-[({4-[(4-methylpiperazin-1-yl)methyl]phenyl}amino)(pyridin-4-yl)methylene]-2-oxo-*N*-[(1*R*)-1-phenylpropyl]indoline-5-carboxamide |
| 136 | (3*Z*)-*N*-benzyl-3-[({4-[(4-methylpiperazin-1-yl)methyl]phenyl}amino)(pyridin-3-yl)methylene]-2-oxoindoline-5-carboxamide |
| 137 | (3*Z*)-*N*-(4-fluorobenzyl)-3-[({4-[(4-methylpiperazin-1-yl)methyl]phenyl}amino)(pyridin-3-yl)methylene]-2-oxoindoline-5-carboxamide |
| 138 | (3*Z*)-3-[({4-[(4-methylpiperazin-1-yl)methyl]phenyl}amino)(pyridin-3-yl)methylene]-2-oxo-*N*-[(1*R*)-1-phenylethyl]indoline-5-carboxamide |
| 139 | (3*Z*)-3-[({4-[(4-methylpiperazin-1-yl)methyl]phenyl}amino)(pyridin-3-yl)methylene]-2-oxo-*N*-[(1*R*)-1-phenylpropyl]indoline-5-carboxamide |
| 140 | (3*Z*)-2-oxo-*N*-[(1*R*)-1-phenylethyl]-3-[{[4-(piperidin-1-ylmethyl)phenyl]amino}(pyridin-3-yl)methylene]indoline-5-carboxamide |
| 141 | (3*Z*)-2-oxo-*N*-[(1*S*)-1-phenylethyl]-3-[{[4-(piperidin-1-ylmethyl)phenyl]amino}(pyridin-3-yl)methylene]indoline-5-carboxamide |
| 142 | (3*Z*)-2-oxo-*N*-[(1*R*)-1-phenylpropyl]-3-[{[4-(piperidin-1-ylmethyl)phenyl]amino}(pyridin-3-yl)methylene]indoline-5-carboxamide |
| 143 | (3*Z*)-3-[({4-[(diethylamino)methyl]phenyl}amino)(pyridin-3-yl)methylene]-2-oxo-*N*-[(1*R*)-1-phenylpropyl]indoline-5-carboxamide |
| 144 | (3*Z*)-3-[({4-[(diethylamino)methyl]phenyl}amino)(pyridin-3-yl)methylene]-2-oxo-*N*-[(1*R*)-1-phenylethyl]indoline-5-carboxamide |
| 145 | (3*Z*)-2-oxo-*N*-[(1*S*)-1-phenylpropyl]-3-[{[4-(piperidin-1-ylmethyl)phenyl]amino}(pyridin-3-yl)methylene]indoline-5-carboxamide |
| 146 | (3*Z*)-3-[{[4-(morpholin-4-ylmethyl)phenyl]amino}(pyridin-3-yl)methylene]-2-oxo-*N*-[(1*R*)-1-phenylethyl]indoline-5-carboxamide |
| 147 | (3*Z*)-3-[{[4-(morpholin-4-ylmethyl)phenyl]amino}(pyridin-3-yl)methylene]-2-oxo-*N*-[(1*R*)-1-phenylpropyl]indoline-5-carboxamide |
| 148 | (3*Z*)-3-[({3-[(4-methylpiperazin-1-yl)methyl]phenyl}amino)(pyridin-3-yl)methylene]-2-oxo-*N*-[(1*R*)-1-phenylethyl]indoline-5-carboxamide |
| 149 | (3*Z*)-3-[({3-[(4-methylpiperazin-1-yl)methyl]phenyl}amino)(pyridin-3-yl)methylene]-2-oxo-*N*-[(1*R*)-1-phenylpropyl]indoline-5-carboxamide |
| 150 | (3*Z*)-2-oxo-*N*-[(1*R*)-1-phenylpropyl]-3-[{[3-(piperidin-1-ylmethyl)phenyl]amino}(pyridin-3-yl)methylene]indoline-5-carboxamide |
| 151 | (3*Z*)-2-oxo-*N*-[(1*S*)-1-phenylpropyl]-3-[{[3-(piperidin-1-ylmethyl)phenyl]amino}(pyridin-3-yl)methylene]indoline-5-carboxamide |
| 152 | (3*Z*)-2-oxo-*N*-[(1*S*)-1-phenylethyl]-3-[{[3-(piperidin-1-ylmethyl)phenyl]amino}(pyridin-3-yl)methylene]indoline-5-carboxamide |
| 153 | (3*Z*)-2-oxo-*N*-[(1*R*)-1-phenylethyl]-3-[{[3-(piperidin-1-ylmethyl)phenyl]amino}(pyridin-3-yl)methylene]indoline-5-carboxamide |
| 154 | (3*Z*)-2-oxo-*N*-[(1*R*)-1-phenylethyl]-3-[{[4-(piperidin-1-ylmethyl)phenyl]amino}(pyridin-2-yl)methylene]indoline-5-carboxamide |
| 155 | (3*Z*)-2-oxo-*N*-[(1*R*)-1-phenylpropyl]-3-[{[4-(piperidin-1-ylmethyl)phenyl]amino}(pyridin-2-yl)methylene]indoline-5-carboxamide |
| 156 | (3*Z*)-2-oxo-*N*-[(1*R*)-1-phenylethyl]-3-(pyridin-2-yl{[3-(pyrrolidin-1-ylmethyl)phenyl]amino}methylene)indoline-5-carboxamide |
| 157 | (3*Z*)-2-oxo-*N*-[(1*R*)-1-phenylpropyl]-3-(pyridin-2-yl{[3-(pyrrolidin-1-ylmethyl)phenyl]amino}methylene)indoline-5-carboxamide |
| 158 | (3*Z*)-2-oxo-*N*-[(1*R*)-1-phenylethyl]-3-[{[3-(piperidin-1-ylmethyl)phenyl]amino}(pyridin-2-yl)methylene]indoline-5-carboxamide |
| 159 | (3*Z*)-2-oxo-*N*-[(1*R*)-1-phenylpropyl]-3-[{[3-(piperidin-1-ylmethyl)phenyl]amino}(pyridin-2-yl)methylene]indoline-5-carboxamide |
| 160 | (3*Z*)-3-[({4-[(4-methylpiperazin-1-yl)methyl]phenyl}amino)(pyridin-4-yl)methylene]-2-oxo-*N*-[(1*R*)-1-phenylethyl]indoline-5-carboxamide |
| 161 | (3*Z*)-2-oxo-*N*-[(1*R*)-1-phenylpropyl]-3-[{[4-(piperidin-1-ylmethyl)phenyl]amino}(pyridin-4-yl)methylene]indoline-5-carboxamide |
| 162 | (3*Z*)-2-oxo-*N*-[(1*S*)-1-phenylpropyl]-3-[{[4-(piperidin-1-ylmethyl)phenyl]amino}(pyridin-4-yl)methylene]indoline-5-carboxamide |
| 163 | (3*Z*)-2-oxo-*N*-[(1*R*)-1-phenylethyl]-3-[{[4-(piperidin-1-ylmethyl)phenyl]amino}(pyridin-4-yl)methylene]indoline-5-carboxamide |
| 164 | (3*Z*)-2-oxo-*N*-[(1*S*)-1-phenylethyl]-3-[{[4-(piperidin-1-ylmethyl)phenyl]amino}(pyridin-4-yl)methylene]indoline-5-carboxamide |
| 165 | (3*Z*)-3-[({4-[(diethylamino)methyl]phenyl}amino)(pyridin-4-yl)methylene]-2-oxo-*N*-[(1*R*)-1-phenylethyl]indoline-5-carboxamide |
| 166 | (3*Z*)-3-[({4-[(diethylamino)methyl]phenyl}amino)(pyridin-4-yl)methylene]-2-oxo-*N*-[(1*R*)-1-phenylpropyl]indoline-5-carboxamide |
| 167 | (3*Z*)-3-[{[4-(morpholin-4-ylmethyl)phenyl]amino}(pyridin-4-yl)methylene]-2-oxo-*N*-[(1*R*)-1-phenylethyl]indoline-5-carboxamide |
| 168 | (3*Z*)-3-[{[4-(morpholin-4-ylmethyl)phenyl]amino}(pyridin-4-yl)methylene]-2-oxo-*N*-[(1*R*)-1-phenylpropyl]indoline-5-carboxamide |
| 169 | (3*Z*)-2-oxo-*N*-[(1*R*)-1-phenylpropyl]-3-(pyridin-4-yl{[4-(pyrrolidin-1-ylmethyl)phenyl]amino}methylene)indoline-5-carboxamide |
| 170 | (3*Z*)-2-oxo-*N*-[(1*R*)-1-phenylethyl]-3-(pyridin-4-yl{[4-(pyrrolidin-1-ylmethyl)phenyl]amino}methylene)indoline-5-carboxamide |
| 171 | (3*Z*)-2-oxo-*N*-[(1*R*)-1-phenylethyl]-3-[{[4-(pyrrolidin-1-ylmethyl)phenyl]amino}(3-thienyl)methylene]indoline-5-carboxamide |
| 172 | (3*Z*)-2-oxo-*N*-[(1*R*)-1-phenylpropyl]-3-[{[4-(pyrrolidin-1-ylmethyl)phenyl]amino}(3-thienyl)methylene]indoline-5-carboxamide |
| 173 | (3*Z*)-2-oxo-*N*-[(1*R*)-1-phenylethyl]-3-[{[4-(piperidin-1-ylmethyl)phenyl]amino}(3-thienyl)methylene]indoline-5-carboxamide |
| 174 | (3*Z*)-2-oxo-*N*-[(1*R*)-1-phenylpropyl]-3-[{[4-(piperidin-1-ylmethyl)phenyl]amino}(3-thienyl)methylene]indoline-5-carboxamide |
| 175 | (3*Z*)-3-[{[4-(morpholin-4-ylmethyl)phenyl]amino}(3-thienyl)methylene]-2-oxo-*N*-[(1*R*)-1-phenylethyl]indoline-5-carboxamide |
| 176 | (3*Z*)-3-[{[4-(morpholin-4-ylmethyl)phenyl]amino}(3-thienyl)methylene]-2-oxo-*N*-[(1*R*)-1-phenylpropyl]indoline-5-carboxamide |
| 177 | (3*Z*)-3-[({4-[(4-methylpiperazin-1-yl)methyl]phenyl}amino)(3-thienyl)methylene]-2-oxo-*N*-[(1*R*)-1-phenylethyl]indoline-5-carboxamide |
| 178 | (3*Z*)-3-[({4-[(4-methylpiperazin-1-yl)methyl]phenyl}amino)(3-thienyl)methylene]-2-oxo-*N*-[(1*R*)-1-phenylpropyl]indoline-5-carboxamide |
| 179 | (3*Z*)-2-oxo-*N*-[(1*R*)-1-phenylethyl]-3-[{[3-(pyrrolidin-1-ylmethyl)phenyl]amino}(3-thienyl)methylene]indoline-5-carboxamide |
| 180 | (3*Z*)-2-oxo-*N*-[(1*R*)-1-phenylpropyl]-3-[{[3-(pyrrolidin-1-ylmethyl)phenyl]amino}(3-thienyl)methylene]indoline-5-carboxamide |
| 181 | (3*Z*)-2-oxo-*N*-[(1*R*)-1-phenylethyl]-3-[{[3-(piperidin-1-ylmethyl)phenyl]amino}(3-thienyl)methylene]indoline-5-carboxamide |
| 182 | (3*Z*)-2-oxo-*N*-[(1*R*)-1-phenylpropyl]-3-[{[3-(piperidin-1-ylmethyl)phenyl]amino}(3-thienyl)methylene]indoline-5-carboxamide |
| 183 | (3*Z*)-3-[({3-[(4-methylpiperazin-1-yl)methyl]phenyl}amino)(3-thienyl)methylene]-2-oxo-*N*-[(1*R*)-1-phenylethyl]indoline-5-carboxamide |
| 184 | (3*Z*)-3-[({3-[(4-methylpiperazin-1-yl)methyl]phenyl}amino)(3-thienyl)methylene]-2-oxo-*N*-[(1*R*)-1-phenylpropyl]indoline-5-carboxamide |
| 185 | (3*Z*)-3-[{[3-(morpholin-4-ylmethyl)phenyl]amino}(3-thienyl)methylene]-2-oxo-*N*-[(1*R*)-1-phenylethyl]indoline-5-carboxamide |
| 186 | (3*Z*)-3-[{[3-(morpholin-4-ylmethyl)phenyl]amino}(3-thienyl)methylene]-2-oxo-*N*-[(1*R*)-1-phenylpropyl]indoline-5-carboxamide |
| 187 | (3*Z*)-3-(3-furyl{[4-(pyrrolidin-1-ylmethyl)phenyl]amino}methylene)-2-oxo-*N-*[(1*R*)-1-phenylethyl]indoline-5-carboxamide |
| 188 | (3*Z*)-3-(3-furyl{[4-(piperidin-1-ylmethyl)phenyl]amino}methylene)-2-oxo-*N-*[(1*R*)-1-phenylpropyl]indoline-5-carboxamide |
| 189 | (3*Z*)-3-(2-furyl{[4-(piperidin-1-ylmethyl)phenyl]amino}methylene)-2-oxo-*N-*[(1 R)-1-phenylpropyl]indoline-5-carboxamide |
| 190 | (3*Z*)-3-[(1-methyl-1*H*-pyrazol-4-yl){[4-(pyrrolidin-1-ylmethyl)phenyl]amino}methylene]-2-oxo-*N*-[(1*R*)-1-phenylethyl]indoline-5-carboxamide |
| 191 | (3*Z*)-3-[(1-methyl-1*H*-pyrazol-4-yl){[4-(pyrrolidin-1-ylmethyl)phenyl]amino}methylene]-2-oxo-*N*-[(1*R*)-1-phenylpropyl]indoline-5-carboxamide |
| 192 | (3*Z*)-3-[(1-methyl-1*H*-pyrazol-4-yl){[4-(piperidin-1-ylmethyl)phenyl]amino}methylene]-2-oxo-*N*-[(1*R*)-1-phenylethyl]indoline-5-carboxamide |
| 193 | (3*Z*)-3-[(1-methyl-1*H*-pyrazol-4-yl){[4-(piperidin-1-ylmethyl)phenyl]-amino}methylene]-2-oxo-*N*-[(1*R*)-1-phenylpropyl]indoline-5-carboxamide |
| 194 | (3*Z*)-3-[(1-methyl-1*H*-pyrazol-4-yl){[3-(pyrrolidin-1-ylmethyl)phenyl]amino}methylene]-2-oxo-*N*-[(1*R*)-1-phenylethyl]indoline-5-carboxamide |
| 195 | (3*Z*)-3-[(1-methyl-1*H*-pyrazol-4-yl){[3-(pyrrolidin-1-ylmethyl)phenyl]amino}methylene]-2-oxo-*N*-[(1*R*)-1-phenylpropyl]indoline-5-carboxamide |
| 196 | (3*Z*)-3-[(1-methyl-1*H*-pyrazol-4-yl){[3-(piperidin-1-ylmethyl)phenyl]amino}methylene]-2-oxo-*N*-[(1*R*)-1-phenylethyl]indoline-5-carboxamide |
| 197 | (3*Z*)-3-[(1-methyl-1*H*-pyrazol-4-yl){[3-(piperidin-1-ylmethyl)phenyl]amino}methylene]-2-oxo-*N*-[(1*R*)-1-phenylpropyl]indoline-5-carboxamide |
| 198 | (3*Z*)-3-[(1-methyl-1*H*-indol-5-yl){[4-(pyrrolidin-1-ylmethyl)phenyl]amino}methylene]-2-oxo-*N*-[(1*R*)-1-phenylethyl]indoline-5-carboxamide |
| 199 | (3*Z*)-3-[(1-methyl-1*H*-indol-5-yl){[4-(pyrrolidin-1-ylmethyl)phenyl]amino}methylene]-2-oxo-*N*-[(1*R*)-1-phenylpropyl]indoline-5-carboxamide |
| 200 | (3*Z*)-3-[(1-methyl-1*H*-indol-5-yl){[4-(piperidin-1-ylmethyl)phenyl]amino}methylene]-2-oxo-*N*-[(1*R*)-1-phenylethyl]indoline-5-carboxamide |
| 201 | (3*Z*)-3-[(1-methyl-1*H*-indol-5-yl){[4-(piperidin-1-ylmethyl)phenyl]amino}methylene]-2-oxo-*N*-[(1*R*)-1-phenylpropyl]indoline-5-carboxamide |
| 202 | (3*Z*)-3-[(1-methyl-1*H*-indol-5-yl){[3-(pyrrolidin-1-ylmethyl)phenyl]amino}methylene]-2-oxo-*N*-[(1*R*)-1-phenylethyl]indoline-5-carboxamide |
| 203 | (3*Z*)-3-[(1-methyl-1*H*-indol-5-yl){[3-(pyrrolidin-1-ylmethyl)phenyl]amino}methylene]-2-oxo-*N*-[(1*R*)-1-phenylpropyl]indoline-5-carboxamide |
| 204 | (3*Z*)-3-[(1-methyl-1*H*-indol-5-yl){[3-(piperidin-1-ylmethyl)phenyl]amino}methylene]-2-oxo-*N*-[(1*R*)-1-phenylethyl]indoline-5-carboxamide |
| 205 | (3*Z*)-3-[(1-methyl-1*H*-indol-5-yl){[3-(piperidin-1-ylmethyl)phenyl]-amino}methylene]-2-oxo-*N*-[(1*R*)-1-phenylpropyl]indoline-5-carboxamide |
| 206 | (3*Z*)-3-(cyclopropyl{[4-(pyrrolidin-1-ylmethyl)phenyl]amino}methylene)-2-oxo-*N*-[(1*R*)-1-phenylethyl]indoline-5-carboxamide |
| 207 | (3*Z*)-3-(cyclopropyl{[4-(pyrrolidin-1-ylmethyl)phenyl]amino}methylene)-2-oxo-*N*-[(1*R*)-1-phenylpropyl]indoline-5-carboxamide |
| 208 | (3Z)-3-(cyclopropyl{[4-(piperidin-1-ylmethyl)phenyl]amino}methylene)-2-oxo-*N*-[(1*R*)-1-phenylethyl]indoline-5-carboxamide |
| 209 | (3*Z*)-3-(cyclopropyl{[4-(piperidin-1-ylmethyl)phenyl]amino}methylene)-2-oxo-*N*-[(1*R*)-1-phenylpropyl]indoline-5-carboxamide |
| 210 | (3*Z*)-3-(cyclohexyl{[4-(piperidin-1-ylmethyl)phenyl]amino}methylene)-2-oxo-*N*-[(1*R*)-1-phenylethyl]indoline-5-carboxamide |
| 211 | (3*Z*)-3-(cyclohexyl{[4-(piperidin-1-ylmethyl)phenyl]amino}methylene)-2-oxo-*N*-[(1*R*)-1-phenylpropyl]indoline-5-carboxamide |
| 212 | (3*Z*)-2-oxo-*N*-[(1*R*)-1-phenylethyl]-3-(1-{[4-(piperidin-1-ylmethyl)phenyl]amino}propylidene) indoline-5-carboxamide |
| 213 | (3*Z*)-2-oxo-*N*-[(1*R*)-1-phenylpropyl]-3-(1-{[4-(piperidin-1-ylmethyl)phenyl]amino}propylidene)indoline-5-carboxamide |
| 214 | (3*Z*)-3-(2-methyl-1-{[4-(piperidin-1-ylmethyl)phenyl]amino}propylidene)-2-oxo-*N*-[(1*R*)-1-phenylethyl]indoline-5-carboxamide |
| 215 | (3Z)-3-(2-methyl-1-{[4-(piperidin-1-ylmethyl)phenyl]amino}propylidene)-2-oxo-*N*-[(1*R*)-1-phenylpropyl]indoline-5-carboxamide |
| 216 | (3*Z*)-3-[(4-fluorophenyl){[3-(piperidin-1-ylmethyl)phenyl]amino}methylene]-2-oxo-*N*-[(1*R*)-1-phenylethyl]indoline-5-carboxamide |
| 217 | (3Z)-3-[(4-fluorophenyl){[3-(piperidin-1-ylmethyl)phenyl]amino}methylene]-2-oxo-*N*-[(1*R*)-1-phenylpropyl]indoline-5-carboxamide |
| 218 | (3Z)-3-[(4-fluorophenyl){[3-(piperidin-1-ylmethyl)phenyl]amino}methylene]-*N*-[(1*R*)-1-(4-methoxyphenyl)ethyl]-2-oxoindoline-5-carboxamide |
| 219 | (3*Z*)-3-[(4-fluorophenyl){[3-(piperidin-1-ylmethyl)phenyl]amino}methylene]-*N*-[(1*R*)-1-(3-methoxyphenyl)ethyl]-2-oxoindoline-5-carboxamide |
| 220 | (3*Z*)-*N*-[(1*R*)-1-(3-chlorophenyl)ethyl]-3-[(4-fluorophenyl){[3-(piperidin-1-ylmethyl)phenyl]amino}methylene]-2-oxoindoline-5-carboxamide |
| 221 | (3*Z*)-*N*-[(1*R*)-1-(4-chlorophenyl)ethyl]-3-[(4-fluorophenyl){[3-(piperidin-1-ylmethyl)phenyl]amino}methylene]-2-oxoindoline-5-carboxamide |
| 222 | (3Z)-3-[(4-fluorophenyl){[3-(piperidin-1-ylmethyl)phenyl]amino}methylene]-*N*-[(1*R*)-1-(4-methylphenyl)ethyl]-2-oxoindoline-5-carboxamide |
| 223 | (3*Z*)-*N*-[1-(3,4-difluorophenyl)propyl]-3-[(4-fluorophenyl){[3-(piperidin-1-ylmethyl)phenyl]amino}methylene]-2-oxoindoline-5-carboxamide |
| 224 | (3*Z*)-*N*-[1-(3-chlorophenyl)propyl]-3-[(4-fluorophenyl){[3-(piperidin-1-ylmethyl)phenyl]amino}methylene]-2-oxoindoline-5-carboxamide |
| 225 | (3*Z*)-3-[({2-[(ethylcarbamoyl)amino]phenyl}amino)(phenyl)methylene]-2-oxo-*N*-[(1*R*)-1-phenylethyl]indoline-5-carboxamide |
| 226 | (3*Z*)-*N*-benzyl-3-{[(2-{[(ethylamino)carbonothioyl]amino}phenyl)amino] (phenyl)methylene}-2-oxoindoline-5-carboxamide |
| 227 | (3*Z*)-3-{[(2-{[(4-hydroxyphenyl)carbamoyl]amino}phenyl)amino](phenyl) methylene}-2-oxo-*N*-[(1*R*)-1-phenylpropyl]indoline-5-carboxamide |
| 228 | (3*Z*)-3-{[(2-{[(4-hydroxyphenyl)carbamoyl]amino}phenyl)amino](phenyl) methylene}-2-oxo-*N*-[(1*R*)-1-phenylpropyl]indoline-5-carboxamide |
| 229 | (3Z)-3-{[(2-{[(4-hydroxyphenyl)carbamoyl]amino}phenyl)amino](phenyl)methylene}-2-oxo-*N*-[(1*S*)-1-phenylpropyl]indoline-5-carboxamide |
| 230 | (3*Z*)-3-[{[2-(2,5-dioxoimidazolidin-1-yl)phenyl]amino}(phenyl)methylene]-2-oxo-*N*-[(1*R*)-1-phenylethyl]indoline-5-carboxamide |
| 231 | (3*Z*)-3-[({2-[(4Z)-2,5-dioxo-4-(pyridin-4-ylmethylene)imidazolidin-1-yl]phenyl}amino)(phenyl)methylene]-2-oxo-*N*-[(1*R*)-1-phenylethyl]indoline-5-carboxamide |
| 232 | (3*Z*)-3-[({3-[(ethylcarbamoyl)amino]phenyl}amino)(phenyl)methylene]-2-oxo-*N*-[(1*R*)-1-phenylpropyl]indoline-5-carboxamide |
| 233 | (3Z)-3-{[(3-{[(3-chlorophenyl)carbamoyl]amino}phenyl)amino](phenyl)methylene}-2-oxo-*N*-[(1*R*)-1-phenylpropyl]indoline-5-carboxamide |
| 234 | (3Z)-3-{[(3-{[(ethylamino)carbonothioyl]amino}phenyl)amino](phenyl)methylene}-2-oxo-*N*-[(1*S*)-1-phenylethyl]indoline-5-carboxamide |
| 235 | (3Z)-3-{[(3-{[(4-hydroxyphenyl)carbamoyl]amino}phenyl)amino](phenyl)methylene}-2-oxo-*N*-[(1*R*)-1-phenylpropyl]indoline-5-carboxamide |
| 236 | (3*Z*)-3-[{[3-({[4-(2,5-dioxoimidazolidin-1-yl)phenyl]carbamoyl}amino)phenyl]amino} (phenyl)methylene]-2-oxo-*N*-[(1*S*)-1-phenylethyl]indoline-5-carboxamide |
| 237 | (3Z)-3-[({3-[({4-[(4Z)-2,5-dioxo-4-(pyridin-3-ylmethylene)imidazolidin-1-yl]phenyl}carbamoyl)amino]phenyl}amino)(phenyl)methylene]-2-oxo-*N-*[(1*S*)-1-phenylethyl]indoline-5-carboxamide |
| 238 | (3Z)-3-{[(4-{[(ethylamino)carbonothioyl]amino}phenyl)amino](phenyl)methylene}-2-oxo-*N*-[(1*S*)-1-phenylpropyl]indoline-5-carboxamide |
| 239 | (3Z)-3-{[(4-{[(3-chlorophenyl)carbamoyl]amino}phenyl)amino](phenyl)methylene}-2-oxo-*N*-[(1*R*)-1-phenylpropyl]indoline-5-carboxamide |
| 240 | (3*Z*)-3-{[(4-{[(4-hydroxyphenyl)carbamoyl]amino}phenyl)amino](phenyl) methylene}-2-oxo-*N*-[(1*R*)-1-phenylpropyl]indoline-5-carboxamide |
| 241 | (3*Z*)-3-[{[4-(2,5-dioxoimidazolidin-1-yl)phenyl]amino}(pyridin-4-yl)methylene]-2-oxo-*N*-[(1*R*)-1-phenylethyl]indoline-5-carboxamide |
| 242 | (3*Z*)-3-[({4-[(4Z)-4-benzylidene-2,5-dioxoimidazolidin-1-yl]phenyl}amino)(pyridin-4-yl)methylene]-2-oxo-*N*-[(1*R*)-1-phenylethyl]indoline-5-carboxamide |
| 243 | (3*Z*)-3-{[(2-{[(ethylamino)carbonothioyl]amino}benzyl)amino](3-thienyl)methylene}-2-oxo-*N*-[(1*R*)-1-phenylethyl]indoline-5-carboxamide |
| 244 | (3*Z*)-3-[({2-[(1,3-benzodioxol-5-ylcarbamoyl)amino]benzyl}amino)(phenyl)methylene]-2-oxo-*N*-[(1*R*)*-*1-phenylpropyl]indoline-5-carboxamide |
| 245 | (3*Z*)-3-[({3-[(ethylcarbamoyl)amino]benzyl}amino)(pyridin-3-yl)methylene]-2-oxo-*N*-[(1*R*)-1-phenylethyl]indoline-5-carboxamide |
| 246 | (3*Z*)-3-{[(3-{[(3-chlorophenyl)carbamoyl]amino}benzyl)amino](pyridin-3-yl)methylene}-2-oxo-*N*-[(1*R*)-1-phenylethyl]indoline-5-carboxamide |
| 247 | (3*Z*)-3-[{[3-(2,5-dioxoimidazolidin-1-yl)benzyl]amino}(pyridin-3-yl)methylene]-2-oxo-*N*-[(1*R*)-1-phenylethyl]indoline-5-carboxamide |
| 248 | (3*Z*)-3-[({3-[(4Z)-2,5-dioxo-4-(pyridin-4-ylmethylene)imidazolidin-1-yl]benzyl}amino)(pyridin-3-yl)methylene]-2-oxo-*N*-[(1*R*)-1-phenylethyl]indoline-5-carboxamide |
| 249 | (3*Z*)-*N*-benzyl-3-[{[4-({[(3-chlorophenyl)amino]carbonothioyl}amino)benzyl]amino} (phenyl)methylene]-2-oxoindoline-5-carboxamide |
| 250 | (3*Z*)-*N*-benzyl-3-[{[4-(2,5-dioxoimidazolidin-1-yl)benzyl]amino}(phenyl)methylene]-2-oxoindoline-5-carboxamide |
| 251 | (3Z)-3-[{[4-(2,5-dioxoimidazolidin-1-yl)benzyl]amino}(phenyl)methylene]-2-oxo-*N*-[(1*R*)-1-phenylethyl]indoline-6-carboxamide |
| 252 | (3*Z*)-3-[({4-[(4Z)-2,5-dioxo-4-(pyridin-4-ylmethylene)imidazolidin-1-yl]benzyl}amino)(phenyl)methylene]-2-oxo-*N*-[(1*R*)-1-phenylethyl]indoline-6-carboxamide |
| 253 | (3*Z*)-*N*-benzyl-3-[({4-[(4Z)-2,5-dioxo-4-(pyridin-4-ylmethylene)imidazolidin-1-yl]benzyl}amino)(phenyl)methylene]-2-oxoindoline-5-carboxamide |
| 254 | (3Z)-3-{[(2-{2-[(ethylcarbamoyl)amino]phenyl}ethyl)amino](phenyl)methylene}-2-oxo-*N-*[(1*R*)-1-phenylpropyl]indoline-7-carboxamide |
| 255 | (3Z)-3-[{[2-(2-{[(3-chlorophenyl)carbamoyl]amino}phenyl)ethyl]amino}(pyridin-4-yl)methylene]-2-oxo-*N*-[(1*S*)-1-phenylpropyl]indoline-7-carboxamide |
| 256 | (3Z)-3-{[(2-{3-[(ethylcarbamoyl)amino]phenyl}ethyl)amino](phenyl)methylene}-2-oxo-*N-*[(1*R*)-1-phenylethyl]indoline-5-carboxamide |
| 257 | (3Z)-3-{[(2-{3-[(ethylcarbamoyl)amino]phenyl}ethyl)amino](phenyl)methylene}-2-oxo-*N-*[(1*S*)-1-phenylethyl]indoline-5-carboxamide |
| 258 | (3Z)-3-[{[2-(3-{[(3-chlorophenyl)carbamoyl]amino}phenyl)ethyl]amino}(phenyl)methylene]-2-oxo-*N*-[(1*R*)-1-phenylethyl]indoline-5-carboxamide |
| 259 | (3*Z*)-*N*-benzyl-3-{[(2-{3-[(4Z)-2,5-dioxo-4-(pyridin-4-ylmethylene)imidazolidin-1-yl]phenyl}ethyl)amino](phenyl)methylene}-2-oxoindoline-5-carboxamide |
| 260 | (3Z)-3-{[(2-{4-[(ethylcarbamoyl)amino]phenyl}ethyl)amino](phenyl)methylene}-2-oxo-*N-*[(1*R*)-1-phenylethyl]indoline-5-carboxamide |
| 261 | (3*Z*)-3-[{[2-(4-{[(3-chlorophenyl)carbamoyl]amino}phenyl)ethyl]amino} (phenyl)methylene]-2-oxo-*N*-[(1*R*)-1-phenylethyl]indoline-5-carboxamide |
| 262 | (3*Z*)-3-{[(3S)-1-azabicyclo[2.2.2]oct-3-ylamino](phenyl)methylene}-2-oxo*N*-[(1*R*)-1-phenylethyl]indoline-5-carboxamide |
| 263 | (3*Z*)-3-{[(3R)-1-azabicyclo[2.2.2]oct-3-ylamino](phenyl)methylene}-2-oxo*N*-[(1*R*)-1-phenylethyl]indoline-5-carboxamide |
| 264 | (3*Z*)-3-{[(3R)-1-azabicyclo[2.2.2]oct-3-ylamino](phenyl)methylene}-*N-*benzyl-2-oxoindoline-5-carboxamide |
| 265 | (3*Z*)-3-{[(3S)-1-azabicyclo[2.2.2]oct-3-ylamino](phenyl)methylene}-2-oxo-*N*-[(1*R*)-1-phenylethyl]indoline-5-carboxamide |
| 266 | (3*Z*)-3-{[(3*R*)-1-azabicyclo[2.2.2]oct-3-ylamino](phenyl)methylene}-2-oxo*N*-[(1*R*)-1-phenylethyl]indoline-5-carboxamide |
| 267 | (3*Z*)-3-{[(3S)-1-azabicyclo[2.2.2]oct-3-ylamino](phenyl)methylene}-2-oxo*N*-[(1*R*)-1-phenylpropyl]indoline-6-carboxamide |
| 268 | (3Z)-3-{[(3R)-1-azabicyclo[2.2.2]oct-3-ylamino](pyridin-4-yl)methylene}-2-oxo-*N*-[(1*R*)-1-phenylpropyl]indoline-6-carboxamide |
| 269 | (3Z)-3-{[(3R)-1-azabicyclo[2.2.2]oct-3-ylamino](3-thienyl)methylene}-N-benzyl-2-oxoindoline-6-carboxamide |

and prodrugs, tautomers, hydrates, solvates of the above mentioned compounds and pharmaceutically acceptable salts thereof, for the use in the treatment of infectious diseases and cancer.

### Chemical synthesis

The inventive compounds **(I)** according to the present invention can be prepared by methods known to one skilled in the art. The synthesis is preferably carried out according to the general synthetic routes, shown in **scheme 1.**

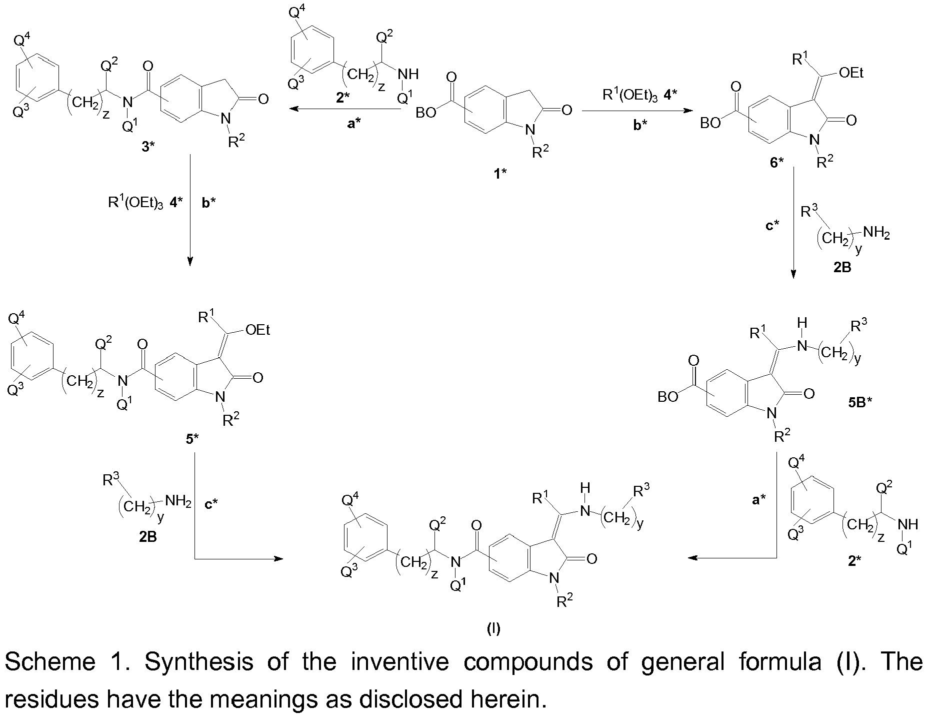

Compound 3* can be synthesised from the commercially available carboxy-oxindoles 1* and commercially available amines 2* via amide bond formation a*. Conveniently, many amines of general formula 2* are commercially available or can be easily prepared by a person skilled in the art following known synthetic procedures that are disclosed in patent or non-patent literature. Examples of suitable amines are: (1*R*)-1-(4-chlorophenyl)ethanamine, (1*R*)-1-phenylpropan-1-amine, (1*R*)-1- phenylethanamine, (1*S*)-1-phenylethanamine, (1*R*)-1-(4-methoxyphenyl)-ethanamine, (1*R*)-1-(3-methoxyphenyl)-ethanamine, (1*R*)-1-cyclohexylethanamine (1*R*)-1-(3-chlorophenyl)-ethanamine, (1*R*)-1-(2-naphthyl)ethanamine, (1*S*)-indan-1-amine, (1*R*)-indan-1-amine, (1*R*)-*N*-methyl-1-phenylethanamine, (1*R*)-1-(4-methylphenyl)-ethanamine, (1*S*)-1-(4-methylphenyl)-ethanamine, (1R)-1-(4-chlorophenyl)-ethanamine, (1*R*)-1-(3-bromophenyl)-ethanamine, (1*R*)-1-(3-chlorophenyl)propan-1-amine, (1*R*)-1-(4-chlorophenyl)propan-1-amine, (1*R*)-1-(3,4-difluorophenyl)propan-1-amine, 1-(4-methoxyphenyl)propan-1-amine,, (1*R*)-1-(4-methoxyphenyl)propan-1-amine, (1*R*)-1-(4-methylphenyl)propan-1-amine, (1*R*)-1-(3-methylphenyl)propan-1-amine, (1*R*)-1-(3-methoxyphenyl)propan-1-amine, etc.

Amide bond formation involves activation of the carboxylic group, followed by nucleophile attack of the amino group. Activation of the carboxylic acid includes conversion of the carboxylic acid to the corresponding active ester, carboxyl chloride or bromide, or anhydride. For facility reasons, the activation of the carboxylic acid performed *in situ* by treatment of the mixture of carboxylic acid and amine with an activating agent. Activating agents include carbodiimides such as DCC (N,N'-dicyclohexylcarbodiimide), DIC (N,N'-diisopropylcarbodiimide) and EDCI (N-(3-dimethylaminopropyl)-N'-ethylcarbodiimide hydrochloride), phosphonium salts such as BOP (benzotriazol-1-yloxy)tris(dimethylamino)phosphonium hexafluorophosphate, PyBOP (benzotriazol-1-yloxy)tripyrrolidinophosphonium 5 hexafluorophosphate, PyAOP ((7-azabenzotriazol-1-yloxy)tripyrrolidinophosphonium hexafluorophosphate), BroP (bromotris(dimethylamino)phosphonium hexafluorophosphate), PyCloP (chlorotripyrrolidinophosphonium hexafluorophosphate), DEPBT (3-(diethoxyphosphoryloxy)-1,2,3-benzotriazin-4(3H)-one), uronium and guanidinium salts such as HBTU (*O*-(benzotriazol-1-yl)-N,N,N',N'-tetramethyluronium hexafluorophosphate), TBTU (*O*-(Benzotriazol-1-yl)-N,N,N',N'-tetramethyluronium tetrafluoroborate), HATU (*O*-(7-azabenzotriazol-1-yl)-N,N,N',N'-tetramethyluronium hexafluorophosphate), HBPyU (O-(benzotriazol-1-yl)-N,N,N',N'-bis(tetramethylene)uranium hexafluorophosphate), HCTU (0-(6-chlorobenzotriazol-1-yl)-N,N,N',N'- 8 tetramethyluronium hexafluorophosphate). Amide bond formation is conducted preferably in presence of a base, such as tertiary amines including TEA (triethylamine) and (DIPEA) diisopropylethyl amine. Thus, the amide bond formation can be carried out using reagent systems such as HATU/DIPEA, HBTU/TEA or EDCI/HOBT/TEA.

Compound 5* can be made from 3* with commercially available trimethoxymethyl or triethoxymethyl derivatives 4*, such as (trimethoxymethyl)benzene, (triethoxymethyl)benzene, 2-(trimethoxymethyl)pyridine, 2-(triethoxymethyl)pyridine, 3-(trimethoxymethyl)pyridine, 3-triethoxymethyl)pyridine, 4-trimethoxymethyl)pyridine, 4-triethoxymethyl)pyridine, 2-(trimethoxymethyl)thiophene, 2-(triethoxymethyl)thiophene, 3-trimethoxymethyl)thiophene, 3-(triethoxymethyl)thiophene, by reacting them via an aldol reaction b* at reflux, in acetic anhydride or in the mixture of acetic anhydride and other appropriate solvent such as THF (tetrahydrofurane), dioxane, DMF (dimethylformamide), MeOH (methanol), EtOH (ethanol), propanol, *iso*propanol, butanol, *tert*-BuOH, forming an α,β-unsaturated vinyl ether.

Reacting the vinyl ether 5* with anilines and amines, in the presence of DMF, MeOH, EtOH, DMSO (dimethyl sulfoxid), or in their appropriate mixtures, via reaction c* gives the inventive compounds (I).

Anilines and amines are commercially available or can be easily prepared by a person skilled in the art following known synthetic procedures that are disclosed in patent or non-patent literature. Some examples for the anilines and amines are: 4-[(dimethylamino)methyl]aniline, 3-[(dimethylamino)methyl]aniline, 4-[(diethylamino)methyl]aniline, 3-[(diethylamino)methyl]aniline, 4-(piperidin-1-ylmethyl)aniline, 3-(piperidin-1-ylmethyl)aniline, 2-(piperidin-1-ylmethyl)aniline, 4-(pyrrolidin-1-ylmethyl)aniline, 3-(pyrrolidin-1-ylmethyl)aniline, 2-(pyrrolidin-1-ylmethyl)aniline, 4-[(4-methylpiperazin-1-yl)methyl]aniline, 3-[(4-methylpiperazin-1-yl)methyl]aniline, 2-[(4-methylpiperazin-1-yl)methyl]aniline, 2-[4-(3-aminobenzyl)piperazin-1-yl]ethanol, 2-[4-(3-aminobenzyl)piperazin-1-yl]ethanol, 2-[4-(4-aminobenzyl)piperazin-1-yl]ethanol, 2-[4-(2-aminobenzyl)piperazin-1-yl]ethanol, 4-(morpholin-4-ylmethyl)aniline, 3-(morpholin-4-ylmethyl)aniline, 2-(morpholin-4-ylmethyl)aniline, [1-(4-aminobenzyl)piperidin-2-yl]methanol, [1-(3-aminobenzyl)piperidin-2-yl]methanol, [1-(2-aminobenzyl)piperidin-2-yl]methanol, 3-(thiomorpholin-4-ylmethyl)aniline, 2-(thiomorpholin-4-ylmethyl)aniline, 4-(thiomorpholin-4-ylmethyl)aniline, *N*-(4-aminophenyl)-2-piperidin-1-ylacetamide, *N-*(3-aminophenyl)-2-piperidin-1-ylacetamide, *N*-(2-aminophenyl)-2-piperidin-1-ylacetamide, *N*-(3-aminophenyl)-*N*-methyl-2-piperidin-1-ylacetamide, *N-*(4-aminophenyl)-*N*-methyl-2-piperidin-1-ylacetamide, N-(2-aminophenyl)-N-methyl-2-piperidin-1-ylacetamide, *N*-(4-aminophenyl)-*N*-methyl-2-(4-methylpiperazin-1-yl)acetamide, *N*-(3-aminophenyl)-*N*-methyl-2-(4-methylpiperazin-1-yl)acetamide, *N*-(2-aminophenyl)-*N*-methyl-2-(4-methylpiperazin-1-yl)acetamide, *N*-(3-aminophenyl)-2-[4-(2-hydroxyethyl)piperazin-1-yl]-*N*-methylacetamide, *N*-(4-aminophenyl)-2-[4-(2-hydroxyethyl)piperazin-1-yl]-*N*-methylacetamide, *N*-(3-aminophenyl)-*N-*methyl-2-pyrrolidin-1-ylacetamide, *N*-(4-aminophenyl)-N-methyl-2-pyrrolidin-1-ylacetamide, *N*-(4-aminophenyl)-2-pyrrolidin-1-ylacetamide, etc. 1-[2-(aminomethyl)phenyl]-3-ethylurea, 1-[2-(aminomethyl)phenyl]-3-phenylurea, 1-[2-(aminomethyl)phenyl]-3-(3-chlorophenyl)urea, 1-[2-(aminomethyl)phenyl]-3-(4-hydroxyphenyl)urea, 1-[3-(aminomethyl)phenyl]-3-ethylurea, 1-[3-(aminomethyl)phenyl]-3-phenylurea, 1-[3-(aminomethyl)phenyl]-3-(3-chlorophenyl)urea, 1-[3-(aminomethyl)phenyl]-3-(4-hydroxyphenyl)urea, 1-[3-(aminomethyl)phenyl]-3-(1,3-benzodioxol-5-yl)urea, 1-[4-(aminomethyl)phenyl]-3-ethylurea, 1-[4-(aminomethyl)phenyl]-3-phenylurea, 1-[4-(aminomethyl)phenyl]-3-(3-chlorophenyl)urea, 1-[4-(aminomethyl)phenyl]-3-(4-hydroxyphenyl)urea, 1-[2-(2-aminoethyl)phenyl]-3-ethylurea, 1-[2-(2-aminoethyl)phenyl]-3-phenylurea, 1-[2-(2-aminoethyl)phenyl]-3-(3-chlorophenyl)urea, 1-[2-(2-aminoethyl)phenyl]-3-(4-hydroxyphenyl)urea, 1-[3-(2-aminoethyl)phenyl]-3-ethylurea, 1-[3-(2-aminoethyl)phenyl]-3-phenylurea, 1-[3-(2-aminoethyl)phenyl]-3-(3-chlorophenyl)urea, 1-[3-(2-aminoethyl)phenyl]-3-(4-hydroxyphenyl)urea, 1-[3-(2-aminoethyl)phenyl]-3-(1,3-benzodioxol-5-yl)urea, 1-[4-(2-aminoethyl)phenyl]-3-ethylurea, 1-[4-(2-aminoethyl)phenyl]-3-phenylurea, 1-[4-(2-aminoethyl)phenyl]-3-(3-chlorophenyl)urea, 1-[4-(2-aminoethyl)phenyl]-3-(4-hydroxyphenyl)urea, (3S)-quinuclidin-3-amine, (3R)-quinuclidin-3-amine. The order of reaction a* and b* during the synthesis of 5* is reversible, as shown in scheme 1. If one would chose the other synthetic route, than one should react 1*(oxindole carboxylic acid or oxindole carboxylic acid ester) with 4* in reaction b* first, gaining compound 6*. Reacting the vinyl ether 6* with anilines and amines in the presence of DMF, MeOH, EtOH, DMSO (dimethyl sulfoxid), or in their appropriate mixtures, via reaction c* gives **5B.**

In addition, the inventive compounds **(I)** according to the present invention can also (but not limited to) be prepared according to the general synthetic routes, shown in **Scheme 2.**

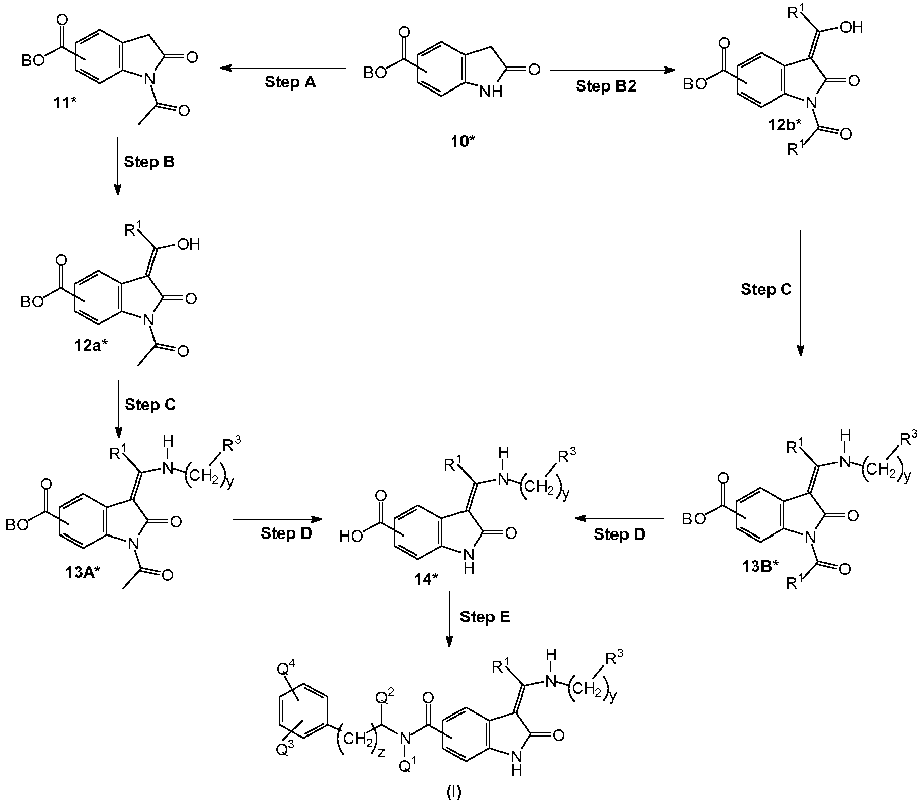

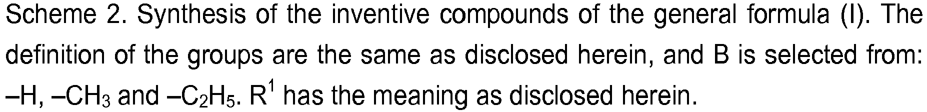

Compound **11*** can be synthesised from the commercially available carboxy-oxindoles (acid or ester) **10*** according to procedure **A.** In procedure **B** or **B2** (aldol-reaction) compound **12a,b*** can be prepared from **10*** and **11*** reacted with commercially avaible acyl-derivatives, especially acyl-chlorides: isonicotinoyl chloride, nicotinoyl chloride, thiophene-3-carbonyl chloride, thiophene-2-carbonyl chloride, 1-methyl-1H-pyrazole-4-carbonyl chloride, 1-methyl-1H-indole-5-carbonyl chloride, 3-furoyl chloride, 2-furoyl chloride, cyclohexanecarbonyl chloride, cyclopropanecarbonyl chloride, propanoyl chloride, 2-methylpropanoyl chloride, etc. in the presence of N,N-dimethylpyridin-4-amine and TEA or DIEA in any aprotic solvents, especially dichloromethane, chloroform or dichororoethane.

Compounds **12b*** were obtained via procedure **B2,** where 2 molequivalent activated acyl-derivatives are needed for simultenously protection of acyl NH. Compound **13A*** and **13B*** can be obtained by activation of vinyl-OH and reacted by amines (N-C bond formation) in procedure **c***. Amide bond formation involves activation of the hydroxy group by known methods via silylation, especially chlorotrimethylsilane, bromotrimethylsilane, iodotrimethylsilane, via mesylation by methanesulfonyl chloride or activation by trifluoromethanesulfonic anhydride in any aprotic solvent like dioxane, tetrahydrofurane.

In some cases under condition of this reaction, N-acyl groups were cleaved from the oxindole NH, giving the desired **14*** compounds. In other cases, N-acyl type protecting groups must be cleaved under acidic (especially aqueous HCl) or basic (especially aqueous LiOH, NaOH, or KOH) conditions (procedure **D**)

In procedure **E** the activation of the carboxylic acid performed *in situ* by treatment of the mixture of the amine and compound **14*** with an activating agents, such as DCC (*N,N*'-dicyclohexylcarbodiimide), DIC (*N,N*'-diisopropylcarbodiimide) and EDCI (N-(3-dimethylaminopropyl)-N'-ethylcarbodiimide hydrochloride), phosphonium salts such as BOP (benzotriazol-1-yloxy)tris(dimethylamino)phosphonium hexafluorophosphate, PyBOP (benzotriazol-1-yloxy)tripyrrolidinophosphonium 5 hexafluorophosphate, PyAOP ((7-azabenzotriazol-1-yloxy)tripyrrolidinophosphonium hexafluorophosphate), BroP (bromotris(dimethylamino)phosphonium hexafluorophosphate), PyCloP (chlorotripyrrolidinophosphonium hexafluorophosphate), DEPBT (3-(diethoxyphosphoryloxy)-1,2,3-benzotriazin-4(3H)-one), uronium and guanidinium salts such as HBTU (*O*-(benzotriazol-1-yl)-N,N,N',N'-tetramethyluronium hexafluorophosphate), TBTU (*O*-(Benzotriazol-1-yl)-N,N,N',N'-tetramethyluronium tetrafluoroborate), HATU (O-(7-azabenzotriazol-1-yl)-N,N,N',N'-tetramethyluronium hexafluorophosphate), HBPyU (O-(benzotriazol-1-yl)-N,N,N',N'-bis(tetramethylene)uranium hexafluorophosphate), HCTU (O-(6-chlorobenzotriazol-1-yl)-N,N,N',N'- 8 tetramethyluronium hexafluorophosphate). Amide bond formation is conducted preferably in presence of a base, such as tertiary amines including TEA (triethylamine) and (DIPEA) diisopropylethyl amine. Thus, the amide bond formation can be carried out using reagent systems such as HATU/DIPEA, HBTU/TEA or EDCI/HOBT/TEA.

Acylation of NH of the benzopyrrolidones was carried out by acylation agents (especially acetic-anhydride) at 80 °C - 140 °C obtained the compounds covered by general formula (I).

The present invention refers further to use of the compounds according to the general formula (I) are in the manufacture of a medicament for the treatment of infectios diseases. Another aspect of the present invention relates to a method comprising administering to a patient suffering from an infectious disease a therapeutically effective amount of at least one compound according to general formula (I) as defined herein in order to treat that infectious disease.

As used herein the term infectious diseases comprises selected from the group comprising or consisting of virally induced infectious diseases, bacterially induced infectious diseases, and parasitic induced infectious diseases. Within the present invention it is preferred that the infectious diseases are virally induced infectious diseases, including opportunistic diseases.

A preferred embodiment of the present invention refers to the compounds of general formula I for use in the treatment of virally induced infectious diseases, including opportunistic diseases, being infections with dsDNA viruses, ssDNA viruses, (+)ssRNA viruses, (-)ssRNA viruses, ssRNA-RT viruses, or dsDNA-RT viruses. Within the present invention it is preferred that the infection with (-)ssRNA viruses are an infection with Orthomyxoviridae, Bornaviridae, Filoviridae, Paramyxoviridae, or Rhabdoviridae.

Another aspect of the present invention refers to the compounds of general formula I for use in the treatment of an infection with Orthomyxoviridae selected from the group comprising or consisting of an infection with Influenza virus A, Influenza virus B, or Influenza virus C. Especially preferred are compounds of general formula I for use in the treatment of an infection with Influenza virus A.

The term "opportunistic diseases" as used herein refers to infections caused by pathogens, particularly opportunistic pathogens that usually do not cause disease or symptoms in a healthy host, one with a healthy immune system but in a host with a damaged or weakened immune system. This is because a healthy immune system is able to successfully fight off the disease, or keep it under control. Examples for opportunistic diseases are caused by an infection with aspergillus sp., candida albicans, cytomegalovirus, polyomavirus, kaposi's sarcoma caused by human herpesvirus 8 (HHV8), pseudomonas aeruginosa, staphylococcus aureus, streptococcus pneumonia and toxoplasma gondii.

Another preferred embodiment of the present invention refers to compounds of the general formula (I) for the use in treatment of cancer, wherein cancer is selected from the group consisting of adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer, breast cancer, Burkitt's lymphoma, corpus cancer, colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors, brain metastases, testicle cancer, hypophysis tumor, carcinoids, Kaposi's sarcoma, laryngeal cancer, germ cell tumor, bone cancer, colorectal carcinoma, head and neck tumors, colon carcinoma, craniopharyngiomas, oral cancer, cancer of the central nervous system, liver cancer, liver metastases, leukemia, eyelid tumor, lung cancer, lymph node cancer, lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, nasal cancer, neurinoma, neuroblastoma, kidney cancer, renal cell carcinomas, non-Hodgkin's lymphomas, oligodendroglioma, esophageal carcinoma, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, esophageal cancer, spinalioms, T-cell lymphoma, thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. A more preferred embodiment of the present invention refers to compounds of the general formula (I) for the use in treatment of cancer, wherein the cancer is a colorectal cancer.

Still another aspect of the present invention deals with pharmaceutical compositions comprising at least one compound according to general formula (I) as an active ingredient, together with at least one pharmaceutically acceptable carrier, excipient and/or diluents. The pharmaceutical compositions of the present invention can be prepared in a conventional solid or liquid carrier or diluents and a conventional pharmaceutically-made adjuvant at suitable dosage level in a known way. The preferred preparations are adapted for oral application or for injection. These administration forms include, for example, liquids, pills, tablets, film tablets, coated tablets, capsules, liposomal formulations, micro- and nano-formulations, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound as described herein and/or a pharmaceutical acceptable salt thereof as active ingredient.

The pharmaceutical compositions according to the present invention containing at least one compound as described in the present invention, especially one pure optical isomer, and/or a pharmaceutically acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are gels, elixirs, dispersable granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

As pharmaceutically acceptable carrier, excipient and/or diluents can be used carriers such as preferably with an inert carrier like lactose, starch, sucrose, cellulose, magnesium stearate, dicalcium phosphate, calcium sulfate, talc, mannitol, ethyl alcohol (liquid filled capsules).

Suitable binders include starch, gelatin, natural sugars, corn sweeteners, natural and synthetic gums such as acacia, sodium alginate, carboxymethylcellulose, polyethylene glycol and waxes, sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate.

Suitable lubricants are selected from the group comprising boric acid, sodium benzoate, sodium acetate, sodium chloride, magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine.

Disintegrating agents (disintegrates) such as starch, methylcellulose, guar gum, modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures may be added.

Further suitable additives are coloring agents, sweetening agents, flavoring agents, preservatives, glidents such as silicon dioxide and talc, and adsorbent such as clay, aluminum oxide.

Suitable diluents are water or water/propylene glycol solutions for parenteral injections, juice, sugars such as lactose, sucrose, mannitol, and sorbitol, starches derived from wheat, corn rice, and potato, and celluloses such as microcrystalline cellulose.

Compounds of general formula (I) may be administered as the sole active agent or in combination with one or more additional therapeutic agents, wherein the combination causes no unacceptable adverse effects. For example, a compound of general formula (I) and an active agent may be administered to the patient together in a single oral dosage composition such as a tablet or capsule, or each agent may be administered in separate dosage formulations. Where separate dosage formulations are used, the compound of general formula (I) and one or more additional therapeutic agents may be administered at essentially the same time (e.g., concurrently) or at separately staggered times (e.g., sequentially). In particular, the compounds of the present invention may be used in fixed or separate combination with other anti-viral agents such as interferons, acyclovir, neuraminidase inhibitors (oseltamivir and zanamivir) and M2 protein inhibitors (rimantadine).

Thus, another aspect of the present invention relates to drug combinations comprising at least one compound according to general formula (I) and/or pharmaceutically acceptable salts thereof together with at least one additional anti-viral agent, especially at least one of the active agents mentioned above.

### Description of the figures:

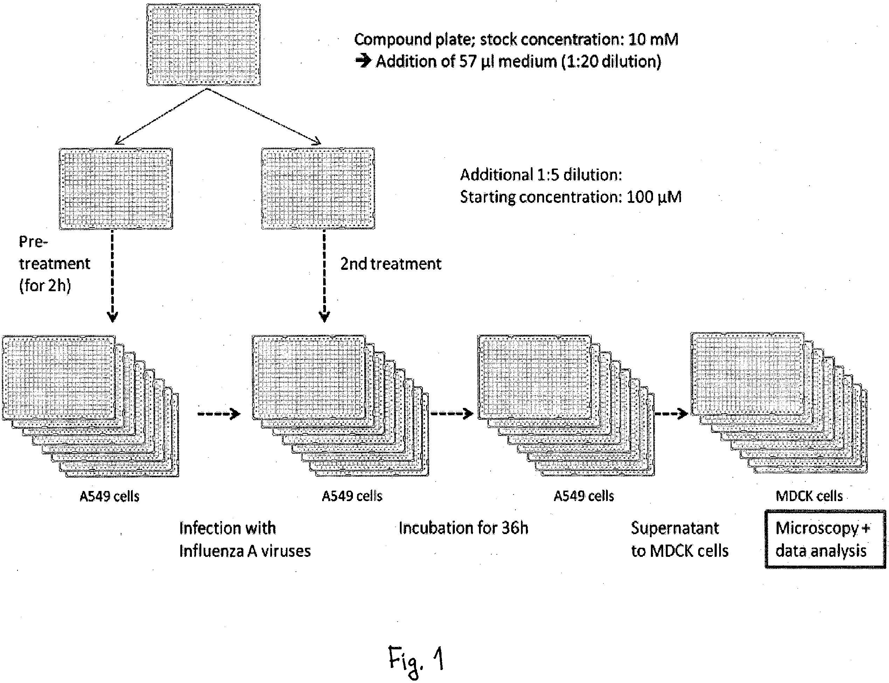

- Figure 1:: shows an overview of the virus screening process to identify chemical compounds that inhibit influenza virus replication

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Part A: Chemical Synthesis,

### Genaral chemical procedures

All reagents were commercial grade and were used as received without further purification, unless otherwise specified. Reagent grade solvents were used in all other cases, unless otherwise specified, respectively. Silica gel generally used for column chromatography was Fisher silica gel (60A, 35-70 micron). Thin layer chromatography was carried out using pre-coated silica gel F-254 plates (thickness 0.25 mm). The ¹H-NMR spectra were recorded on a Bruker Avance 300 NMR spectometer controlled by TopSpin software package in deuterodimethylsulfoxide (DMSO-*d*₆) solution at 30 °C. The data were processed by TopSpin software. The chemical shifts are referred to tetramethylsilane (δ_{TMS} = 0 ppm).The chemical shifts are expressed in ppm using the residual solvent as internal standard. Splitting patterns are designated as s (singlet), d (doublet), dd (double-doublet), t (triplet), q (quartet), quint (quintet), m (multiplet), and bs (broad singlet). Electrospray MS spectra were obtained on a Waters LC-MS system Acquity SQD detector, Waters 2795 Alliance HPLC equipped with Waters 996 PDA Detector (HPLC column: Waters XBridge, RP C18, 3.5 µm, 4.6 mm x 50 mm; gradient MeCN/H2O, containing 0.1% HCOOH: 5% MeCN (0.5 min), 5% to 95% MeCN (5 min), 95% MeCN (0.5 min); flow rate: 2.0 ml/min.

### Example 1:

### Step A: (3Z)-1-acetyl-3-[ethoxy(phenyl)methylene]-2-oxoindoline-5-carboxylic acid

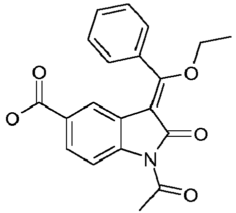

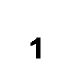

A mixture of 2-oxoindoline-5-carboxilic acid (5.221 g, 29.50 mmol) and triethylorthobenzoate (20.16 g, 90 mmol) in acetic anhydride (75 ml) were stirred at 100 °C for 8 h.The solvent was removed under reduced pressure. The residue was stirred with i-Pr₂O (250 ml) for 2 h to give a solid which was filtered and washed with i-Pr₂O to provide (3*Z*)-1-acetyl-3-[ethoxy(phenyl)methylene]-2-oxoindoline-5-carboxylic acid (3.5 g, 33 %) ¹H-NMR in DMSO-*d*₆ 12.3(bs,1H); 8.61(d,1H); 8.22(d,1H); 7.90(dd,1H); 7.54(m,5H); 3.99(q,2H); 2.45(s,3H); 1.35(t,3H). t_{R}: 3.82 min, MS(ESI):m/z (M+H)⁺352^{,} (M+H)⁻ 350.

The following compound was obtained according to the procedure described by **example1, step A:**

### (3Z)-1-acetyl-3-[ethoxy(phenyl)methylene]-2-oxoindoline-6-carboxylic acid

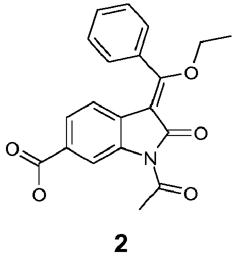

Starting from 2-oxoindoline-6-carboxilic acid and triethylorthobenzoate compound 2 was obtained (3.8 g, 36 %).

### Step B: (3Z)-2-oxo-3-(phenyl{[3-(piperidin-1-ylmethyl)phenyl]amino}methylene) indoline-5-carboxylic acid

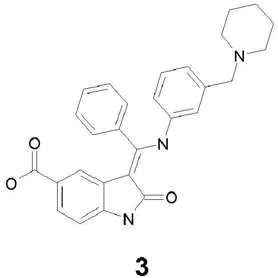

C₂₈H₂₇N₃O₃, Mw calc: 453.2

A mixture of compound (3*Z*)-1-acetyl-3-[ethoxy(phenyl)methylene]-2-oxoindoline-5-carboxylic acid (4.35g ,12.39 mmol) and 3-(piperidine-1-ylmethyl)aniline (2.47 g, 13 mmol) in dry DMF were stirred at 100 C for 6 h. After cooling down the reaction mixture, MeOH (70 ml) and NaOMe (30 % wt solution in MeOH, 5 ml) were added and the reaction mixture was further stirred overnight. The resulting solid was filtered and washed with cold MeOH to provide (3Z)-2-oxo-3-(phenyl{[3-(piperidin-1-ylmethyl)phenyl]amino}methylene) indoline-5-carboxylic acid (3.27 g, 58 %)
¹H-NMR in DMSO-*d*₆ δ: 12.01 (s, 1 H), 10.71 (s, 1H), 7.55 (dd, *J* = 7.9 and 1.5 Hz, 1 H), 7.47-7.54 (ovl. m, 3H), 7.44 (m, 2H), 7.08 (dd, *J* = 7.8 and 7.6 Hz, 1 H), 6.89 (dm, *J* = 7.6 Hz, 1 H), 6.78 (d, *J* = 1.5 Hz, 1 H), 6.85 (d, *J* = 8.1 Hz, 1 H), 6.68 (dm, *J* = 7.8 Hz, 1 H), 6.61 (dd, *J* ∼ 2.5 and 2.0 Hz, 1 H), 3.18 (s, 2H), 2.10 (m, 4H), 1.42 (m, 4H), 1.35 (m, 2H)
t_{R}: 2.51 min; MS(ESI): m/z (M+H)⁺454; (M+H)⁻ 452.

The following compounds were obtained according to the procedure described by **example 1, step B:**

### (3Z)-2-oxo-3-(phenyl{[4-(piperidin-1-ylmethyl)phenyl]amino}methylene)indoline-5-carboxylic acid

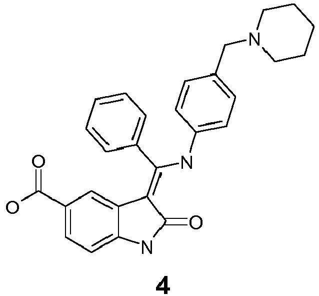

Compound **4** (3.17 g, 56%) was obtained satrting from (3Z)-1-acetyl-3-[ethoxy(phenyl)methylene]-2-oxoindoline-5-carboxylic acid and 4-(piperidine-1-ylmethyl)aniline:
C₂₈H₂₇N₃O₃, Mw calc: 453.2
¹H-NMR in DMSO-*d*₆ δ: 12.02 (s, 1 H), 11.01 (s, 1H), 7.56 (dd, *J* = 8.1 and 1.5 Hz, 1 H), 7.50-7.62 (ovl. m, 3H), 7.47 (ovl. m, 2H), 7.05 (dm, *J* = 8.3 Hz, 2H), 6.91 (d, *J* = 8.1 Hz, 1H), 6.76 (dm, *J* = 8.3 Hz, 2H), 6.58 (d, *J* = 1.5 Hz, 1H), 3.28 (s, 2H), 2.22 (m, 4H), 1.43 (m, 4H), 1.36 (m, 2H)
t_{R}: 2.48 min ; MS(ESI): m/z (M+H)⁺454; (M+H)⁻ 452.

### (3Z)-3-[({4-[(4-methylpiperazin-1-yl)methyl]phenyl}amino)(phenyl)methylene]-2-oxoindoline-5-carboxylic acid

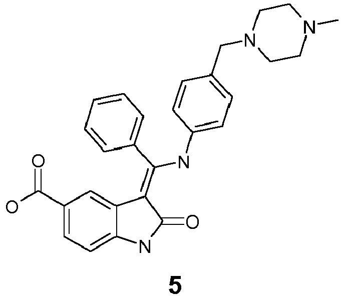

Compound 5 (3.24 g, 55 %) was obtained from (3*Z*)-1-acetyl-3-[ethoxy(phenyl) methylene]-2-oxoindoline-5-carboxylic acid and 4-[(4-methylpiperazin-1-yl)methyl]aniline:
C₂₈H₂₈N₄O₃, Mw calc: 468.2
¹H-NMR in DMSO-*d*₆ δ: 12.02 (s, 1 H), 10.99 (s, 1 H), 7.55 (dd, *J* = 8.1 and 1.5 Hz, 1 H), 7.51-7.62 (ovl. m, 3H), 7.48 (m, 2H), 7.05 (dm, *J* = 8.2 Hz, 2H), 6.89 (dm, *J =* 8.1 Hz, 1 H), 6.75 (dm, *J* = 8.2 Hz, 2H), 6.60 (d, *J* ∼ 1.5 Hz, 1 H), 3.31 (s, 2H), 2.17-2.38 (m, 8H), 2.11 (s, 3H)
t_{R}: 2.17 min; MS(ESI): m/z (M+H)⁺469; (M+H)⁻467.

Compound **6:** (3*Z*)-3-[({3-[(4-methylpiperazin-1-yl)methyl]phenyl}amino)(phenyl) methylene]-2-oxoindoline-5-carboxylic acid
C₂₈H₂₈N₄O₃, Mw calc: 468.2
MS(ESI): m/z (M+H)⁺469

Compound **7:** (3*Z*)-2-oxo-3-(phenyl{[3-(piperidin-1-ylmethyl)phenyl]amino} methylene)indoline-6-carboxylic acid
C₂₈H₂₇N₃O₃, Mw calc: 453.2
MS(ESI): m/z (M+H)⁺454

Compound **8:** (3*Z*)-2-oxo-3-(phenyl{[4-(piperidin-1-ylmethyl)phenyl]amino} methylene)indoline-6-carboxylic acid
C₂₈H₂₇N₃O₃, Mw calc: 453.2
MS(ESI): m/z (M+H)⁺454

Compound **9:** (3*Z*)-3-[({3-[(4-methylpiperazin-1-yl)methyl]phenyl}amino) (phenyl)methylene]-2-oxoindoline-6-carboxylic acid
C₂₈H₂₈N₄O₃, Mw calc: 468.2
MS(ESI): m/z (M+H)⁺469;

Compound **10:** (3Z)-3-{[(4-{[4-(2-hydroxyethyl)piperazin-1-yl]methyl}phenyl)amino](phenyl)methylene}-2-oxoindoline-5-carboxylic acid
C₂₉H₃₀N₄O₄ MW cal.:498.2
MS(ESI): m/z (M+H)⁺499

Compound **11:** (3*Z*)-3-[({4-[(4-methylpiperazin-1-yl)methyl]phenyl}amino) (phenyl)methylene]-2-oxoindoline-6-carboxylic acid
C₂₈H₂₈N₄O₃, Mw calc: 468.2
MS(ESI): m/z (M+H)⁺469;

### Step C: (3Z)-2-oxo-3-(phenyl{[3-(piperidin-1-ylmethyl)phenyl]amino} methylene)-N-[(1R)-1-phenylpropyl]indoline-5-carboxamide

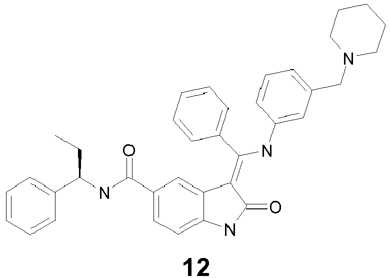

C₃₇H₃₈N₄O₂, Mw calc.: 570.3

A solution of (3*Z*)-2-oxo-3-(phenyl{[3-(piperidin-1-ylmethyl)phenyl]amino} methylene) indoline-5-carboxylic acid (0.113 g, 0.25 mmol), HBTU (0.113 g, 0.30mmol), DIEA (0.116g, 0.9 mmol) and (1 R)-1-phenylpropane-1-amine (0.040g, 0.30 mmol) in dry DMF (3 ml) were stirred overnight. The reaction mixture was poured into saturated sodium carbonate solution((100 ml),) and extracted with EtOAc (3X30 ml).Washed with brine,dried(MgSO4), filtered and concentrated in vacuo. Residue was chromatographed on silica gel eluted with CHCl₃-MeOH-TEA(15:1:0.1) provide the . (3*Z*)-2-oxo-3-(phenyl{[3-(piperidin-1-ylmethyl)phenyl] amino}methylene)-*N*-[(1*R*)-1-phenylpropyl]indoline-5-carboxamide (0.62 mg, 44 %)
¹H-NMR in DMSO-*d*₆ δ: 12.03 (s, 1 H), 10.94 (s, 1 H), 8.05 (d, *J =* 8.5 Hz, 1 H), 7.50-7.57 (ovl. m, 3H), 7.50 (dd, *J =* 8.1 and 1.6 Hz, 1 H), 7.46 (m, 2H), 7.23-7.35 (ovl. m, 4H), 7.20 (m, 1 H), 7.13 (dd, *J* = 7.8 and 7.5 Hz, 1 H), 6.95 (dm, *J* = 7.5 Hz, 1 H), 6.89 (d, *J* = 8.1 Hz, 1 H), 6.77 (dm, *J* = 7.8 Hz, 1 H), 6.71 (m, 1 H), 6.47 (d, *J* = 1.6 Hz, 1 H), 4.73 (ddd, *J* = 8.5, 7.3 and 7.3 Hz, 1 H), 3.30 (s, 2H), 2.14 (m, 4H), 1.61-1.81 (m, 2H), 1.45 (m, 4H), 1.36 (m, 2H), 0.81 (t, *J* = 7.2 Hz, 3H)
t_{R}: 3.3 min; MS(ESI): m/z (M+H)⁺571; (M+H)⁻569.

The following compounds were obtained according to the procedure described by **example 1, step C:**

### (3Z)-2-oxo-N-[(1R)-1-phenylethyl]-3-(phenyl{[3-(piperidin-1-ylmethyl)phenyl] amino}methylene)indoline-5-carboxamide

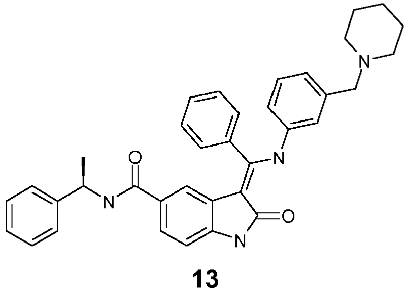

Compound 13 (64 mg, 46%) was obtained starting from (3Z)-2-oxo-3-(phenyl{[3-(piperidin-1-ylmethyl)phenyl]amino}methylene) indoline-5-carboxylic acid and (1*R*)-1-phenylethanamine
C₃₆H₃₆N₄O₂, Mw calc, : 556.2
¹H-NMR in DMSO-*d*₆ δ: 12.03 (s, 1 H), 10.93 (s, 1 H), 8.12 (d, *J =* 7.8 Hz, 1 H), 7.50-7.55 (ovl. m, 3H), 7.50 (dd, *J =* 8.1 and 1.5 Hz, 1 H), 7.46 (m, 2H), 7.24-7.34 (ovl. m, 4H), 7.20 (m, 1 H), 7.12 (dd, *J* = 7.8 and 7.6 Hz, 1 H), 6.93 (dm, *J* = 7.6 Hz, 1 H), 6.88 (d, *J* = 8.1 Hz, 1 H), 6.76 (dm, *J* = 7.8 Hz, 1 H), 6.69 (m, 1 H), 6.46 (d, *J* = 1.5 Hz, 1 H), 4.98 (dq, *J* = 7.8 and 7.0 Hz, 1 H), 3.22 (s, 2H), 2.11 (m, 4H), 1.43 (m, 4H), 1.36 (d, *J* = 7.0 Hz, 3H), 1.35 (m, 2H),
t_{R}: 3.17 min; MS(ESI): m/z (M+H)⁺557; (M+H)⁻555.

### (3Z)-2-oxo-3-(phenyl{[3-(piperidin-1-ylmethyl)phenyl]amino}methylene)-N-[(1S)-1-phenylpropyl]indoline-5-carboxamide

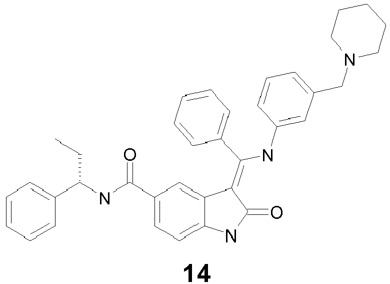

Compound **14** (64 mg, 45%) was obtained starting from (3Z)-2-oxo-3-(phenyl{[3-(piperidin-1-ylmethyl)phenyl]amino}methylene) indoline-5-carboxylic acid and (1*S*)-1-phenylpropan-1-amine
C₃₇H₃₈N₄O₂, Mw calc. : 570.3
¹H-NMR in DMSO-*d*₆ δ: 12.04 (s, 1 H), 10.94 (s, 1 H), 8.06 (d, *J =* 8.5 Hz, 1 H), 7.50-7.55 (ovl. m, 3H), 7.49 (dd, *J* = 8.2 and 1.5 Hz, 1 H), 7.46 (m, 2H), 7.24-7.34 (ovl. m, 4H), 7.20 (m, 1 H), 7.12 (dd, *J* = 7.8 and 7.7 Hz, 1 H), 6.93 (dm, *J* = 7.7 Hz, 1 H), 6.89 (d, *J* = 8.2 Hz, 1 H), 6.75 (dm, *J* = 7.8 Hz, 1 H), 6.68 (dd, *J* ∼ 2.5 and 2.0 Hz, 1 H), 6.47 (d, *J* = 1.5 Hz, 1 H), 4.73 (ddd, *J* = 8.5, 7.2 and 7.2 Hz, 1 H), 3.21 (s, 2H), 2.10 (m, 4H), 1.60-1.80 (m, 2H), 1.43 (m, 4H), 1.34 (m, 2H), 0.81 (t, *J* = 7.2 Hz, 3H)
t_{R}: 3.29 min; MS(ESI): m/z (M+H)⁺571; (M+H)⁻569.

### (3Z)-N-[(1S)-1-(4-chlorophenyl)ethyl]-2-oxo-3-(phenyl{[3-(piperidin-1-ylmethyl) phenyl]amino}methylene)indoline-5-carboxamide

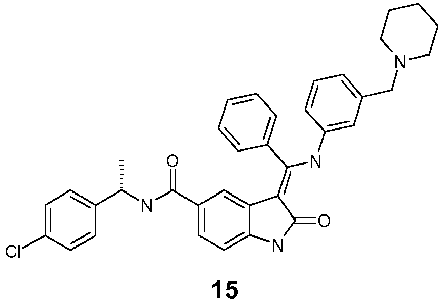

Compound **15** (57 mg, 38%) was obtained starting from (3Z)-2-oxo-3-(phenyl{[3-(piperidin-1-ylmethyl)phenyl]amino}methylene) indoline-5-carboxylic acid and (1*S*)-1-(4-chlorophenyl)ethanamine
C₃₆H₃₅ClN₄O₂ Mw calc.: 590.2
¹H-NMR in DMSO-*d*₆ δ: 12.03 (s, 1 H), 10.94 (s, 1 H), 8.17 (d, *J =* 7.8 Hz, 1 H), 7.49-7.56 (ovl. m, 3H), 7.49 (dd, *J* = 8.1 and 1.5 Hz, 1 H), 7.46 (m, 2H), 7.35 (dm, *J =* 8.5 Hz, 2H), 7.30 (dm, *J* = 8.5 Hz, 2H), 7.12 (dd, *J* = 7.8 and 7.6 Hz, 1 H), 6.92 (dm, *J* = 7.6 Hz, 1 H), 6.88 (d, *J* = 8.1 Hz, 1 H), 6.75 (dm, *J* = 7.8 Hz, 1 H), 6.68 (dd, *J* ∼ 2.5 and 2.0 Hz, 1 H), 6.45 (d, *J* = 1.5 Hz, 1 H), 4.96 (dq, *J* = 7.8 and 7.0 Hz, 1 H), 3.20 (s, 2H), 2.10 (m, 4H), 1.40 (m, 4H), 1.35 (d, *J* = 7.0 Hz, 3H), 1.34 (m, 2H)
t_{R}: 3.37 min; MS(ESI): m/z (M+H)⁺591; (M+H)⁻589.

### (3Z)-N-[(1R)-1-(4-chlorophenyl)ethyl]-2-oxo-3-(phenyl{[3-(piperidin-1-ylmethyl)phenyl]amino}methylene)indoline-5-carboxamide

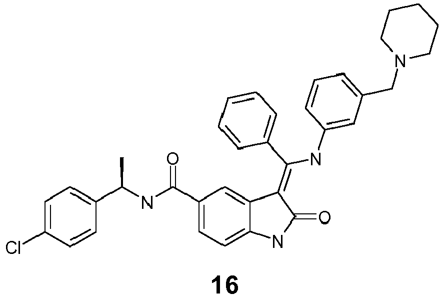

Compound **16** (69 mg, 46%) was obtained starting from (3Z)-2-oxo-3-(phenyl{[3-(piperidin-1-ylmethyl)phenyl]amino}methylene) indoline-5-carboxylic acid and (1*R*)-1-(4-chlorophenyl)ethanamine
C₃₆H₃₅ClN₄O₂, Mw calc. : 590.2
¹H-NMR in DMSO-*d*₆ δ: 12.02 (s, 1 H), 10.94 (s, 1 H), 8.16 (d, *J =* 7.8 Hz, 1 H), 7.49-7.56 (ovl. m, 3H), 7.49 (dd, *J* = 8.1 and 1.5 Hz, 1 H), 7.46 (m, 2H), 7.35 (dm, *J* = 8.5 Hz, 2H), 7.30 (dm, *J* = 8.5 Hz, 2H), 7.12 (dd, *J* = 7.8 and 7.6 Hz, 1 H), 6.92 (dm, *J* = 7.6 Hz, 1 H), 6.88 (d, *J* = 8.1 Hz, 1 H), 6.75 (dm, *J* = 7.8 Hz, 1 H), 6.68 (dd, *J* ∼ 2.5 and 2.0 Hz, 1 H), 6.45 (d, *J* = 1.5 Hz, 1 H), 4.96 (dq, *J* = 7.8 and 7.0 Hz, 1 H), 3.20 (s, 2H), 2.10 (m, 4H), 1.40 (m, 4H), 1.35 (d, *J* = 7.0 Hz, 3H), 1.34 (m, 2H)
t_{R}: 3.35 min; MS(ESI): m/z (M+H)⁺591; (M+H)⁻589.

### (3Z)-N-[(1R)-1-(4-fluorophenyl)ethyl]-2-oxo-3-(phenyl{[3-(piperidin-1-ylmethyl)phenyl]amino}methylene)indoline-5-carboxamide

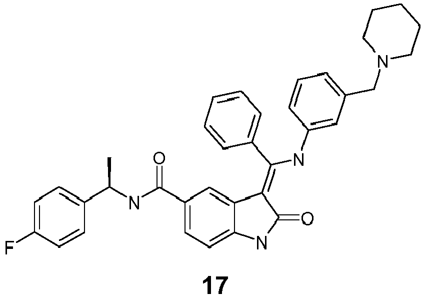

Compound **17** (65 mg, 45%) was obtained starting from (3Z)-2-oxo-3-(phenyl{[3-(piperidin-1-ylmethyl)phenyl]amino}methylene) indoline-5-carboxylic acid and (1*R*)-1-(4-fluorophenyl)ethanamine
C₃₆H₃₅FN₄O₂, Mw calc. : 574.2
¹H-NMR in DMSO-*d*₆ δ: 12.02 (s, 1 H), 10.94 (s, 1 H), 8.14 (d, *J =* 7.8 Hz, 1 H), 7.49-7.57 (ovl. m, 3H), 7.49 (dd, *J* = 8.1 and 1.5 Hz, 1 H), 7.46 (m, 2H), 7.31 (ddm, *J =* 8.6 and 5.6 Hz, 2H), 7.12 (ddm, *J* = 9.0 and 8.6 Hz, 2H), 7.12 (dd, *J* = 7.8 and 7.6 Hz, 1 H), 6.93 (dm, *J* = 7.6 Hz, 1 H), 6.88 (d, *J* = 8.1 Hz, 1 H), 6.76 (dm, *J* = 7.8 Hz, 1 H), 6.69 (dd, *J* ∼ 2.5 and 2.0 Hz, 1 H), 6.46 (d, *J* = 1.5 Hz, 1 H), 4.98 (dq, *J* = 7.8 and 7.0 Hz, 1 H), 3.21 (s, 2H), 2.10 (m, 4H), 1.42 (m, 4H), 1.35 (d, *J =* 7.0 Hz, 3H), 1.34 (m, 2H)
t_{R}: 3.16 min; MS(ESI): m/z (M+H)⁺575; (M+H)⁻573.

### (3Z)-2-oxo-3-(phenyl{[4-(piperidin-1-ylmethyl)phenyl]amino}methylene)-N-[(1R)-1-phenylpropyl]indoline-5-carboxamide

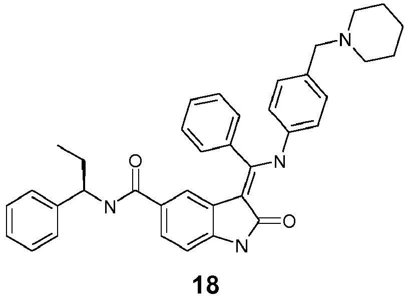

Compound **18** (65 mg, 45%) was obtained starting from (3Z)-2-oxo-3-(phenyl{[4-(piperidin-1-ylmethyl)phenyl]amino}methylene)indoline-5-carboxylic acid and (1R)-1-phenylpropan-1-amine
C₃₇H₃₈N₄O₂, Mw calc.: 570.3
¹H-NMR in DMSO-*d*₆ δ: 12.04 (s, 1 H), 10.92 (s, 1 H), 8.04 (d, *J =* 8.3 Hz, 1 H), 7.49-7.57 (ovl. m, 3H), 7.49 (dd, *J =* 8.1 and 1.5 Hz, 1 H), 7.46 (m, 2H), 7.24-7.34 (ovl. m, 4H), 7.21 (m, 1 H), 7.04 (dm, *J* = 8.2 Hz, 2H), 6.88 (d, *J* = 8.1 Hz, 1 H), 6.75 (dm, *J* = 8.2 Hz, 2H), 6.46 (d, *J* = 1.5 Hz, 1 H), 4.73 (ddd, *J* = 8.3, 7.3 and 7.3 Hz, 1 H), 3.30 (s, 2H), 2.23 (m, 4H), 1.60-1.80 (m, 2H), 1.44 (m, 4H), 1.36 (m, 2H), 0.81 (t, *J* = 7.2 Hz, 3H)
t_{R}: 3.26 min; MS(ESI): m/z (M+H)⁺571; (M+H)⁻569

### (3Z)-N-methyl-2-oxo-N-[(1R)-1-phenylethyl]-3-(phenyl{[3-(piperidin-1-ylmethyl)phenyl]amino}methylene)indoline-5-carboxamide

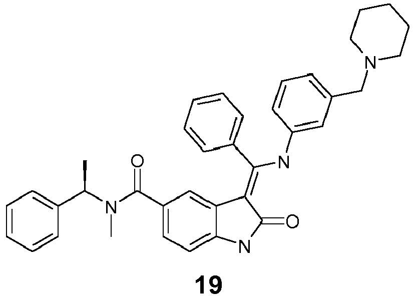

Compound **19** (75 mg, 52%) was obtained starting from (3Z)-2-oxo-3-(phenyl{[3-(piperidin-1-ylmethyl)phenyl]amino}methylene)indoline-5-carboxylic acid and (1R)-N-methyl-1-phenylethanamine
C₃₇H₃₈N₄O₂, Mw calc.: 570.3
¹H-NMR in DMSO-*d*₆ δ: 12.03 (s, 1 H), 10.88 (s, 1 H), 7.42-7.58 (ovl. m, 5H), 7.37 (ddm, *J* = 7.5 and 7.0 Hz, 2H), 7.38 (tm, *J* ∼ 7.0 Hz, 1H), 7.13 (dm, *J* ∼ 7.5 Hz, 2H), 7.11 (dd, *J* = 7.8 and 7.6 Hz, 1 H); 7.03 (dd, *J* = 8.00 and 1.3 Hz, 1 H), 6.92 (dm, *J* = 7.6 Hz, 1 H), 6.91 (d, *J* = 8.0 Hz, 1 H), 6.75 (dm, *J* = 7.8 Hz, 1 H), 6.70 (dd, *J* ∼ 2.5 and 2.0 Hz, 1 H), 5.81 (d, *J* ∼ 1.5 Hz, 1 H), 5.40 (br, 1 H), 3.20 (br. s, 2H), 2.36 (s, 3H), 2.10 (m, 4H), 1.43 (m, 4H), 1.39 (d, *J* ∼ 7.0 Hz, 3H), 1.35 (m, 2H)
t_{R}: 3.21 min ; MS(ESI): m/z (M+H)⁺ 571; (M+H)⁻569

### (3Z)-N-benzyl-2-oxo-3-(phenyl{[4-(piperidin-1-ylmethyl)phenyl]amino} methylene)indoline-5-carboxamide

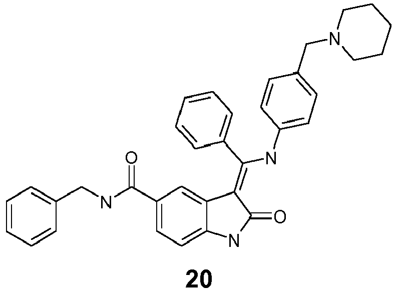

Compound **20** (70 mg, 51%) was obtained starting from (3Z)-2-oxo-3-(phenyl{[4-(piperidin-1-ylmethyl)phenyl]amino}methylene)indoline-5-carboxylic acid and 1-phenylmethanamine
C₃₅H₃₄N₄O₂, Mw calc.: 542.3
¹H-NMR in DMSO-*d*₆ δ: 12.04 (s, 1 H), 10.93 (s, 1 H), 8.42 (t, *J* = 5.9 Hz, 1 H), 7.50-7.60 (ovl. m, 3H), 7.47 (m, 2H), 7.46 (dd, *J =* 8.1 and 1.0 Hz, 1 H), 7.31 (ddm, *J* ∼ 7.5 and 7.0 Hz, 2H), 7.16-7.26 (ovl. m, 3H), 7.05 (dm, *J* = 8.2 Hz, 2H), 6.88 (d, *J =* 8.1 Hz, 1 H), 6.75 (dm, *J* = 8.2 Hz, 2H), 6.53 (d, *J* ∼ 1.0 Hz, 1 H), 4.32 (d, *J* = 5.9 Hz, 2H), 3.28 8s, 2H), 2.22 (m, 4H), 1.44 (m, 4H), 1.36 (m, 2H)
t_{R}: 3.03 min ; MS(ESI): m/z (M+H)⁺543; (M+H)⁻541

### (3Z)-N-[(1R)-1-(4-methoxyphenyl)ethyl]-2-oxo-3-(phenyl{[4-(piperidin-1-ylmethyl)phenyl]amino}methylene)indoline-5-carboxamide

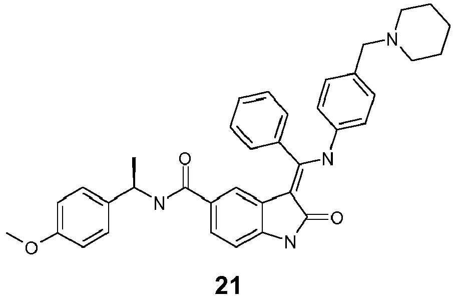

Compound **21** (60 mg, 41%) was obtained starting from (3Z)-2-oxo-3-(phenyl{[4-(piperidin-1-ylmethyl)phenyl]amino}methylene)indoline-5-carboxylic acid and (1R)-1-(4-methoxyphenyl)ethanamine
C₃₇H₃₈N₄O₃, Mw calc. : 586.3
¹H-NMR in DMSO-*d*₆ δ: 12.03 (s, 1 H), 10.92 (s, 1 H), 8.02 (d, *J =* 8.0 Hz, 1 H), 7.50-7.57 (ovl. m, 3H), 7.48 (dd, *J* = 8.1 and 1.5 Hz, 1 H), 7.45 (m, 2H), 7.19 (dm, *J* = 8.6 Hz, 2H), 7.04 (dm, *J* = 8.3 Hz, 2H), 6.87 (d, *J* = 8.1 Hz, 1 H), 6.86 (dm, *J* = 8.6 Hz, 2H), 6.75 (dm, *J* = 8.3 Hz, 2H), 6.44 (d, *J* = 1.5 Hz, 1 H), 4.94 (dq, *J* = 8.0, and 7.0 Hz, 1 H), 3.72 (s, 3H), 3.29 (s, 2H), 2.23 (m, 4H), 1.44 (m, 4H), 1.36 (m, 2H), 1.34 d, *J* = 7.0 Hz, 3H)
t_{R}: 3.08 min; MS(ESI): m/z (M+H)⁺587; (M+H)⁻585

### (3Z)-N-[(1R)-1-(3-methoxyphenyl)ethyl]-2-oxo-3-(phenyl{[4-(piperidin-1-ylmethyl)phenyl]amino}methylene)indoline-5-carboxamide

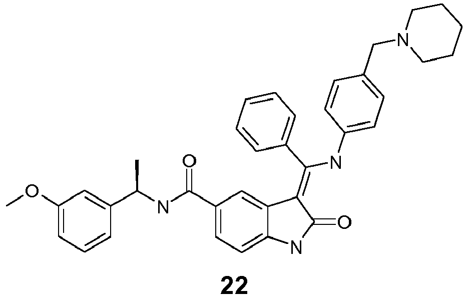

Compound **22** (67 mg, 46%) was obtained starting from (3Z)-2-oxo-3-(phenyl{[4-(piperidin-1-ylmethyl)phenyl]amino}methylene)indoline-5-carboxylic acid and (1R)-1-(3-methoxyphenyl)ethanamine
C₃₇H₃₈N₄O₃, Mw calc.: 586.3
¹H-NMR in DMSO-*d*₆ δ: 12.03 (s, 1 H), 10.92 (s, 1 H), 8.09 (d, *J =* 8.0 Hz, 1 H), 7.50-7.55 (ovl. m, 3H), 7.50 (dd, *J* = 8.1 and 1.0 Hz, 1 H), 7.46 (m, 2H), 7.21 (dd, *J* = 8.0 and 7.8 Hz, 1 H), 7.05 (dm, *J* = 8.3 Hz, 2H), 6.88 (d, *J* = 8.1 Hz, 1 H), 6.87 (dd, *J* ∼ 1.5 and 1.5 Hz, 1 H), 6.85 (dm, *J* = 7.8 Hz, 1 H), 6.78 (dm, *J* = 8.0 Hz, 1 H); 6.75 (dm, *J* = 8.3 Hz, 2H), 6.44 (d, *J* = 1.0 Hz, 1 H), 4.95 (dq, *J* = 8.0, and 7.0 Hz, 1 H), 3.73 (s, 3H), 3.28 (s, 2H), 2.23 (m, 4H), 1.44 (m, 4H), 1.36 (m, 2H), 1.35 d, *J* = 7.0 Hz, 3H)
t_{R}: 3.11 min; MS(ESI): m/z (M+H)⁺587; (M+H)⁻585

### (3Z)-N-[(1R)-1-(4-fluorophenyl)ethyl]-2-oxo-3-(phenyl{[4-(piperidin-1-ylmethyl)phenyl]amino}methylene)indoline-5-carboxamide

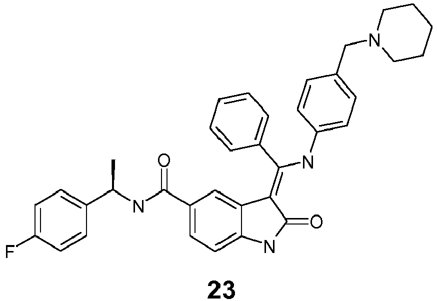

Compound **23** (71 mg, 49%) was obtained starting from (3Z)-2-oxo-3-(phenyl{[4-(piperidin-1-ylmethyl)phenyl]amino}methylene)indoline-5-carboxylic acid and (1R)-1-(4-fluorophenyl)ethanamine
C₃₆H₃₅FN₄O₂, Mw calc.: 574.3
¹H-NMR in DMSO-*d*₆ δ: 12.03 (s, 1 H), 10.92 (s, 1 H), 8.18 (d, *J =* 8.0 Hz, 1 H), 7.50-7.55 (ovl. m, 3H), 7.49 (dd, *J* = 8.1 and 1.0 Hz, 1 H), 7.46 (m, 2H), 7.31 (ddm, *J =* 8.6 and 5.5 Hz, 2H), 7.12 (ddm, *J* = 9.1 and 8.6 Hz, 2H), 7.05 (dm, *J* = 8.3 Hz, 2H), 6.88 (d, *J* = 8.1 Hz, 1 H), 6.75 (dm, *J* = 8.3 Hz, 2H), 6.45 (d, *J* = 1.0 Hz, 1 H), 4.98 (dq, *J =* 8.0, and 7.0 Hz, 1 H), 3.28 (s, 2H), 2.23 (m, 4H), 1.44 (m, 4H), 1.36 (m, 2H), 1.35 d, *J* = 7.0 Hz, 3H)
t_{R}: 3.16 min ; MS(ESI): m/z (M+H)⁺575; (M+H)⁻573

### (3Z)-N-[(1R)-1-(3-methoxyphenyl)ethyl]-2-oxo-3-(phenyl{[3-(piperidin-1-ylmethyl)phenyl]amino}methylene)indoline-5-carboxamide

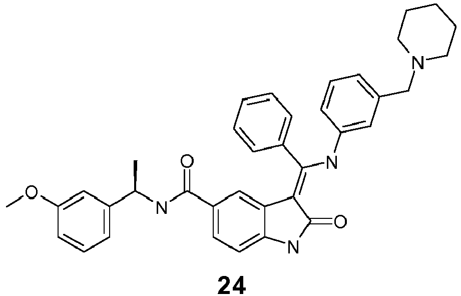

Compound **24** (57 mg, 39%) was obtained starting from (3Z)-2-oxo-3-(phenyl{[3-(piperidin-1-ylmethyl)phenyl]amino}methylene)indoline-5-carboxylic acid and (1*R*)-1-(3-methoxyhenyl)ethanamine
C₃₇H₃₈N₄O₃, Mw calc.: 586.3
¹H-NMR in DMSO-*d*₆ δ: 12.03 (s, 1 H), 10.93 (s, 1 H), 8.09 (d, *J =* 7.8 Hz, 1 H), 7.50-7.55 (ovl. m, 3H), 7.50 (dd, *J* = 8.1 and 1.0 Hz, 1 H), 7.46 (m, 2H), 7.21 (dd, *J* = 8.0 and 7.5 Hz, 1 H), 7.11 (dd, *J* = 7.8 and 7.8 Hz, 1 H), 6.92 (dm, *J* = 7.8 Hz, 1 H), 6.88 (d, *J* = 8.1 Hz, 1 H), 6.86 (dd, *J* = 1.5 and 1.5 Hz, 1 H), 6.85 (dm, *J* = 8.0 Hz, 1 H), 6.78 (dm, *J* = 7.5 Hz, 1 H), 6.75 (dm, *J* = 7.8 Hz, 1 H), 6.69 (dd, *J* ∼ 1.5 and 1.5 Hz, 1 H), 6.45 (d, *J* = 1.0 Hz, 1 H), 4.95 (dq, *J* = 8.0, and 7.0 Hz, 1 H), 3.73 (s, 3H), 3.20 (s, 2H), 2.10 (m, 4H), 1.42 (m, 4H), 1.35 d, *J* = 7.0 Hz, 3H)1.34 (m, 2H),
t_{R}: 3.13 min ; MS(ESI): m/z (M+H)⁺587; (M+H)⁻585

### (3Z)-2-oxo-N-[(1S)-1-phenylethyl]-3-(phenyl{[3-(piperidin-1-ylmethyl)phenyl]amino} methylene)indoline-5-carboxamide

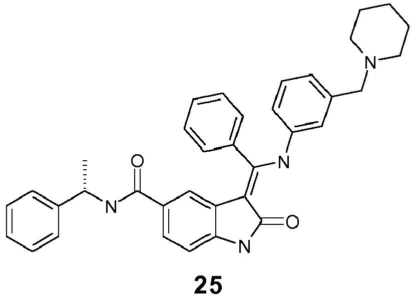

Compound **25** (51 mg, 37%) was obtained starting from (3Z)-2-oxo-3-(phenyl{[3-(piperidin-1-ylmethyl)phenyl]amino}methylene)indoline-5-carboxylic acid and (1S)-1-phenylethanamine
C₃₆H₃₆N₄O₂, Mw calc. : 556.3
¹H-NMR in DMSO-*d*₆ δ: 12.03 (s, 1 H), 10.93 (s, 1 H), 8.12 (d, *J =* 7.8 Hz, 1 H), 7.50-7.55 (ovl. m, 3H), 7.50 (dd, *J =* 8.1 and 1.5 Hz, 1 H), 7.46 (m, 2H), 7.24-7.34 (ovl. m, 4H), 7.20 (m, 1 H), 7.12 (dd, *J* = 7.8 and 7.6 Hz, 1 H), 6.93 (dm, *J* = 7.6 Hz, 1 H), 6.88 (d, *J* = 8.1 Hz, 1 H), 6.76 (dm, *J* = 7.8 Hz, 1 H), 6.69 (m, 1 H), 6.46 (d, *J* = 1.5 Hz, 1 H), 4.98 (dq, *J* = 7.8 and 7.0 Hz, 1 H), 3.22 (s, 2H), 2.11 (m, 4H), 1.43 (m, 4H), 1.36 (d, *J* = 7.0 Hz, 3H), 1.35 (m, 2H)
t_{R}: 3.12 min; MS(ESI): m/z (M+H)⁺557; (M+H)⁻555

### (3Z)-N-[(1S)-1-(4-fluorophenyl)ethyl]-2-oxo-3-(phenyl{[3-(piperidin-1-ylmethyl)phenyl]amino}methylene)indoline-5-carboxamide

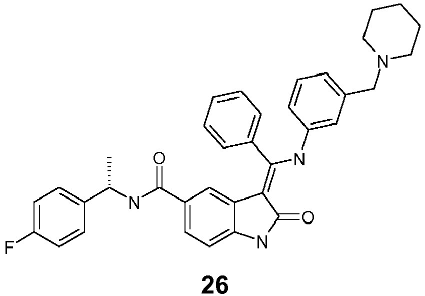

Compound **26** (67 mg, 47%) was obtained starting from (3Z)-2-oxo-3-(phenyl{[3-(piperidin-1-ylmethyl)phenyl]amino}methylene)indoline-5-carboxylic acid and (1S)-1-(4-fluorophenyl)ethanamine
C₃₆H₃₅FN₄O₂, Mw calc. : 574.3
¹H-NMR in DMSO-*d*₆ δ: 12.02 (s, 1 H), 10.93 (s, 1 H), 8.14 (d, *J =* 7.8 Hz, 1 H), 7.49-7.57 (ovl. m, 3H), 7.49 (dd, *J* = 8.1 and 1.5 Hz, 1 H), 7.46 (m, 2H), 7.31 (ddm, *J =* 8.6 and 5.6 Hz, 2H), 7.12 (ddm, *J* = 9.0 and 8.6 Hz, 2H), 7.12 (dd, *J* = 7.8 and 7.6 Hz, 1 H), 6.93 (dm, *J* = 7.6 Hz, 1 H), 6.88 (d, *J* = 8.1 Hz, 1 H), 6.76 (dm, *J* = 7.8 Hz, 1 H), 6.69 (dd, *J* ∼ 2.5 and 2.0 Hz, 1 H), 6.46 (d, *J* = 1.5 Hz, 1 H), 4.98 (dq, *J* = 7.8 and 7.0 Hz, 1 H), 3.20 (s, 2H), 2.10 (m, 4H), 1.42 (m, 4H), 1.35 (d, *J =* 7.0 Hz, 3H), 1.34 (m, 2H)
t_{R}: 3.17 min; MS(ESI): m/z (M+H)⁺575; (M+H)⁻573

### (3Z)-N-[(1R)-1-(4-methylphenyl)ethyl]-2-oxo-3-(phenyl{[3-(piperidin-1-ylmethyl)phenyl]amino}methylene)indoline-5-carboxamide

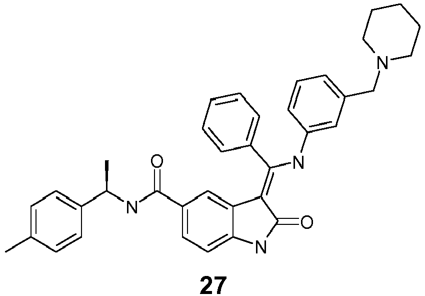

Compound **27** (67 mg, 47%) was obtained starting from (3Z)-2-oxo-3-(phenyl{[3-(piperidin-1-ylmethyl)phenyl]amino}methylene)indoline-5-carboxylic acid and (1R)-1-(4-methylphenyl)ethanamine
C₃₇H₃₈N₄O₂, Mw calc.: 570.3
¹H-NMR in DMSO-*d*₆ δ: 12.03 (s, 1 H), 10.93 (s, 1 H), 8.04 (d, *J =* 7.8 Hz, 1 H), 7.49-7.55 (ovl. m, 3H), 7.49 (dd, *J* = 8.1 and 1.0 Hz, 1 H), 7.46 (m, 2H), 7.16 (dm, *J =* 8.0 Hz, 2H), 7.12 (dd, *J* = 7.8 and 7.6 Hz, 1 H), 7.10 (dm, *J* = 8.0 Hz, 2H), 6.92 (dm, *J* = 7.6 Hz, 1 H), 6.87 (d, *J* = 8.1 Hz, 1 H), 6.75 (dm, *J* = 7.8 Hz, 1 H), 6.68 (dd, *J* ∼ 2.5 and 2.0 Hz, 1 H), 6.46 (d, *J* ∼ 1.0 Hz, 1 H), 4.94 (dq, *J* = 7.8 and 7.0 Hz, 1 H), 3.21 (s, 2H), 2.26 (s, 3H), 2.10 (m, 4H), 1.42 (m, 4H), 1.35 (m, 2H), 1.34 (d, *J* = 7.0 Hz, 3H)
t_{R}: 3.16 min; MS(ESI): m/z (M+H)⁺571; (M+H)⁻569

### (3Z)-N-[(1S)-1-(4-methylphenyl)ethyl]-2-oxo-3-(phenyl{[3-(piperidin-1-ylmethyl)phenyl]amino}methylene)indoline-5-carboxamide

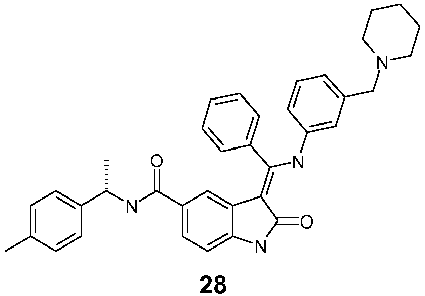

Compound **28** (58 mg, 38%) was obtained starting from (3Z)-2-oxo-3-(phenyl{[3-(piperidin-1-ylmethyl)phenyl]amino}methylene)indoline-5-carboxylic acid and (1S)-1-(4-methylphenyl)ethanamine
C₃₇H₃₈N₄O₂, Mw calc.: 570.3
¹H-NMR in DMSO-*d*₆ δ: 12.03 (s, 1 H), 10.93 (s, 1 H), 8.04 (d, *J =* 7.8 Hz, 1H), 7.49-7.55 (ovl. m, 3H), 7.49 (dd, *J* = 8.1 and 1.0 Hz, 1 H), 7.46 (m, 2H), 7.16 (dm, *J =* 8.0 Hz, 2H), 7.12 (dd, *J* = 7.8 and 7.6 Hz, 1 H), 7.10 (dm, *J* = 8.0 Hz, 2H), 6.92 (dm, *J* = 7.6 Hz, 1 H), 6.87 (d, *J* = 8.1 Hz, 1 H), 6.75 (dm, *J* = 7.8 Hz, 1 H), 6.68 (dd, *J* ∼ 2.5 and 2.0 Hz, 1 H), 6.46 (d, *J* ∼ 1.0 Hz, 1 H), 4.94 (dq, *J* = 7.8 and 7.0 Hz, 1 H), 3.21 (s, 2H), 2.26 (s, 3H), 2.10 (m, 4H), 1.42 (m, 4H), 1.35 (m, 2H), 1.34 (d, *J* = 7.0 Hz, 3H)
t_{R}: 3.26 min; MS(ESI): m/z (M+H)⁺571; (M+H)⁻569

### (3Z)-N-[(1R)-1-(4-methoxyphenyl)ethyl]-2-oxo-3-(phenyl{[3-(piperidin-1-ylmethyl)phenyl]amino}methylene)indoline-5-carboxamide

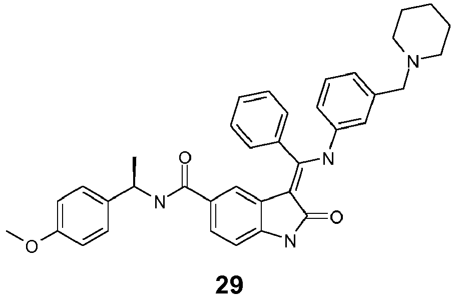

Compound **29** (72 mg, 49%) was obtained starting from (3Z)-2-oxo-3-(phenyl{[3-(piperidin-1-ylmethyl)phenyl]amino}methylene)indoline-5-carboxylic acid and (1R)-1-(4-methoxyphenyl)ethanamine
C₃₇H₃₈N₄O₃, Mw calc.. 586.3
¹H-NMR in DMSO-*d*₆ δ: 12.03 (s, 1 H), 10.93 (s, 1 H), 8.02 (d, *J =* 8.0 Hz, 1 H), 7.49-7.55 (ovl. m, 3H), 7.48 (dd, *J* = 8.1 and 1.0 Hz, 1 H), 7.46 (m, 2H), 7.20 (dm, *J =* 8.6 Hz, 2H), 7.12 (dd, *J* = 7.8 and 7.6 Hz, 1 H), 6.93 (dm, *J* = 7.6 Hz, 1 H), 6.87 (d, *J* = 8.1 Hz, 1 H), 6.86 (dm, *J* = 8.6 Hz, 2H), 6.75 (dm, *J* = 7.8 Hz, 1 H), 6.68 (dd, *J* ∼ 2.5 and 2.0 Hz, 1H), 6.45 (d, *J* ∼ 1.0 Hz, 1H), 4.94 (dq, *J* = 8.0 and 7.0 Hz, 1H), 3.72 (s, 3H), 3.20 (s, 2H), 2.10 (m, 4H), 1.42 (m, 4H), 1.35 (m, 2H), 1.34 (d, *J* = 7.0 Hz, 3H)
t_{R}: 3.02 min; MS(ESI): m/z (M+H)⁺587; (M+H)⁻585

### (3Z)-2-oxo-N-[(1S)-1-phenylethyl]-3-(phenyl{[4-(piperidin-1-ylmethyl)phenyl]amino}methylene)indoline-5-carboxamide

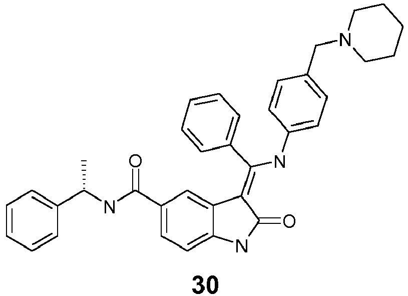

Compound **30** (60 mg, 43%) was obtained starting from (3Z)-2-oxo-3-(phenyl{[4-(piperidin-1-ylmethyl)phenyl]amino}methylene)indoline-5-carboxylic acid and (1S)-1-phenylethanamine
C₃₆H₃₆N₄O₂, Mw calc.: 556.3
¹H-NMR in DMSO-*d*₆ δ: 12.02 (s, 1 H), 10.91 (s, 1 H), 8.10 (d, *J =* 8.0 Hz, 1 H), 7.50-7.56 (ovl. m, 3H), 7.50 (dd, *J =* 8.1 and 1.0 Hz, 1 H), 7.46 (m, 2H), 7.24-7.34 (ovl. m, 4H), 7.20 8m, 1H), 7.04 (dm, *J* = 8.3 Hz, 2H), 6.88 (d, *J* = 8.1 Hz, 1 H), 6.75 (dm, *J* = 8.3 Hz, 2H), 6.45 (d, *J* = 1.0 Hz, 1 H), 4.98 (dq, *J* = 8.0, and 7.0 Hz, 1 H), 3.29 (s, 2H), 2.23 (m, 4H), 1.44 (m, 4H), 1.36 d, *J* = 7.0 Hz, 3H)1.35 (m, 2H),
t_{R}: 3.16 min; MS(ESI): m/z (M+H)⁺557; (M+H)⁻555

### (3Z)-2-oxo-N-[(1R)-1-phenylethyl]-3-(phenyl{[4-(piperidin-1-ylmethyl)phenyl]amino}methylene)indoline-5-carboxamide

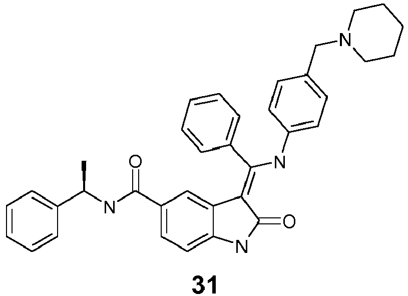

Compound **31** (66 mg, 47%) was obtained starting from (3Z)-2-oxo-3-(phenyl{[4-(piperidin-1-ylmethyl)phenyl]amino}methylene)indoline-5-carboxylic acid and (1R)-1-phenylethanamine
C₃₆H₃₆N₄O₂, Mw calc.: 556.3
¹H-NMR in DMSO-*d*₆ δ: 12.02 (s, 1 H), 10.91 (s, 1 H), 8.10 (d, *J =* 8.0 Hz, 1 H), 7.50-7.56 (ovl. m, 3H), 7.50 (dd, *J =* 8.1 and 1.0 Hz, 1 H), 7.46 (m, 2H), 7.24-7.34 (ovl. m, 4H), 7.20 8m, 1H), 7.04 (dm, *J* = 8.3 Hz, 2H), 6.88 (d, *J* = 8.1 Hz, 1 H), 6.75 (dm, *J* = 8.3 Hz, 2H), 6.45 (d, *J* = 1.0 Hz, 1 H), 4.98 (dq, *J* = 8.0, and 7.0 Hz, 1 H), 3.29 (s, 2H), 2.23 (m, 4H), 1.44 (m, 4H), 1.36 d, *J* = 7.0 Hz, 3H)1.35 (m, 2H),
t_{R}: 3.16 min; MS(ESI): m/z (M+H)⁺557; (M+H)⁻555

### (3Z)-2-oxo-3-(phenyl{[4-(piperidin-1-ylmethyl)phenyl]amino}methylene)-N-[(1S)-1-phenylpropyl]indoline-5-carboxamide

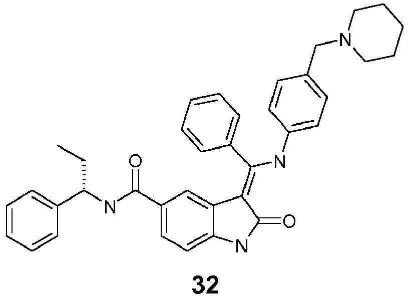

Compound **32** (65 mg, 45%) was obtained starting from (3Z)-2-oxo-3-(phenyl{[4-(piperidin-1-ylmethyl)phenyl]amino}methylene)indoline-5-carboxylic acid and (1S)-1-phenylpropan-1-amine
C₃₇H₃₈N₄O₂, Mw calc.: 570.3
¹H-NMR in DMSO-*d*₆ δ: 12.04 (s, 1 H), 10.91 (s, 1 H), 8.03 (d, *J =* 8.3 Hz, 1 H), 7.49-7.57 (ovl. m, 3H), 7.49 (dd, *J =* 8.1 and 1.5 Hz, 1 H), 7.46 (m, 2H), 7.24-7.34 (ovl. m, 4H), 7.21 (m, 1 H), 7.04 (dm, *J* = 8.2 Hz, 2H), 6.88 (d, *J* = 8.1 Hz, 1 H), 6.75 (dm, *J* = 8.2 Hz, 2H), 6.46 (d, *J* = 1.5 Hz, 1 H), 4.73 (ddd, *J* = 8.3, 7.3 and 7.3 Hz, 1 H), 3.29 (s, 2H), 2.23 (m, 4H), 1.60-1.80 (m, 2H), 1.44 (m, 4H), 1.36 (m, 2H), 0.81 (t, *J* = 7.2 Hz, 3H)
t_{R}: 3.25 min; MS(ESI): m/z (M+H)⁺571; (M+H)⁻569

### (3Z)-N-[(1S)-1-(4-methoxyphenyl)ethyl]-2-oxo-3-(phenyl{[4-(piperidin-1-ylmethyl)phenyl]amino}methylene)indoline-5-carboxamide

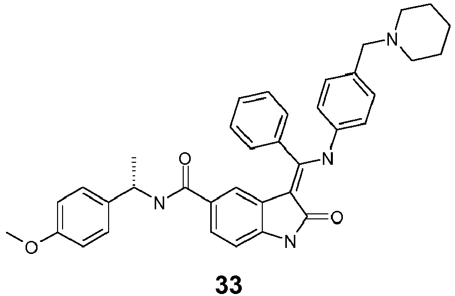

Compound **33** (70 mg, 48%) was obtained starting from (3Z)-2-oxo-3-(phenyl{[4-(piperidin-1-ylmethyl)phenyl]amino}methylene)indoline-5-carboxylic acid and (1S)-1-(4-methoxyphenyl)ethanamine
C₃₇H₃₈N₄O₃, Mw calc.: 586.3
¹H-NMR in DMSO-*d*₆ δ: 12.02 (s, 1 H), 10.91 (s, 1 H), 8.02 (d, *J =* 8.0 Hz, 1 H), 7.50-7.57 (ovl. m, 3H), 7.48 (dd, *J* = 8.1 and 1.5 Hz, 1 H), 7.45 (m, 2H), 7.19 (dm, *J =* 8.6 Hz, 2H), 7.04 (dm, *J* = 8.3 Hz, 2H), 6.87 (d, *J* = 8.1 Hz, 1 H), 6.86 (dm, *J* = 8.6 Hz, 2H), 6.75 (dm, *J* = 8.3 Hz, 2H), 6.44 (d, *J* = 1.5 Hz, 1 H), 4.94 (dq, *J* = 8.0, and 7.0 Hz, 1 H), 3.72 (s, 3H), 3.29 (s, 2H), 2.23 (m, 4H), 1.44 (m, 4H), 1.36 (m, 2H), 1.34 d, *J* = 7.0 Hz, 3H)
t_{R}: 3.07 min; MS(ESI): m/z (M+H)⁺587; (M+H)⁻585

### (3Z)-N-[(1S)-1-(3-methoxyphenyl)ethyl]-2-oxo-3-(phenyl{[4-(piperidin-1-ylmethyl)phenyl]amino}methylene)indoline-5-carboxamide

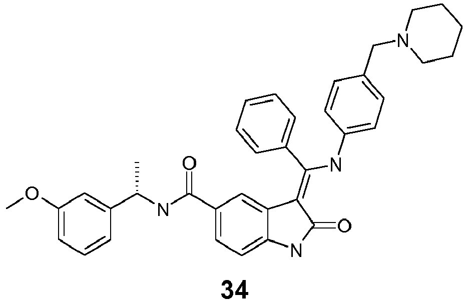

Compound **34** (70 mg, 48%) was obtained starting from (3Z)-2-oxo-3-(phenyl{[4-(piperidin-1-ylmethyl)phenyl]amino}methylene)indoline-5-carboxylic acid and (1S)-1-(3-methoxyphenyl)ethanamine
C₃₇H₃₈N₄O₃, Mw calc.: 586.3
¹H-NMR in DMSO-*d*₆ δ: 12.03 (s, 1 H), 10.92 (s, 1 H), 8.08 (d, *J =* 8.0 Hz, 1 H), 7.50-7.55 (ovl. m, 3H), 7.50 (dd, *J* = 8.1 and 1.0 Hz, 1 H), 7.46 (m, 2H), 7.21 (dd, *J* = 8.0 and 7.8 Hz, 1 H), 7.05 (dm, *J* = 8.3 Hz, 2H), 6.88 (d, *J* = 8.1 Hz, 1 H), 6.87 (dd, *J* ∼ 1.5 and 1.5 Hz, 1 H), 6.85 (dm, *J* = 7.8 Hz, 1 H), 6.78 (dm, *J* = 8.0 Hz, 1 H); 6.75 (dm, *J* = 8.3 Hz, 2H), 6.44 (d, *J* = 1.0 Hz, 1 H), 4.95 (dq, *J* = 8.0, and 7.0 Hz, 1 H), 3.73 (s, 3H), 3.28 (s, 2H), 2.23 (m, 4H), 1.44 (m, 4H), 1.36 (m, 2H), 1.35 d, *J* = 7.0 Hz, 3H)
t_{R}: 3.09 min; MS(ESI): m/z (M+H)⁺587; (M+H)⁻585

### (3Z)-N-[(1R)-1-(3-methoxyphenyl)ethyl]-3-[({4-[(4-methylpiperazin-1-yl)methyl]phenyl}amino)(phenyl)methylene]-2-oxoindoline-5-carboxamide

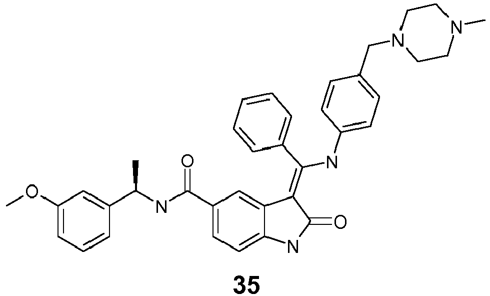

Compound **35** (84 mg, 56%) was obtained starting from (3Z)-3-[({4-[(4-methylpiperazin-1-yl)methyl]phenyl}amino)(phenyl)methylene]-2-oxoindoline-5-carboxylic acid and (1R)-1-(3-methoxyphenyl)ethanamine
C₃₇H₃₉N₅O₃, Mw calc.: 601.3
¹H-NMR in DMSO-*d*₆ δ: 12.03 (s, 1 H), 10.93 (s, 1 H), 8.10 (d, *J =* 8.0 Hz, 1 H), 7.50-7.56 (ovl. m, 3H), 7.50 (dd, *J* = 8.1 and 1.0 Hz, 1 H), 7.46 (m, 2H), 7.21 (dd, *J* = 8.1 and 7.8 Hz, 1 H), 7.05 (dm, *J* = 8.3 Hz, 2H), 6.88 (d, *J* ∼ 8.1 Hz, 1 H), 6.87 (dd, *J* ∼ 2.0 and 1.5 Hz, 1 H), 6.85 (dm, *J* = 7.8 Hz, 1 H), 6.78 (dm, *J* = 8.1 Hz, 1 H), 6.76 (dm, *J* = 8.3 Hz, 2H), 6.44 (d, *J* = 1.0 Hz, 1 H), 4.95 (dq, *J* = 8.0 and 7.0 Hz, 1 H), 3.73 (s, 3H), 3.32 (s, 2H), 2.22-2.42 (m, 8H), 2.18 (s, 3H), 1.35 (d, *J* = 7.0 Hz, 3H)
t_{R}: 2.8 min; MS(ESI): m/z (M+H)⁺602; (M+H)⁻600

### (3Z)-N-[(1R)-1-(3-chlorophenyl)ethyl]-3-[({4-[(4-methylpiperazin-1-yl)methyl]phenyl}amino)(phenyl)methylene]-2-oxoindoline-5-carboxamide

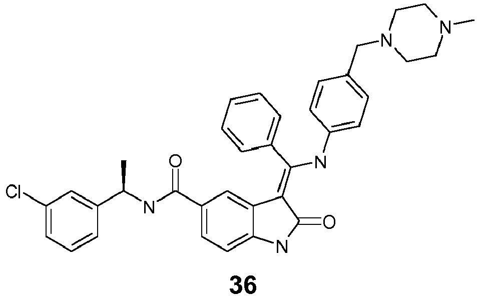

Compound **36** (72 mg, 47%) was obtained starting from (3Z)-3-[({4-[(4-methylpiperazin-1-yl)methyl]phenyl}amino)(phenyl)methylene]-2-oxoindoline-5-carboxylic acid and (1R)-1-(3-chlorophenyl)ethanamine
C₃₆H₃₆ClN₅O₂, Mw calc.: 605.2
¹H-NMR in DMSO-*d*₆ δ: 12.03 (s, 1 H), 10.93 (s, 1 H), 8.18 (d, *J* = 7.7 Hz, 1 H), 7.50-7.57 (ovl. m, 3H), 7.50 (dd, *J* = 8.1 and 1.0 Hz, 1 H), 7.46 (m, 2H), 7.35 (dd, *J* ∼ 1.5 and 1.5 Hz, 1 H), 7.33 (dd, *J* ∼ 7.5 and 7.5 Hz, 1 H), 7.27 (dm, *J* ∼ 7.5 Hz, 1 H), 7.24 (dm, *J* ∼ 7.5 Hz, 1 H), 7.05 (dm, *J* = 8.3 Hz, 2H), 6.89 (d, *J* ∼ 8.1 Hz, 1 H), 6.76 (dm, *J* = 8.3 Hz, 2H), 6.44 (d, *J* = 1.0 Hz, 1 H), 4.96 (dq, *J* = 7.7 and 7.0 Hz, 1 H), 3.31 (s, 2H), 2.19-2.39 (m, 8H), 2.14 (s, 3H), 1.36 (d, *J =* 7.0 Hz, 3H)
t_{R}: 3.00 min; MS(ESI): m/z (M+H)⁺606; (M+H)⁻604

### (3Z)-3-[({4-[(4-methylpiperazin-1-yl)methyl]phenyl}amino)(phenyl)methylene]-2-oxo-N-[(1R)-1-phenylpropyl]indoline-5-carboxamide

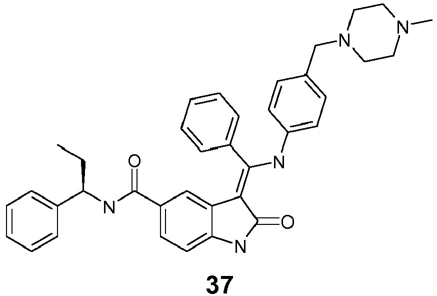

Compound **37** (57 mg, 39%) was obtained starting from (3Z)-3-[({4-[(4-methylpiperazin-1-yl)methyl]phenyl}amino)(phenyl)methylene]-2-oxoindoline-5-carboxylic acid and (1 R)-1-phenylpropan-1-amine
C₃₇H₃₉N₅O₂, MW calc.: 585.3
¹H-NMR in DMSO-*d*₆ δ: 12.04 (s, 1 H), 10.93 (s, 1 H), 8.06 (d, *J =* 8.2 Hz, 1 H), 7.50-7.57 (ovl. m, 3H), 7.49 (dd, *J =* 8.1 and 1.0 Hz, 1 H), 7.46 (m, 2H), 7.24-7.34 (ovl. m, 4H), 7.20 (m, 1 H), 7.05 (dm, *J* = 8.3 Hz, 2H), 6.88 (d, *J* ∼ 8.1 Hz, 1 H), 6.75 (dm, *J* = 8.3 Hz, 2H), 6.46 (d, *J* = 1.0 Hz, 1 H), 4.73 (ddd, *J* = 8.2, 7.4 and 7.4 Hz, 1 H), 3.29 (s, 2H), 2.21-2.41 (m, 8H), 2.16 (s, 3H), 1.60-1.80 (ovl. m, 2H), 0.81 (t, *J =* 7.0 Hz, 3H)
t_{R}: 2.91 min; MS(ESI): m/z (M+H)⁺586; (M+H)⁻584

### (3Z)-3-[({4-[(4-methylpiperazin-1-yl)methyl]phenyl}amino)(phenyl)methylene]-2-oxo-N-[(1R)-1-phenylethyl]indoline-5-carboxamide

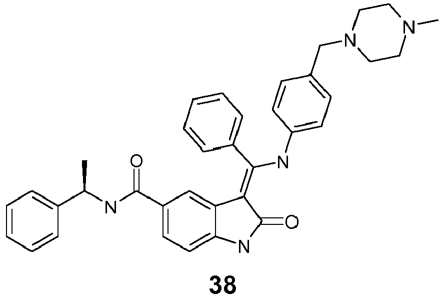

Compound **38** (60 mg, 42%) was obtained starting from (3Z)-3-[({4-[(4-methylpiperazin-1-yl)methyl]phenyl}amino)(phenyl)methylene]-2-oxoindoline-5-carboxylic acid and (1R)-1-phenylethanamine
C₃₆H₃₇N₅O₂, Mw calc.: 571.3
¹H-NMR in DMSO-*d*₆ δ: 12.03 (s, 1 H), 10.93 (s, 1 H), 8.12 (d, *J =* 8.1 Hz, 1 H), 7.50-7.56 (ovl. m, 3H), 7.49 (dd, *J =* 8.1 and 1.0 Hz, 1 H), 7.46 (m, 2H), 7.24-7.34 (ovl. m, 4H), 7.21 (m, 1 H), 7.06 (dm, *J* = 8.3 Hz, 2H), 6.88 (d, *J* ∼ 8.1 Hz, 1 H), 6.76 (dm, *J* = 8.3 Hz, 2H), 6.45 (d, *J* = 1.0 Hz, 1 H), 4.98 (dq, *J* = 8.1 and 7.0 Hz, 1 H), 3.32 (s, 2H), 2.23-2.43 (m, 8H), 2.18 (s, 3H), 1.36 (d, *J* = 7.0 Hz, 3H)
t_{R}: 2.78 min; MS(ESI): m/z (M+H)⁺572; (M+H)⁻570

### (3Z)-N-(3,4-dimethoxybenzyl)-3-[({4-[(4-methylpiperazin-1-yl)methyl]phenyl}amino)(phenyl)methylene]-2-oxoindoline-5-carboxamide

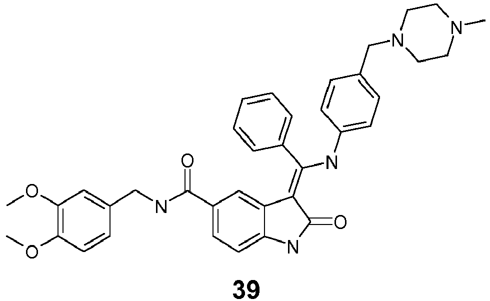

Compound **39** (74 mg, 48%) was obtained starting from (3Z)-3-[({4-[(4-methylpiperazin-1-yl)methyl]phenyl}amino)(phenyl)methylene]-2-oxoindoline-5-carboxylic acid and 1-(3,4-dimethoxyphenyl)methanamine
C₃₇H₃₉N₅O₄, Mw calc.: 617.3
¹H-NMR in DMSO-*d*₆ δ: 12.04 (s, 1 H), 10.92 (s, 1 H), 8.32 (t, *J* = 5.5 Hz, 1 H), 7.49-7.57 (ovl. m, 3H), 7.46 (m, 2H), 7.45 (dd, *J =* 8.1 and 1.0 Hz, 1 H), 7.06 (dm, *J* = 8.0 Hz, 2H), 6.88 (d, *J* = 8.1 Hz, 1 H), 6.87 (d, *J* ∼ 8.0 Hz, 1 H), 6.86 (d, J ∼ 1.5 Hz, 1 H), 6.76 (dm, *J* = 8.0 Hz, 2H), 6.73 (dd, *J* ∼ 8.0 and 1.5 Hz, 1 H), 6.51 (d, *J* = 1.0 Hz, 1 H), 4.25 (d, *J* = 5.5 Hz, 2H), 3.72 (s, 3H), 3.71 (s, 3H), 3.36 (s, 2H), 2.31-2.33 (m, 8H), 2.30 (s, 3H)
t_{R}: 2.50 min; MS(ESI): m/z (M+H)⁺618; (M+H)⁻616

### (3Z)-N-(3-methoxybenzyl)-3-[({4-[(4-methylpiperazin-1-yl)methyl]phenyl}amino)(phenyl)methylene]-2-oxoindoline-5-carboxamide

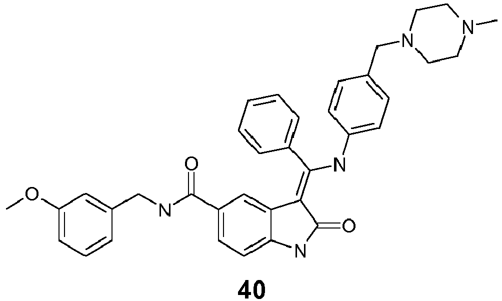

Compound **40** (72 mg, 49%) was obtained starting from (3Z)-3-[({4-[(4-methylpiperazin-1-yl)methyl]phenyl}amino)(phenyl)methylene]-2-oxoindoline-5-carboxylic acid and 1-(3-methoxyphenyl)methanamine
C₃₆H₃₇N₅O₃, Mw calc.: 587.3
¹H-NMR in DMSO-*d*₆ δ: 12.03 (s, 1 H), 10.93 (s, 1 H), 8.40 (t, *J* = 5.6 Hz, 1 H), 7.49-7.55 (ovl. m, 3H), 7.47 (dd, *J* = 8.1 and 1.0 Hz, 1 H), 7.46 (m, 2H), 7.21 (dd, *J* = 8.4 and 8.0 Hz, 1 H), 7.05 (dm, *J* = 8.3 Hz, 2H), 6.88 (d, *J* = 8.1 Hz, 1 H), 6.76-6.80 (ovl. m, 3H), 6.75 (dm, *J* = 8.3 Hz, 2H), 6.52 (d, *J* = 1.0 Hz, 1 H), 4.29 (d, *J* = 5.6 Hz, 2H), 3.72 (s, 3H), 3.28 (s, 2H), 2.19-2.33 (m, 8H), 2.12 (s, 3H)
t_{R}: 2.68 min; MS(ESI): m/z (M+H)⁺588; (M+H)⁻586

### (3Z)-2-oxo-N-[(1R)-1-phenylethyl]-3-(phenyl{[4-(piperidin-1-ylmethyl)phenyl]amino} methylene)indoline-6-carboxamide

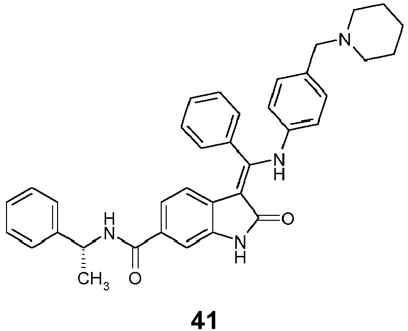

Compound **41** (72 mg, 49%) was obtained starting from (3Z)-2-oxo-3-(phenyl{[4-(piperidin-1-ylmethyl)phenyl]amino}methylene)indoline-6-carboxylic acid and (1 R)-1-phenylethanamine
C₃₆H₃₆N₄O₂, Mw calc.: 556.3
¹H-NMR in DMSO-*d*₆ δ: 12.11 (s, 1 H), 10.85 (s, 1 H), 8.50 (d, *J =* 8.0 Hz, 1 H), 7.52-7.62 (ovl. m, 3H), 7.48 (m, 2H), 7.37 (d, *J* ∼ 1.5 Hz, 1 H), 7.34 (dm, *J* ∼ 7.5 Hz, 2H), 7.29 (ddm, *J* ∼ 7.5 and 7.0 Hz, 2H), 7.19 (tm, *J* ∼ 7.0 Hz, 1 H), 7.15 (dd, *J* = 8.1 and 1.5 Hz, 1 H), 7.06 (dm, *J* = 8.2 Hz, 2H), 6.80 (dm, *J* = 8.2 Hz, 2H), 5.71 (d, *J* = 8.1 Hz, 1 H), 5.11 (dq, *J* = 8.0, and 7.0 Hz, 1 H), 3.29 (s, 2H), 2.23 (m, 4H), 1.44 (m, 4H), 1.42 (d, *J* = 7.0 Hz, 3H)1.36 (m, 2H)
t_{R}: 3.15 min.; MS(ESI): m/z (M+H)⁺557; (M+H)⁻555

### (3Z)-2-oxo-3-(phenyl{[4-(piperidin-1-ylmethyl)phenyl]amino}methylene)-N-[(1R)-1-phenylpropyl]indoline-6-carboxamide

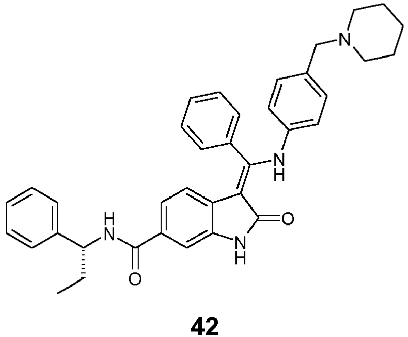

Compound **42** (77 mg, 54%) was obtained starting from (3Z)-2-oxo-3-(phenyl{[4-(piperidin-1-ylmethyl)phenyl]amino}methylene)indoline-6-carboxylic acid and (1R)-1-phenylpropan-1-amine
C₃₇H₃₈N₄O₂, Mw calc.: 570.3
¹H-NMR in DMSO-*d*₆ δ: 12.10 (s, 1 H), 10.85 (s, 1 H), 8.43 (d, *J =* 8.4 Hz, 1 H), 7.52-7.62 (ovl. m, 3H), 7.48 (m, 2H), 7.35 (d, *J* ∼ 1.5 Hz, 1 H), 7.34 (dm, *J* ∼ 7.5 Hz, 2H), 7.28 (ddm, *J* ∼ 7.5 and 7.0 Hz, 2H), 7.19 (tm, *J* ∼ 7.0 Hz, 1H), 7.14 (dd, *J =* 8.2 and 1.5 Hz, 1 H), 7.06 (dm, *J* = 8.3 Hz, 2H), 6.80 (dm, *J* = 8.3 Hz, 2H), 5.70 (d, *J* = 8.1 Hz, 1 H), 4.84 (ddd, *J* = 8.0, 7.5 and 7.5 Hz, 1 H), 3.28 (s, 2H), 2.22 (m, 4H), 1.66-1.86 (ovl. m, 2H), 1.44 (m, 4H), 1.36 (m, 2H)0.85 (t, *J* = 7.3 Hz, 3H)
t_{R}: 3.24 min ; MS(ESI): m/z (M+H)⁺571; (M+H)⁻569

### (3Z)-3-[({3-[(4-methylpiperazin-1-yl)methyl]phenyl}amino)(phenyl)methylene]-2-oxo-N-[(1R)-1-phenylethyl]indoline-5-carboxamide

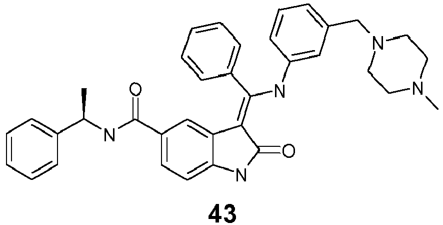

C36H37N5O2, Mw calc.:571.3
MS(ESI): m/z (M+H)⁺572

### (3Z)-3-[({3-[(4-methylpiperazin-1-yl)methyl]phenyl}amino)(phenyl)methylene]-2-oxo-N-[(1R)-1-phenylpropyl]indoline-5-carboxamide

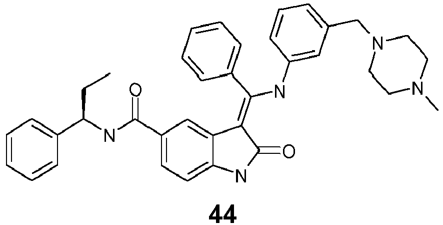

C37H39N5O2, Mw calc.:585.3
MS(ESI): m/z (M+H)⁺586

### (3Z)-N-benzyl-3-[({3-[(4-methylpiperazin-1-yl)methyl]phenyl}amino)(phenyl)methylene]-2-oxoindoline-5-carboxamide

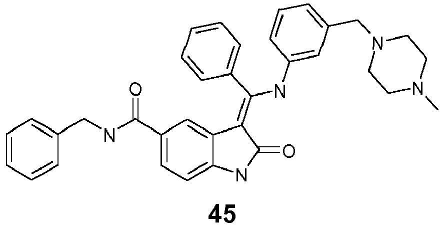

C35H35N5O2, Mw calc.:557.3
MS(ESI): m/z (M+H)⁺558

### (3Z)-N-[(1R)-1-(3-chlorophenyl)ethyl]-3-[({3-[(4-methylpiperazin-1-yl)methyl]phenyl}amino)(phenyl)methylene]-2-oxoindoline-5-carboxamide

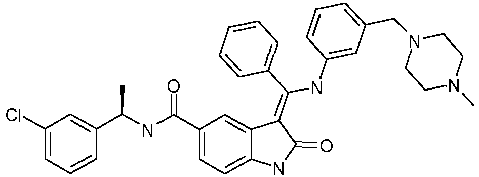

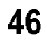

C₃₆H₃₆ClN₅O₂, Mw calc.:605.2
MS(ESI): m/z (M+H)⁺ 606

### (3Z)-N-[(1R)-1-(3-methoxyphenyl)ethyl]-3-[({3-[(4-methylpiperazin-1-yl)methyl]phenyl}amino)(phenyl)methylene]-2-oxoindoline-5-carboxamide

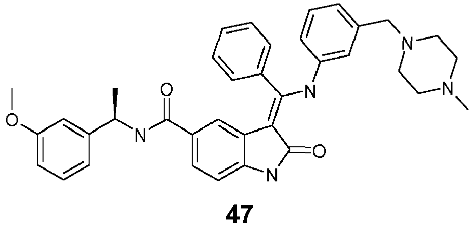

C₃₇H₃₉N₅O₃, Mw calc. :601.3
MS(ESI): m/z (M+H)⁺602

### (3Z)-N-(3-methoxybenzyl)-3-[({3-[(4-methylpiperazin-1-yl)methyl]phenyl}amino) (phenyl)methylene]-2-oxoindoline-5-carboxamide

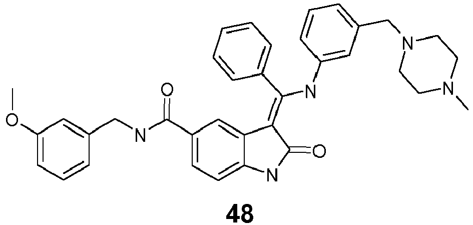

C₃₆H₃₇N₅O₃, Mw calc.:587.3
MS(ESI): m/z (M+H)⁺588

### (3Z)-N-(3,4-dimethoxybenzyl)-3-[({3-[(4-methylpiperazin-1-yl)methyl]phenyl} amino)(phenyl)methylene]-2-oxoindoline-5-carboxamide

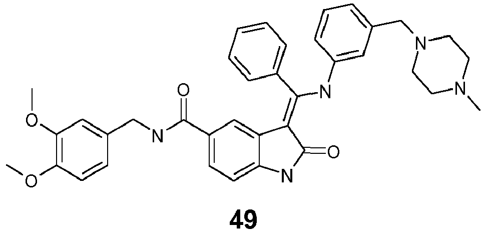

C₃₇H₃₉N₅O₄, Mw calc.:617.3
MS(ESI): m/z (M+H)⁺618

### Example 2

### Step A

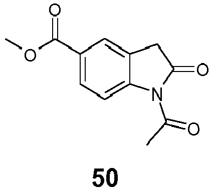

10 mmol (1.91 g) methyl-2-oxoindoline-5-carboxylate was suspended in 20 ml acetic-anhydride and stirred for 2-3 hours at 130-140 °C. The reaction mixture was cooled to rt and poured into crushed ice (100-150 g) then the precipitated product was filtered off.
2.17 g (93 %) Rt: 3.21 min.
MS (ESI) [M+H]⁺: 234 MS (ESI) [M+H]⁻: 232

### Step B

### Methyl-(3Z)-1-acetyl-3-[hydroxy(pyridin-4-yl)methylene]-2-oxoindoline-5-carboxylate

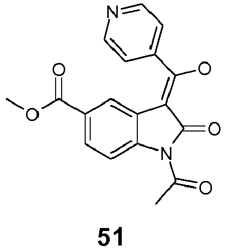

10 mmol (methyl-1-acetyl-2-oxoindoline-5-carboxylate) was dissolved in 40 ml dry dichloromethane and 100 mmol DIPEA, 0.1 mmol DMAP (4-Dimethylaminopyridine) were added to the reaction mixture under inert atmosphere and stirred for 5-10 mins. 1.1 mmol isonicotinoyl chloride was added in one portion and stirred for 2 hours. The reaction was monitored by TLC. After the completion, the reaction mixture was washed with 20-30 ml sat. aq. NaHCO₃ and the organic layer was separated and dried over MgSO₄. The dessicant was filtered off and the solvent was removed under reduced pressure to give methyl-(3*Z*)-1-acetyl-3-[hydroxy(pyridin-4-yl)methylene]-2-oxoindoline-5-carboxylate as brown foamy solid.
C18H14N2O5 calc. 338.32
MS (ESI) [M+H]⁺: 339 MS (ESI) [M+H]⁻: 337

The following compounds were obtained according to the procedure described by **example 2, step B**
Compound **52:**
   Methyl-(3*Z*)-1-acetyl-3-[hydroxy(pyridin-3-yl)methylene]-2-oxoindoline-5-carboxylate
   C18H14N2O5 calc. 338.32
   MS (ESI) [M+H]⁺: 339 MS (ESI) [M+H]⁻: 337
Compound **53:**
   Methyl-(3*Z*)-1-acetyl-3-[hydroxy(pyridin-2-yl)methylene]-2-oxoindoline-5-carboxylate
   C18H14N2O5 calc. 338.32
   MS (ESI) [M+H]⁺: 339 MS (ESI) [M+H]⁻: 337
Compound **54:**
   Methyl-(3*Z*)-1-acetyl-3-[hydroxy(3-thienyl)methylene]-2-oxoindoline-5-carboxylate C17H13NO5S calc. 343.36
   MS (ESI) [M+H]⁺: 344 MS (ESI) [M+H]⁻: 342
Compound **55:**
   Methyl-(3*Z*)-1-acetyl-3-[3-furyl(hydroxy)methylene]-2-oxoindoline-5-carboxylate C17H13NO6 calc. 327.30
   MS (ESI) [M+H]⁺: 328 MS (ESI) [M+H]⁻: 326
Compound **56:**
   Methyl-(3*Z*)-1-acetyl-3-[2-furyl(hydroxy)methylene]-2-oxoindoline-5-carboxylate C17H13NO6 calc. 327.30
   MS (ESI) [M+H]⁺: 328 MS (ESI) [M+H]⁻: 326
Compound **57:**
   Methyl-(3*Z*)-3-[hydroxy(1-methyl-1*H*-pyrazol-4-yl)methylene]-2-oxoindoline-5-carboxylate
   C17H15N3O5 calc. 341.33
   MS (ESI) [M+H]⁺: 342 MS (ESI) [M+H]⁻: 340
Compound **58:**
   Methyl-(3*Z*)-1-acetyl-3-[hydroxy(1-methyl-1*H*-indol-5-yl)methylene]-2-oxoindoline-5-carboxylate
   C22H18N2O5 calc. 390.40
   MS (ESI) [M+H]⁺: 391 MS (ESI) [M+H]⁻: 389
Compound **59:**
   Methyl-(3*Z*)-1-acetyl-3-[cyclopropyl(hydroxy)methylene]-2-oxoindoline-5-carboxylate
   C16H15NO5 calc. 301.30
   MS (ESI) [M+H]⁺: 302 MS (ESI) [M+H]⁻: 300
Compound **60:**
   Methyl-(3*Z*)-1-acetyl-3-[cyclohexyl(hydroxy)methylene]-2-oxoindoline-5-carboxylate
   C19H21NO5 calc. 343.38
   MS (ESI) [M+H]⁺: 342 MS (ESI) [M+H]⁻: 344
Compound **61:**
   Methyl-(3*Z*)-1-acetyl-3-(1-hydroxypropylidene)-2-oxoindoline-5-carboxylate C15H15NO5 calc. 289.29
   MS (ESI) [M+H]⁺: 288 MS (ESI) [M+H]⁻: 287
Compound **62:** Methyl (3Z)-1-acetyl-3-(1-hydroxy-2-methylpropylidene)-2-oxoindoline-5-carboxylate C16H17NO5 calc. 303.32
   MS (ESI) [M+H]⁺: 304 MS (ESI) [M+H]⁻: 302

### Step C

### Methyl-(3Z)-3-[({4-[(4-methylpiperazin-1-yl)methyl]phenyl}amino)(pyridin-4-yl)methylene]-2-oxoindoline-5-carboxylate

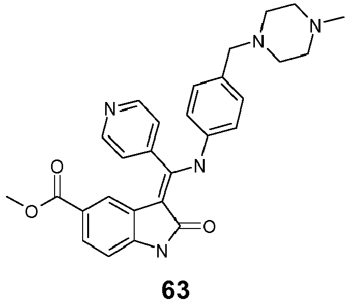

10 mmol (3.38 g) methyl-(3*Z*)-1-acetyl-3-[hydroxy(pyridin-4-yl)methylene]-2-oxoindoline-5-carboxylate intermediate was suspended in 40 ml dry dioxane and 20 mmol (3.22 g, 4.18 ml) HMDS (hexamethyldisilazane), 20 mmol (2.17 g, 2.54 ml) trimethylsilyl chloride and 4-[(4-methylpiperazin-1-yl)methyl]aniline were added to the reaction mixture under inert atmosphere and refluxed overnight. The reaction mixture was poured into 30 ml 10 % Na₂CO₃ and extracted with 3x60 ml ethyl-acetate. The organic layer was separated and dried over MgSO₄. The dessicant was filtered off and the solvent was removed under reduced pressure to give methyl-(3Z)-3-[({4-[(4-methylpiperazin-1-yl)methyl]phenyl}amino)(pyridin-4-yl)methylene]-2-oxoindoline-5-carboxylate as dark brown oil. This product was used in the next step without purification.
C28H29N5O3 calc. 483.58
MS (ESI) [M+H]⁺: 484 MS (ESI) [M+H]⁻: 482

The following compounds were obtained according to the procedure described by **example 2, step C**
Compound **64:**
   Methyl-(3Z)-3-[({4-[(4-methylpiperazin-1-yl)methyl]phenyl}amino)(pyridin-3-yl)methylene]-2-oxoindoline-5-carboxylate
   C28H29N5O3 calc. 483.58
   MS (ESI) [M+H]⁺: 484 MS (ESI) [M+H]⁻: 482
Compound **65:**
   Methyl-(3Z)-3-[{[4-(morpholin-4-ylmethyl)phenyl]amino}(pyridin-3-yl)methylene]-2-oxoindoline-5-carboxylate
   C27H26N4O4 calc. 470.53
   MS (ESI) [M+H]⁺: 471 MS (ESI) [M+H]⁻: 469
Compound **270:**
   Methyl-(3Z)-2-oxo-3-[{[4-(piperidin-1-ylmethyl)phenyl]amino}(pyridin-3-yl)methylene]indoline-5-carboxylate
   C28H28N4O3 calc. 468.56
   MS (ESI) [M+H]⁺: 469 MS (ESI) [M+H]⁻: 470
Compound **66:**
   Methyl-(3Z)-2-oxo-3-(pyridin-3-yl{[4-(pyrrolidin-1-ylmethyl)phenyl]amino}methylene) indoline-5-carboxylate
   C27H26N4O3 calc. 454.53
   MS (ESI) [M+H]⁺: 455 MS (ESI) [M+H]⁻: 453
Compound **67:**
   Methyl-(3Z)-3-[({3-[(4-methylpiperazin-1-yl)methyl]phenyl}amino)(pyridin-3-yl)methylene]-2-oxoindoline-5-carboxylate
   C28H29N5O3 calc. 483.58
   MS (ESI) [M+H]⁺: 484 MS (ESI) [M+H]⁻: 482
Compound **68:**
   Methyl-(3Z)-2-oxo-3-[{[3-(piperidin-1-ylmethyl)phenyl]amino}(pyridin-3-yl)methylene]indoline-5-carboxylate
   C28H28N4O3 calc. 468.56
   MS (ESI) [M+H]⁺: 469 MS (ESI) [M+H]⁻: 467
Compound **69:**
   Methyl-(3*Z*)-3-[({4-[(diethylamino)methyl]phenyl}amino)(pyridin-3-yl)methylene]-2-oxoindoline-5-carboxylate
   C27H28N4O3 calc. 456.55
   MS (ESI) [M+H]⁺: 457 MS (ESI) [M+H]⁻: 455
Compound **70:**
   Methyl-(3Z)-2-oxo-3-[{[4-(piperidin-1-ylmethyl)phenyl]amino}(pyridin-2-yl)methylene]indoline-5-carboxylate
   C28H28N4O3 calc. 468.56
   MS (ESI) [M+H]⁺: 469 MS (ESI) [M+H]⁻: 467
Compound **71:**
   Methyl-(3Z)-2-oxo-3-(pyridin-2-yl{[3-(pyrrolidin-1-ylmethyl)phenyl]amino}methylene)indoline-5-carboxylate
   C27H26N4O3 calc. 454.53
   MS (ESI) [M+H]⁺: 455 MS (ESI) [M+H]⁻: 453
Compound **72:**
   Methyl-(3Z)-2-oxo-3-[{[4-(piperidin-1-ylmethyl)phenyl]amino}(pyridin-4-yl)methylene]indoline-5-carboxylate
   C28H28N4O3 calc. 468.56
   MS (ESI) [M+H]⁺: 469 MS (ESI) [M+H]⁻: 467
Compound **73:**
   Methyl-(3Z)-3-[{[4-(morpholin-4-ylmethyl)phenyl]amino}(pyridin-4-yl)methylene]-2-oxoindoline-5-carboxylate
   C27H26N4O4 calc. 470.53
   MS (ESI) [M+H]⁺: 471 MS (ESI) [M+H]⁻: 469
Compound **74:**
   Methyl-(3Z)-2-oxo-3-(pyridin-4-yl{[4-(pyrrolidin-1-ylmethyl)phenyl]amino}methylene)indoline-5-carboxylate
   C27H26N4O3 calc. 454.53
   MS (ESI) [M+H]⁺: 455 MS (ESI) [M+H]⁻: 452
Compound **75:**
   Methyl-(3*Z*)-3-[({4-[(diethylamino)methyl]phenyl}amino)(pyridin-4-yl)methylene]-2-oxoindoline-5-carboxylate
   C27H26N4O3 calc. 454.53
   MS (ESI) [M+H]⁺: 455 MS (ESI) [M+H]⁻: 452
Compound **76:**
   Methyl-(3Z)-2-oxo-3-[{[4-(pyrrolidin-1-ylmethyl)phenyl]amino}(3-thienyl)methylene]indoline-5-carboxylate
   C26H25N3O3S calc. 459.57
   MS (ESI) [M+H]⁺: 460 MS (ESI) [M+H]⁻: 458
Compound **77:**
   Methyl-(3Z)-2-oxo-3-[{[4-(piperidin-1-ylmethyl)phenyl]amino}(3-thienyl)methylene]indoline-5-carboxylate
   C27H27N3O3S calc. 473.60
   MS (ESI) [M+H]⁺: 474 MS (ESI) [M+H]⁻: 472
Compound **78:**
   Methyl-(3Z)-3-[{[4-(morpholin-4-ylmethyl)phenyl]amino}(3-thienyl)methylene]-2-oxoindoline-5-carboxylate
   C26H25N3O4S calc. 475.57
   MS (ESI) [M+H]⁺: 476 MS (ESI) [M+H]⁻: 474
Compound **79:**
   Methyl-(3Z)-3-[({4-[(4-methylpiperazin-1-yl)methyl]phenyl}amino)(3-thienyl)methylene]-2-oxoindoline-5-carboxylate
   C27H28N4O3S calc. 488.61
   MS (ESI) [M+H]⁺: 489 MS (ESI) [M+H]⁻: 487
Compound **80:**
   Methyl-(3Z)-2-oxo-3-[{[3-(pyrrolidin-1-ylmethyl)phenyl]amino}(3-thienyl)methylene]indoline-5-carboxylate
   C26H25N3O3S calc. 459.57
   MS (ESI) [M+H]⁺: 460 MS (ESI) [M+H]⁻: 468
Compound **81:**
   Methyl-(3Z)-2-oxo-3-[{[3-(piperidin-1-ylmethyl)phenyl]amino}(3-thienyl)methylene]indoline-5-carboxylate
   C27H27N3O3S calc. 473.60
   MS (ESI) [M+H]⁺: 474 MS (ESI) [M+H]⁻: 472
Compound **82:**
   Methyl-(3Z)-3-[({3-[(4-methylpiperazin-1-yl)methyl]phenyl}amino)(3-thienyl)methylene]-2-oxoindoline-5-carboxylate
   C27H28N4O3S calc. 488.61
   MS (ESI) [M+H]⁺: 489 MS (ESI) [M+H]⁻: 487
Compound **83:**
   Methyl-(3Z)-3-[{[3-(morpholin-4-ylmethyl)phenyl]amino}(3-thienyl)methylene]-2-oxoindoline-5-carboxylate
   C26H25N3O4S calc. 475.57
   MS (ESI) [M+H]⁺: 475 MS (ESI) [M+H]⁻: 473
Compound **84:**
   Methyl-(3Z)-3-(3-furyl{[4-(pyrrolidin-1-ylmethyl)phenyl]amino}methylene)-2-oxoindoline-5-carboxylate
   C26H25N3O4 calc. 443.51
   MS (ESI) [M+H]⁺: 444 MS (ESI) [M+H]⁻: 442
Compound **85:**
   Methyl-(3Z)-3-(3-furyl{[4-(piperidin-1-ylmethyl)phenyl]amino}methylene)-2-oxo-*N-*[(1*R*)-1-phenylpropyl]indoline-5-carboxamide
   C27H27N3O4 calc. 457.53
   MS (ESI) [M+H]⁺: 458 MS (ESI) [M+H]⁻: 456
Compound **86:**
   Methyl-(3*Z*)-3-(2-furyl{[4-(piperidin-1-ylmethyl)phenyl]amino}methylene)-2-oxoindoline-5-carboxylate
   C27H27N3O4 calc. 457.53
   MS (ESI) [M+H]⁺: 458 MS (ESI) [M+H]⁻: 456
Compound **87:**
   Methyl-(3Z)-3-[(1-methyl-1*H*-pyrazol-4-yl){[4-(pyrrolidin-1-ylmethyl)phenyl]amino}methylene]-2-oxoindoline-5-carboxylate
   C26H27N5O3 calc. 457.54
   MS (ESI) [M+H]⁺: 458 MS (ESI) [M+H]⁻: 456
Compound **88:**
   Methyl-(3Z)-3-[(1-methyl-1*H*-pyrazol-4-yl){[4-(piperidin-1-ylmethyl)phenyl]amino}methylene]-2-oxoindoline-5-carboxylate
   C27H29N5O3 calc. 471.56
   MS (ESI) [M+H]⁺: 472 MS (ESI) [M+H]⁻: 470
Compound **89:**
   Methyl-(3Z)-3-[(1-methyl-1*H*-pyrazol-4-yl){[3-(pyrrolidin-1-ylmethyl)phenyl]amino}methylene]-2-oxoindoline-5-carboxylate
   C26H27N5O3 calc. 457.54
   MS (ESI) [M+H]⁺: 458 MS (ESI) [M+H]⁻: 456
Compound **90:**
   Methyl-(3Z)-3-[(1-methyl-1*H*-indol-5-yl){[4-(pyrrolidin-1-ylmethyl)phenyl]amino}methylene]-2-oxoindoline-5-carboxylate
   C31H30N4O3 calc. 506.61
   MS (ESI) [M+H]⁺: 507 MS (ESI) [M+H]⁻: 505
Compound **91:**
   Methyl-(3Z)-3-[(1-methyl-1*H*-indol-5-yl){[4-(piperidin-1-ylmethyl)phenyl]amino}methylene]-2-oxoindoline-5-carboxylate
   C32H32N4O3 calc. 520.64
   MS (ESI) [M+H]⁺: 521 MS (ESI) [M+H]⁻: 519
Compound **92:**
   Methyl-(3Z)-3-[(1-methyl-1*H*-indol-5-yl){[3-(pyrrolidin-1-ylmethyl)phenyl]amino}methylene]-2-oxoindoline-5-carboxylate
   C31H30N4O3 calc. 506.61
   MS (ESI) [M+H]⁺: 507 MS (ESI) [M+H]⁻: 505
Compound **93:**
   Methyl-(3Z)-3-[(1-methyl-1*H*-indol-5-yl){[3-(piperidin-1-ylmethyl)phenyl]amino}methylene]-2-oxoindoline-5-carboxylate
   C32H32N4O3 calc. 520.64
   MS (ESI) [M+H]⁺: 521 MS (ESI) [M+H]⁻: 519
Compound **94:**
   Methyl-(3Z)-3-(cyclopropyl{[4-(pyrrolidin-1-ylmethyl)phenyl]amino}methylene)-2-oxoindoline-5-carboxylate
   C25H27N3O3 calc. 417.51
   MS (ESI) [M+H]⁺: 418 MS (ESI) [M+H]⁻: 416
Compound **95:**
   Methyl-(3Z)-3-(cyclopropyl{[4-(piperidin-1-ylmethyl)phenyl]amino}methylene)-2-oxoindoline-5-carboxylate
   C26H29N3O3 calc. 431.54
   MS (ESI) [M+H]⁺: 432 MS (ESI) [M+H]⁻: 430
Compound **96:**
   Methyl-(3Z)-3-(cyclohexyl{[4-(piperidin-1-ylmethyl)phenyl]amino}methylene)-2-oxoindoline-5-carboxylate
   C29H35N3O3 calc. 473.62
   MS (ESI) [M+H]⁺: 474 MS (ESI) [M+H]⁻: 472
Compound **97:**
   Methyl-(3Z)-2-oxo-3-(1-{[4-(piperidin-1-ylmethyl)phenyl]amino}propylidene)indoline-5-carboxylate
   C25H29N3O3 calc. 419.53
   MS (ESI) [M+H]⁺: 420 MS (ESI) [M+H]⁻ : 419
Compound **98:**
   Methyl-(3Z)-3-(2-methyl-1-{[4-(piperidin-1-ylmethyl)phenyl]amino}propylidene)-2-oxoindoline-5-carboxylate
   C26H31N3O3 calc. 433.56
   MS (ESI) [M+H]⁺: 434 MS (ESI) [M+H]⁻: 432

### Step D

### (3Z)-3-[({4-[(4-methylpiperazin-1-yl)methyl]phenyl}amino)(pyridin-4-yl)methylene]-2-oxoindoline-5-carboxylic acid

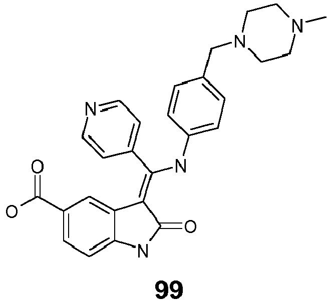

10 mmol (4.83g) Methyl-(3Z)-3-[({4-[(4-methylpiperazin-1-yl)methyl]phenyl}amino) (pyridin-4-yl)methylene]-2-oxoindoline-5-carboxylate was suspended in mixture of 50 ml metanol and 10 ml water and 20 mmol (0.80 g) NaOH was added and stirred at 60 °C for 4-6 hours. The reaction was monitored by TLC. After the completion the pH was adjusted to pH = 4-5 and the precipitated product was filtered off.
C27H27N5O3 calc. 469.55
MS (ESI) [M+H]⁺: 470 MS (ESI) [M+H]⁻: 468

The following compounds were obtained according to the procedure described by **example 2, step D**
Compound **100:**
   (3Z)-3-[({4-[(4-methylpiperazin-1-yl)methyl]phenyl}amino)(pyridin-3-yl)methylene]-2-oxoindoline-5-carboxylic acid
   C27H27N5O3 calc. 469.55
   MS (ESI) [M+H]⁺: 470 MS (ESI) [M+H]⁻ : 468
Compound **101:**
   (3*Z*)-3-[{[4-(morpholin-4-ylmethyl)phenyl]amino}(pyridin-3-yl)methylene]-2-oxoindoline-5-carboxylic acid
   C26H24N4O4 calc. 456.51
   MS (ESI) [M+H]⁺: 457 MS (ESI) [M+H]⁻: 455
Compound **102:**
   (3*Z*)-2-oxo-3-[{[4-(piperidin-1-ylmethyl)phenyl]amino}(pyridin-3-yl)methylene]indoline-5-carboxylic acid
   C27H26N4O3 calc. 454.53
   MS (ESI) [M+H]⁺: 456 MS (ESI) [M+H]⁻: 453
Compound **103:**
   (3*Z*)-2-oxo-3-(pyridin-3-yl{[4-(pyrrolidin-1-ylmethyl)phenyl]amino}methylene)indoline-5-carboxylic acid
   C26H24N4O3 calc. 440.51
   MS (ESI) [M+H]⁺: 441 MS (ESI) [M+H]⁻: 439
Compound **104:**
   (3Z)-3-[({3-[(4-methylpiperazin-1-yl)methyl]phenyl}amino)(pyridin-3-yl)methylene]-2-oxoindoline-5-carboxylic acid
   C27H27N5O3 calc. 469.55
   MS (ESI) [M+H]⁺: 470 MS (ESI) [M+H]⁻: 468
Compound **105:**
   (3Z)-2-oxo-3-[{[3-(piperidin-1-ylmethyl)phenyl]amino}(pyridin-3-yl)methylene]indoline-5-carboxylic acid
   C27H26N4O3 calc. 454.53
   MS (ESI) [M+H]⁺: 455 MS (ESI) [M+H]⁻: 453
Compound **106:**
   (3*Z*)-2-oxo-3-[{[4-(piperidin-1-ylmethyl)phenyl]amino}(pyridin-2-yl)methylene]indoline-5-carboxylic acid
   C27H26N4O3 calc. 454.53
   MS (ESI) [M+H]⁺: 455 MS (ESI) [M+H]⁻: 456
Compound **107:**
   (3*Z*)-2-oxo-3-(pyridin-2-yl{[3-(pyrrolidin-1-ylmethyl)phenyl]amino}methylene) indoline-5-carboxylic acid
   C26H24N4O3 calc. 440.51
   MS (ESI) [M+H]⁺: MS (ESI) [M+H]⁻:
Compound **108:**
   (3*Z*)-2-oxo-3-[{[4-(piperidin-1-ylmethyl)phenyl]amino}(pyridin-4-yl)methylene] indoline-5-carboxylic acid
   C27H26N4O3 calc. 454.53
   MS (ESI) [M+H]⁺: 455 MS (ESI) [M+H]⁻: 453
Compound **109:**
   (3*Z*)-3-[{[4-(morpholin-4-ylmethyl)phenyl]amino}(pyridin-4-yl)methylene]-2-oxoindoline-5-carboxylic acid
   C26H24N4O4 calc. 456.51
   MS (ESI) [M+H]⁺: 457 MS (ESI) [M+H]⁻: 455
Compound **110:**
   (3*Z*)-2-oxo-3-(pyridin-4-yl{[4-(pyrrolidin-1-ylmethyl)phenyl]amino}methylene)indoline-5-carboxylic acid
   C26H24N4O3 calc. 440.51
   MS (ESI) [M+H]⁺: 441 MS (ESI) [M+H]⁻: 439
Compound **111:**
   (3*Z*)-3-[({4-[(diethylamino)methyl]phenyl}amino)(pyridin-4-yl)methylene]-2-oxoindoline-5-carboxylic acid
   C26H26N4O3 calc. 442.52
   MS (ESI) [M+H]⁺: 443 MS (ESI) [M+H]⁻: 441
Compound **112:**
   (3*Z*)-3-[({4-[(diethylamino)methyl]phenyl}amino)(pyridin-3-yl)methylene]-2-oxoindoline-5-carboxylic acid
   C26H26N4O3 calc. 442.52
   MS (ESI) [M+H]⁺: 443 MS (ESI) [M+H]⁻: 441
Compound **113:**
   (3*Z*)-2-oxo-3-[{[4-(pyrrolidin-1-ylmethyl)phenyl]amino}(3-thienyl)methylene]indoline-5-carboxylic acid
   C25H23N3O3S calc. 445.54
   MS (ESI) [M+H]⁺: 446 MS (ESI) [M+H]⁻: 444
Compound **114:**
   (3*Z*)-2-oxo-3-[{[4-(piperidin-1-ylmethyl)phenyl]amino}(3-thienyl)methylene]indoline-5-carboxylic acid
   C26H25N3O3S calc. 459.57
   MS (ESI) [M+H]⁺: 460 MS (ESI) [M+H]⁻: 458
Compound **115:**
   (3*Z*)-3-[{[4-(morpholin-4-ylmethyl)phenyl]amino}(3-thienyl)methylene]-2-oxoindoline-5-carboxylic acid
   C25H23N3O4S calc. 461.54
   MS (ESI) [M+H]⁺: 462 MS (ESI) [M+H]⁻ : 460
Compound **116:**
   (3*Z*)-3-[({4-[(4-methylpiperazin-1-yl)methyl]phenyl}amino)(3-thienyl)methylene]-2-oxoindoline-5-carboxylic acid
   C26H26N4O3S calc. 474.59
   MS (ESI) [M+H]⁺: 475 MS (ESI) [M+H]⁻ : 473
Compound **117:**
   (3*Z*)-2-oxo-3-[{[3-(pyrrolidin-1-ylmethyl)phenyl]amino}(3-thienyl)methylene]indoline-5-carboxylic acid
   C25H23N3O3S calc. 445.54
   MS (ESI) [M+H]⁺: 446 MS (ESI) [M+H]⁻ : 444
Compound **118:**
   (3*Z*)-2-oxo-3-[{[3-(piperidin-1-ylmethyl)phenyl]amino}(3-thienyl)methylene]indoline-5-carboxylic acid
   C26H25N3O3S calc. 459.57
   MS (ESI) [M+H]⁺: 460 MS (ESI) [M+H]⁻ : 458
Compound **119:**
   (3*Z*)-3-[({3-[(4-methylpiperazin-1-yl)methyl]phenyl}amino)(3-thienyl)methylene]-2-oxoindoline-5-carboxylic acid
   C26H26N4O3S calc. 474.59
   MS (ESI) [M+H]⁺: 475 MS (ESI) [M+H]⁻ : 473
Compound **120:**
   (3*Z*)-3-[{[3-(morpholin-4-ylmethyl)phenyl]amino}(3-thienyl)methylene]-2-oxoindoline-5-carboxylic acid
   C25H23N3O4S calc. 461.54
   MS (ESI) [M+H]⁺: 462 MS (ESI) [M+H]⁻ : 460
Compound **121:**
   (3*Z*)-3-(3-furyl{[4-(pyrrolidin-1-ylmethyl)phenyl]amino}methylene)-2-oxoindoline-5-carboxylic acid
   C25H23N3O4 calc. 429.48
   MS (ESI) [M+H]⁺: 430 MS (ESI) [M+H]⁻ : 431
Compound **122:**
   (3*Z*)-3-(3-furyl{[4-(piperidin-1-ylmethyl)phenyl]amino}methylene)-2-oxoindoline-5-carboxylic acid
   C26H25N3O4 calc. 443.51
   MS (ESI) [M+H]⁺: 444 MS (ESI) [M+H]⁻ : 442
Compound **123:**
   (3*Z*)-3-[(1-methyl-1*H*-pyrazol-4-yl){[4-(pyrrolidin-1-ylmethyl)phenyl]amino}methylene]-2-oxoindoline-5-carboxylic acid
   C25H25N5O3 calc. 443.51
   MS (ESI) [M+H]⁺: 444 MS (ESI) [M+H]⁻ : 442
Compound **124:**
   (3*Z*)-3-[(1-methyl-1*H*-pyrazol-4-yl){[4-(piperidin-1-ylmethyl)phenyl]amino}methylene]-2-oxoindoline-5-carboxylic acid
   C26H27N5O3 calc. 457.54
   MS (ESI) [M+H]⁺: 458 MS (ESI) [M+H]⁻ : 456
Compound **125:**
   (3*Z*)-3-[(1-methyl-1*H*-pyrazol-4-yl){[3-(pyrrolidin-1-ylmethyl)phenyl]amino}methylene]-2-oxoindoline-5-carboxylic acid
   C25H25N5O3 calc. 443.51
   MS (ESI) [M+H]⁺: 444 MS (ESI) [M+H]⁻ : 442
Compound **126:**
   (3*Z*)-3-[(1-methyl-1*H*-indol-5-yl){[4-(pyrrolidin-1-ylmethyl)phenyl]amino}methylene]-2-oxoindoline-5-carboxylic acid
   C30H28N4O3 calc. 492.58
   MS (ESI) [M+H]⁺: 493 MS (ESI) [M+H]⁻ : 491
Compound **127:**
   (3*Z*)-3-[(1-methyl-1*H*-indol-5-yl){[4-(piperidin-1-ylmethyl)phenyl]aminolmethylene]-2-oxoindoline-5-carboxylic acid
   C31H30N4O3 calc. 506.61
   MS (ESI) [M+H]⁺: 507 MS (ESI) [M+H]⁻ : 504
Compound **128:**
   (3*Z*)-3-[(1-methyl-1*H*-indol-5-yl){[3-(pyrrolidin-1-ylmethyl)phenyl]amino}methylene]-2-oxoindoline-5-carboxylic acid
   C30H28N4O3 calc. 492.58
   MS (ESI) [M+H]⁺: 493 MS (ESI) [M+H]⁻ : 491
Compound **129:**
   (3*Z*)-3-[(1-methyl-1*H*-indol-5-yl){[3-(piperidin-1-ylmethyl)phenyl]amino}methylene]-2-oxoindoline-5-carboxylic acid
   C31H30N4O3 calc. 506.61
   MS (ESI) [M+H]⁺: 507 MS (ESI) [M+H]⁻ : 505
Compound **130:**
   (3*Z*)-3-(cyclopropyl{[4-(pyrrolidin-1-ylmethyl)phenyl]amino}methylene)-2-oxoindoline-5-carboxylic acid
   C24H25N3O3 calc. 403.49
   MS (ESI) [M+H]⁺: 404 MS (ESI) [M+H]⁻ : 402
Compound **131:**
   (3*Z*)-3-(cyclopropyl{[4-(piperidin-1-ylmethyl)phenyl]amino}methylene)-2-oxoindoline-5-carboxylic acid
   C25H27N3O3 calc. 417.51
   MS (ESI) [M+H]⁺: 418 MS (ESI) [M+H]⁻ : 416
Compound **132:**
   (3*Z*)-3-(cyclohexyl{[4-(piperidin-1-ylmethyl)phenyl]amino}methylene)-2-oxoindoline-5-carboxylic acid
   C28H33N3O3 calc. 459.59
   MS (ESI) [M+H]⁺: 460 MS (ESI) [M+H]⁻ : 458
Compound **133:**
   (3*Z*)-2-oxo-3-(1-{[4-(piperidin-1-ylmethyl)phenyl]amino}propylidene)indoline-5-carboxylic acid
   C24H27N3O3 calc. 405.50
   MS (ESI) [M+H]⁺: 406 MS (ESI) [M+H]⁻ : 404
Compound **134:**
   (3*Z*)-3-(2-methyl-1-{[4-(piperidin-1-ylmethyl)phenyl]amino}propylidene)-2-oxoindoline-5-carboxylic acid
   C25H29N3O3 calc. 419.53
   MS (ESI) [M+H]⁺: 420 MS (ESI) [M+H]⁻ : 418

### Step E

### (3Z)-3-[({4-[(4-methylpiperazin-1-yl)methyl]phenyl}amino)(pyridin-4-yl)methylene]-2-oxo-N-[(1R)-1-phenylpropyl]indoline-5-carboxamide

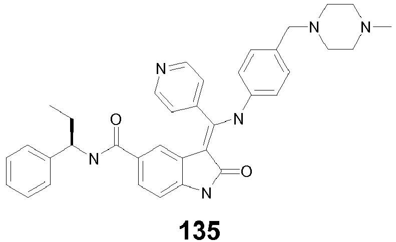

0.5 mmol (230 mg) (3*Z*)-3-[({4-[(4-methylpiperazin-1-yl)methyl]phenyl}amino) (pyridin-4-yl)methylene]-2-oxoindoline-5-carboxylic acid, 0.5 mmol (68 mg) (1R)-1-phenylpropan-1-amine, 0.6 mmol (0.450 g) HATU/HBTU , 1.5 mmol (0.19 g, 0.26 ml)DIPEA were stirred in 8 ml dry DMF at 50 °C for 2-5 hours. The solvent was removed under reduced pressure and the residue was taken up with 20-30 ml sat. K₂CO₃ and extracted with 3x30 ml ethyl-acetate. The organic layer was separated and and dried over MgSO₄. The dessicant was filtered off and the solvent was removed under reduced pressure. The product was purified by column chromatography (Kieselgel, chloroform/methanol (sat. with NH₃15:1)
C36H38N6O2 calc.: 586.74
MS (ESI) [M+H]⁺: 587 MS (ESI) [M+H]⁻ : 585

The following compounds were obtained according to the procedure described by **example 2, step E**

### (3Z)-N-benzyl-3-[({4-[(4-methylpiperazin-1-yl)methyl]phenyl}amino)(pyridin-3-yl)methylene]-2-oxoindoline-5-carboxamide

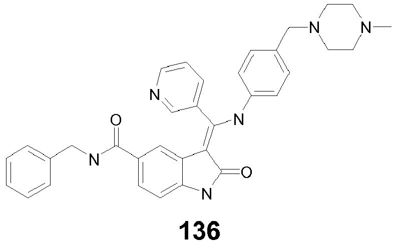

Compound **136** was obtained by reaction of (3*Z*)-3-[({4-[(4-methylpiperazin-1-yl)methyl]phenyl}amino)(pyridin-3-yl)methylene]-2-oxoindoline-5-carboxylic acid and benzyl-amine according to **Step E.**
C34H34N6O2 calc.: 558.69
MS (ESI) [M+H]⁺: 559 MS (ESI) [M+H]⁻ : 557

### (3Z)-N-(4-fluorobenzyl)-3-[({4-[(4-methylpiperazin-1-yl)methyl]phenyl}amino)(pyridin-3-yl)methylene]-2-oxoindoline-5-carboxamide

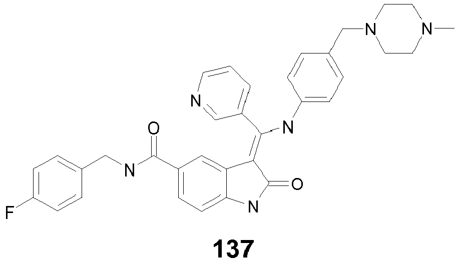

C34H33FN6O2 calc.: 576.68
MS (ESI) [M+H]⁺: 577 MS (ESI) [M+H]⁻ : 575

Compound **137** was obtained by reaction of (3*Z*)-3-[({4-[(4-methylpiperazin-1-yl)methyl]phenyl}amino)(pyridin-3-yl)methylene]-2-oxoindoline-5-carboxylic acid and benzyl-amine according to **Step E.**

### (3Z)-3-[({4-[(4-methylpiperazin-1-yl)methyl]phenyl}amino)(pyridin-3-yl)methylene]-2-oxo-N-[(1R)-1-phenylethyl]indoline-5-carboxamide

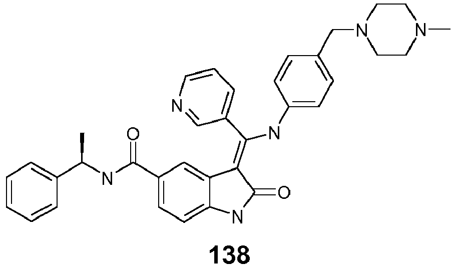

Compound **138** was obtained by reaction of (3*Z*)-3-[({4-[(4-methylpiperazin-1-yl)methyl]phenyl} amino)(pyridin-3-yl)methylene]-2-oxoindoline-5-carboxylic acid and (1*R*)-1-phenylethanamine according to **Step E.**
C35H36N6O2 calc.: 572.72
MS (ESI) [M+H]⁺: 573 MS (ESI) [M+H]⁻ : 571

### (3Z)-3-[({4-[(4-methylpiperazin-1-yl)methyl]phenyl}amino)(pyridin-3-yl)methylene]-2-oxo-N-[(1R)-1-phenylpropyl]indoline-5-carboxamide

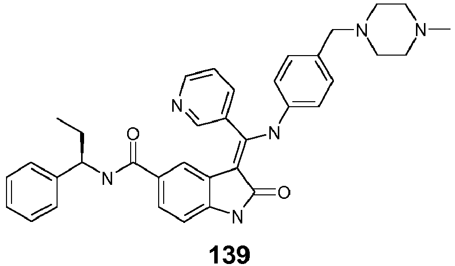

Compound **139** was obtained by reaction of (3*Z*)-3-[({4-[(4-methylpiperazin-1-yl)methyl]phenyl} amino)(pyridin-3-yl)methylene]-2-oxoindoline-5-carboxylic acid and (1*R*)-1-phenylpropan-1-amine according to **Step E.**
C36H38N6O2 calc.: 586.74
MS (ESI) [M+H]⁺: 587 MS (ESI) [M+H]⁻ : 585

### (3Z)-2-oxo-N-[(1R)-1-phenylethyl]-3-[{[4-(piperidin-1-ylmethyl)phenyl]amino}(pyridin-3-yl)methylene]indoline-5-carboxamide

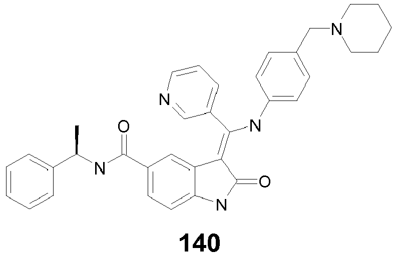

Compound **140** was obtained by reaction (3*Z*)-2-oxo-3-[{[4-(piperidin-1-ylmethyl)phenyl]amino} (pyridin-3-yl)methylene]indoline-5-carboxylic acid and (1*R*)-1-phenylethanamine according to **Step E.**
C35H35N5O2 calc.: 557.70
MS (ESI) [M+H]⁺: 558 MS (ESI) [M+H]⁻ : 556

### (3Z)-2-oxo-N-[(1S)-1-phenylethyl]-3-[{[4-(piperidin-1-ylmethyl)phenyl]amino}(pyridin-3-yl)methylene]indoline-5-carboxamide

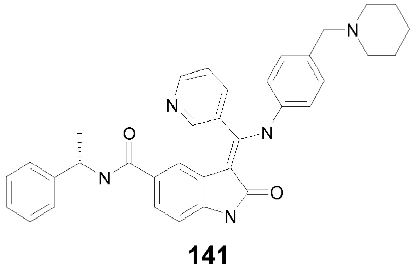

Compound **141** was obtained by reaction (3*Z*)-2-oxo-3-[{[4-(piperidin-1-ylmethyl)phenyl]amino} (pyridin-3-yl)methylene]indoline-5-carboxylic acid and (1*S*)-1-phenylethanamine according to **Step E.**
C35H35N5O2 calc.: 557.70
MS (ESI) [M+H]⁺: 558 MS (ESI) [M+H]⁻ : 556

### (3Z)-2-oxo-N-[(1R)-1-phenylpropyl]-3-[{[4-(piperidin-1-ylmethyl)phenyl]amino}(pyridin-3-yl)methylene]indoline-5-carboxamide

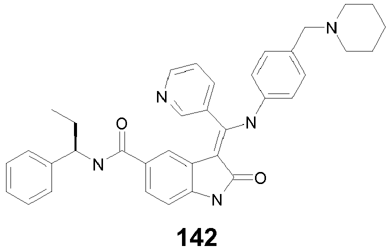

Compound **142** was obtained by reaction (3*Z*)-2-oxo-3-[{[4-(piperidin-1-ylmethyl)phenyl]amino} (pyridin-3-yl)methylene]indoline-5-carboxylic acid and (1R)-1-phenylpropan-1-amine according to **Step E.**
C36H37N5O2 calc.: 571.73
MS (ESI) [M+H]+: 572 MS (ESI) [M+H]- : 570

### (3Z)-3-[({4-[(diethylamino)methyl]phenyl}amino)(pyridin-3-yl)methylene]-2-oxo-N-[(1 R)-1-phenylpropyl]indoline-5-carboxamide

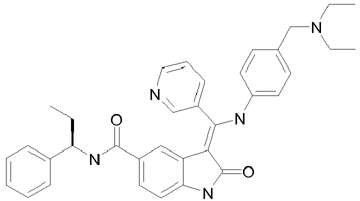

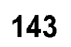

Compound **143** was obtained by reaction (3Z)-3-[({4-[(diethylamino)methyl]phenyl}amino)(pyridin-3-yl)methylene]-2-oxoindoline-5-carboxylic acid and (1*R*)-1-phenylpropan-1-amine according to **Step E.**
C35H37N5O2 calc.: 559.72
MS (ESI) [M+H]⁺: 560 MS (ESI) [M+H]⁻ : 558

### (3Z)-3-[({4-[(diethylamino)methyl]phenyl}amino)(pyridin-3-yl)methylene]-2-oxo-N-[(1R)-1-phenylethyl]indoline-5-carboxamide

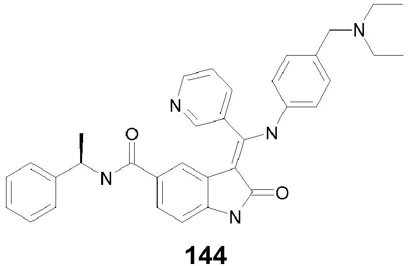

Compound **144** was obtained by reaction (3*Z*)-3-[({4-[(diethylamino)methyl]phenyl} amino)(pyridin-3-yl)methylene]-2-oxoindoline-5-carboxylic acid and (1*R*)-1-phenylethanamine according to **Step E.**
C34H35N5O2 calc.: 545.69
MS (ESI) [M+H]⁺: 546 MS (ESI) [M+H]⁻ : 544

### (3Z)-2-oxo-N-[(1S)-1-phenylpropyl]-3-[{[4-(piperidin-1-ylmethyl)phenyl]amino}(pyridin-3-yl)methylene]indoline-5-carboxamide

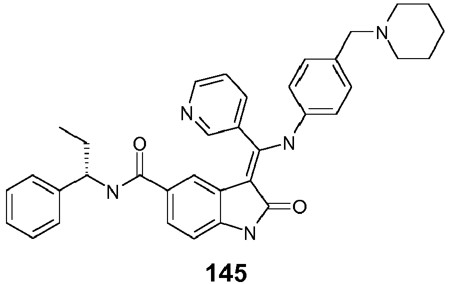

Compound **145** was obtained by reaction (3*Z*)-2-oxo-3-[{[4-(piperidin-1-ylmethyl)phenyl]amino} (pyridin-3-yl)methylene]indoline-5-carboxylic acid and (1*S*)-1-phenylpropan-1-amine according to **Step E.**
C36H37N5O2 calc.: 571.73
MS (ESI) [M+H]⁺: 572 MS (ESI) [M+H]⁻ : 570

### (3Z)-3-[{[4-(morpholin-4-ylmethyl)phenyl]amino}(pyridin-3-yl)methylene]-2-oxo-N-[(1R)-1-phenylethyl]indoline-5-carboxamide

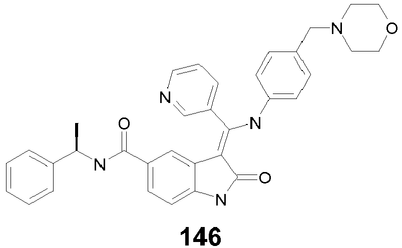

Compound **146** was obtained by reaction and (3*Z*)-3-[{[4-(morpholin-4-ylmethyl)phenyl]amino} (pyridin-3-yl)methylene]-2-oxoindoline-5-carboxylic acid and (1R)-1-phenylethanamine according to **Step E.**
C34H33N5O3 calc.: 559.67
MS (ESI) [M+H]⁺: 560 MS (ESI) [M+H]⁻ : 558

### (3Z)-3-[{[4-(morpholin-4-ylmethyl)phenyl]amino}(pyridin-3-yl)methylene]-2-oxo-N-[(1 R)-1-phenylpropyl]indoline-5-carboxamide

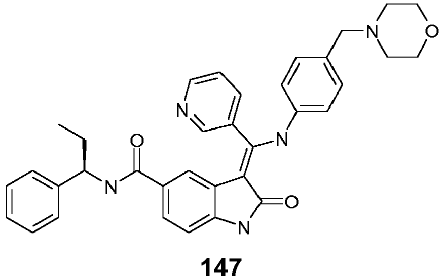

Compound **147** was obtained by reaction of (3*Z*)-3-[{[4-(morpholin-4-ylmethyl)phenyl]amino} (pyridin-3-yl)methylene]-2-oxoindoline-5-carboxylic acid and (1R)-1-phenylpropan-1-amine according to **Step E.**
C35H35N5O3 calc.: 573.70
MS (ESI) [M+H]⁺: 574 MS (ESI) [M+H]⁻ : 572

### (3Z)-3-[({3-[(4-methylpiperazin-1-yl)methyl]phenyl}amino)(pyridin-3-yl)methylene]-2-oxo-N-[(1R)-1-phenylethyl]indoline-5-carboxamide

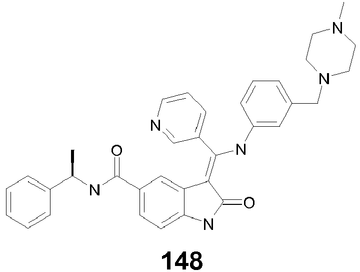

Compound **148** was obtained by reaction of (3*Z*)-3-[({3-[(4-methylpiperazin-1-yl)methyl]phenyl} amino)(pyridin-3-yl)methylene]-2-oxoindoline-5-carboxylic acid and (1 R)-1-phenylethanamine according to **Step E.**
C35H36N6O2 calc.: 572.72
MS (ESI) [M+H]⁺: 573 MS (ESI) [M+H]⁻ : 571

### (3Z)-3-[({3-[(4-methylpiperazin-1-yl)methyl]phenyl}amino)(pyridin-3-yl)methylene]-2-oxo-N-[(1R)-1-phenylpropyl]indoline-5-carboxamide

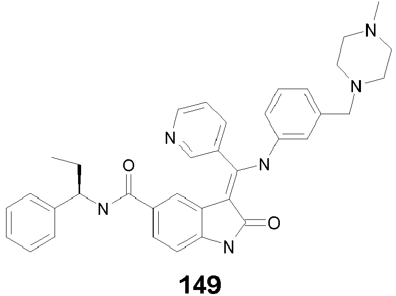

Compound **149** was obtained by reaction of (3*Z*)-3-[({3-[(4-methylpiperazin-1-yl)methyl]phenyl}amino)(pyridin-3-yl)methylene]-2-oxoindoline-5-carboxylic acid and (1R)-1-phenylpropan-1-amine according to **Step E.**
C36H38N6O2 calc.: 586.74
MS (ESI) [M+H]⁺: 587 MS (ESI) [M+H]⁻ : 585

### (3Z)-2-oxo-N-[(1R)-1-phenylpropyl]-3-[{[3-(piperidin-1-ylmethyl)phenyl]amino}(pyridin-3-yl)methylene]indoline-5-carboxamide

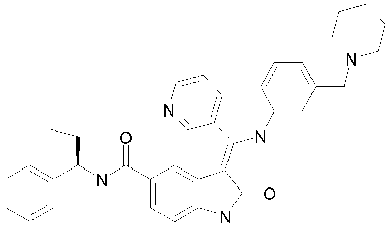

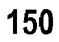

Compound **150** was obtained by reaction of (3*Z*)-2-oxo-3-[{[3-(piperidin-1-ylmethyl)phenyl]amino} (pyridin-3-yl)methylene]indoline-5-carboxylic acid and (1R)-1-phenylpropan-1-amine according to **Step E.**
C36H37N5O2 calc.: 571.73
MS (ESI) [M+H]⁺: 572 MS (ESI) [M+H]⁻ : 570

### (3Z)-2-oxo-N-[(1S)-1-phenylpropyl]-3-[{[3-(piperidin-1-ylmethyl)phenyl]amino}(pyridin-3-yl)methylene]indoline-5-carboxamide

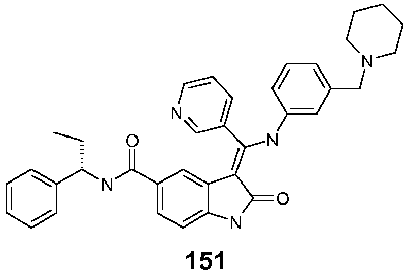

Compound 151 was obtained by reaction of (3*Z*)-2-oxo-3-[{[3-(piperidin-1-ylmethyl)phenyl]amino}(pyridin-3-yl)methylene]indoline-5-carboxylic acid and (1*S*)-1-phenylpropan-1-amine according to **Step E.**
C36H37N5O2 calc.: 571.73
MS (ESI) [M+H]⁺: 572 MS (ESI) [M+H]⁻ : 570

### (3Z)-2-oxo-N-[(1S)-1-phenylethyl]-3-[{[3-(piperidin-1-ylmethyl)phenyl]amino}(pyridin-3-yl)methylene]indoline-5-carboxamide

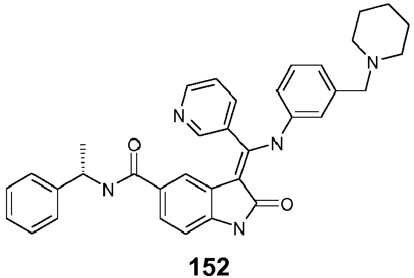

Compound **152** was obtained by reaction of (3*Z*)-2-oxo-3-[{[3-(piperidin-1-ylmethyl)phenyl]amino} (pyridin-3-yl)methylene]indoline-5-carboxylic acid and (1S)-1-phenylethanamine according to **Step E.**
C35H35N5O2 calc.: 557.70
MS (ESI) [M+H]⁺: 558 MS (ESI) [M+H]⁻ : 556

### (3Z)-2-oxo-N-[(1R)-1-phenylethyl]-3-[{[3-(piperidin-1-ylmethyl)phenyl]amino}(pyridin-3-yl)methylene]indoline-5-carboxamide

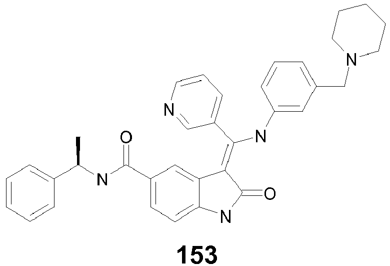

Compound **153** was obtained by reaction of (3*Z*)-2-oxo-3-[{[3-(piperidin-1-ylmethyl)phenyl]amino}(pyridin-3-yl)methylene]indoline-5-carboxylic acid and (1R)-1-phenylethanamine according to **Step E.**
C35H35N5O2 calc.: 557.70
MS (ESI) [M+H]⁺: 558 MS (ESI) [M+H]⁻ : 556

### (3Z)-2-oxo-N-[(1R)-1-phenylethyl]-3-[{[4-(piperidin-1-ylmethyl)phenyl]amino}(pyridin-2-yl)methylene]indoline-5-carboxamide

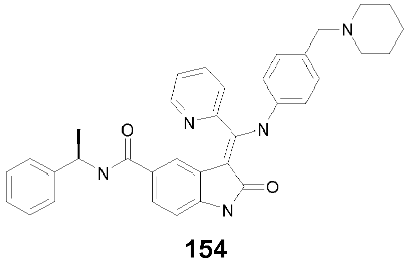

Compound **154** was obtained by reaction of and and according to (3*Z*)-2-oxo-3-[{[4-(piperidin-1-ylmethyl)phenyl]amino}(pyridin-2-yl)methylene]indoline-5-carboxylic acid and (1 R)-1-phenylethanamine **Step E.**
C35H35N5O2 calc.: 557.70
MS (ESI) [M+H]⁺: 558 MS (ESI) [M+H]⁻ : 556

### (3Z)-2-oxo-N-[(1R)-1-phenylpropyl]-3-[{[4-(piperidin-1-ylmethyl)phenyl]amino}(pyridin-2-yl)methylene]indoline-5-carboxamide

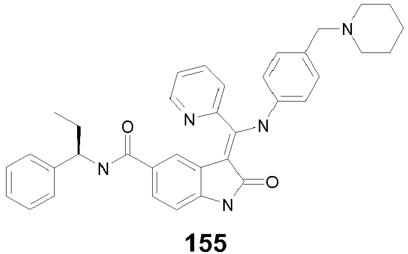

C36H37N5O2 calc.: 571.73
MS (ESI) [M+H]⁺: 572 MS (ESI) [M+H]⁻ : 570

Compound 155 was obtained by reaction of (3*Z*)-2-oxo-3-[{[4-(piperidin-1-ylmethyl)phenyl]amino}(pyridin-2-yl)methylene]indoline-5-carboxylic acid and (1 R)-1-phenylpropan-1-amine according to **Step E.**

### (3Z)-2-oxo-N-[(1R)-1-phenylethyl]-3-(pyridin-2-yl{[3-(pyrrolidin-1-ylmethyl)phenyl]amino}methylene)indoline-5-carboxamide

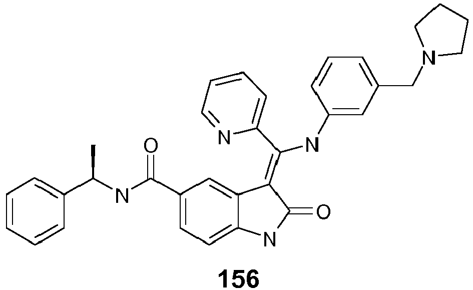

Compound **156** was obtained by reaction of (3*Z*)-2-oxo-3-(pyridin-2-yl{[3-(pyrrolidin-1-ylmethyl)phenyl]amino}methylene)indoline-5-carboxylic acid and (1 R)-1-phenylethanamine according to **Step E.**
C34H33N5O2 calc.: 543.67
MS (ESI) [M+H]⁺: 544 MS (ESI) [M+H]⁻ : 542

### (3Z)-2-oxo-N-[(1R)-1-phenylpropyl]-3-(pyridin-2-yl{[3-(pyrrolidin-1-ylmethyl)phenyl]amino}methylene)indoline-5-carboxamide

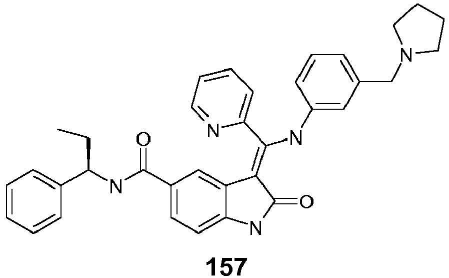

Compound **157** was obtained by reaction of (3*Z*)-2-oxo-3-(pyridin-2-yl{[3-(pyrrolidin-1-ylmethyl)phenyl]amino}methylene)indoline-5-carboxylic acid and (1R)-1-phenylpropan-1-amine according to **Step E.**
C35H35N5O2 calc.: 557.70
MS (ESI) [M+H]⁺: 558 MS (ESI) [M+H]⁻ : 556

### (3Z)-2-oxo-N-[(1R)-1-phenylethyl]-3-[{[3-(piperidin-1-ylmethyl)phenyl]amino}(pyridin-2-yl)methylene]indoline-5-carboxamide

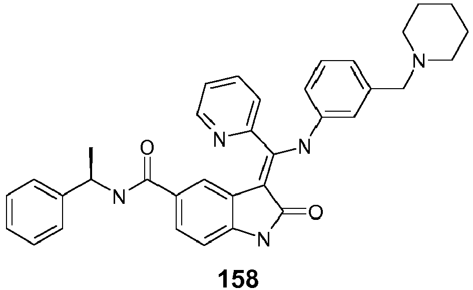

Compound **158** was obtained by reaction of (3*Z*)-2-oxo-3-[{[3-(piperidin-1-ylmethyl)phenyl]amino} (pyridin-2-yl)methylene]indoline-5-carboxylic acid and (1 R)-1-phenylethanamine according to **Step E.**
C35H35N5O2 calc.: 557.70
MS (ESI) [M+H]⁺: 558 MS (ESI) [M+H]⁻ : 556

### (3Z)-2-oxo-N-[(1R)-1-phenylpropyl]-3-[{[3-(piperidin-1-ylmethyl)phenyl]amino}(pyridin-2-yl)methylene]indoline-5-carboxamide

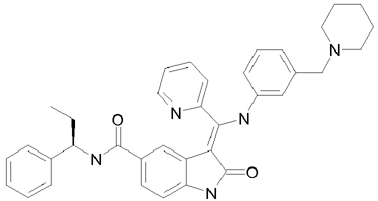

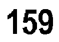

Compound **159** was obtained by reaction of (3*Z*)-2-oxo-3-[{[3-(piperidin-1-ylmethyl)phenyl]amino}(pyridin-2-yl)methylene]indoline-5-carboxylic acid and (1 R)-1-phenylpropan-1-amine according to **Step E.**
C36H37N5O2 calc.: 571.73
MS (ESI) [M+H]⁺: 572 MS (ESI) [M+H]⁻ : 570

### (3Z)-3-[({4-[(4-methylpiperazin-1-yl)methyl]phenyl}amino)(pyridin-4-yl)methylene]-2-oxo-N-[(1R)-1-phenylethyl]indoline-5-carboxamide

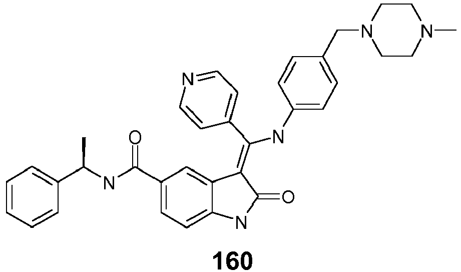

Compound **160** was obtained by reaction of and (3*Z*)-3-[({4-[(4-methylpiperazin-1-yl)methyl]phenyl}amino)(pyridin-4-yl)methylene]-2-oxoindoline-5-carboxylic acid and (1 R)-1-phenylethanamine according to **Step E.**
C35H36N6O2 calc.: 572.72
MS (ESI) [M+H]⁺: 573 MS (ESI) [M+H]⁻ : 571

### (3Z)-2-oxo-N-[(1R)-1-phenylpropyl]-3-[{[4-(piperidin-1-ylmethyl)phenyl]amino}(pyridin-4-yl)methylene]indoline-5-carboxamide

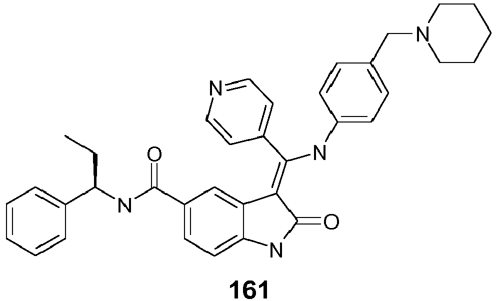

Compound **161** was obtained by reaction of (3*Z*)-2-oxo-3-[{[4-(piperidin-1-ylmethyl)phenyl]amino}(pyridin-4-yl)methylene]indoline-5-carboxylic acid and (1 R)-1-phenylethanamine according to **Step E.**
C36H37N5O2 calc.: 571.73
MS (ESI) [M+H]⁺: 572 MS (ESI) [M+H]⁻ : 570

### (3Z)-2-oxo-N-[(1S)-1-phenylpropyl]-3-[{[4-(piperidin-1-ylmethyl)phenyl]amino}(pyridin-4-yl)methylene]indoline-5-carboxamide

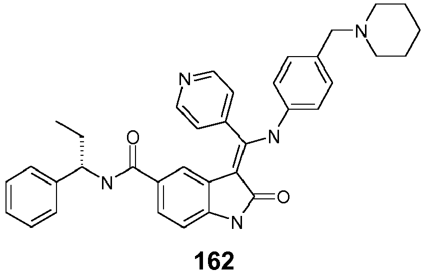

Compound **162** was obtained by reaction of (3*Z*)-2-oxo-3-[{[4-(piperidin-1-ylmethyl)phenyl]amino} (pyridin-4-yl)methylene]indoline-5-carboxylic acid and (1*S*)-1-phenylpropan-1-amine according to **Step E.**
C36H37N5O2 calc.: 571.73
MS (ESI) [M+H]⁺: 572 MS (ESI) [M+H]⁻ : 570

### (3Z)-2-oxo-N-[(1R)-1-phenylethyl]-3-[{[4-(piperidin-1-ylmethyl)phenyl]amino}(pyridin-4-yl)methylene]indoline-5-carboxamide

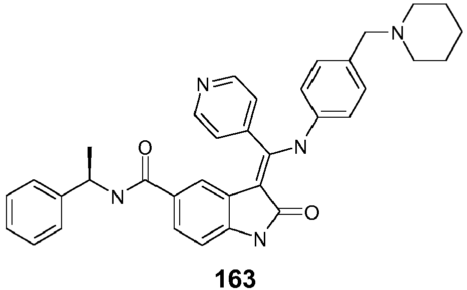

Compound **163** was obtained by reaction of (3*Z*)-2-oxo-3-[{[4-(piperidin-1-ylmethyl)phenyl]amino}(pyridin-4-yl)methylene]indoline-5-carboxylic acid and (1 R)-1-phenylethanamine according to **Step E.**
C35H35N5O2 calc.: 557.70
MS (ESI) [M+H]⁺: 558 MS (ESI) [M+H]⁻ : 556

### (3Z)-2-oxo-N-[(1S)-1-phenylethyl]-3-[{[4-(piperidin-1-ylmethyl)phenyl]amino}(pyridin-4-yl)methylene]indoline-5-carboxamide

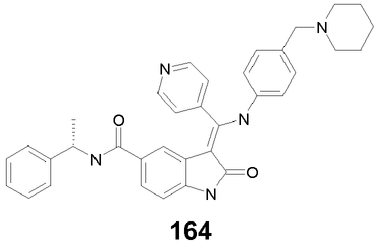

Compound **164** was obtained by reaction of (3*Z*)-2-oxo-3-[{[4-(piperidin-1-ylmethyl)phenyl]amino}(pyridin-4-yl)methylene]indoline-5-carboxylic acid and (1S)-1-phenylpropan-1-amine according to **Step E.**
C35H35N5O2 calc.: 557.70
MS (ESI) [M+H]⁺: 558 MS (ESI) [M+H]⁻ : 556

### (3Z)-3-[({4-[(diethylamino)methyl]phenyl}amino)(pyridin-4-yl)methylene]-2-oxo-N-[(1R)-1-phenylethyl]indoline-5-carboxamide

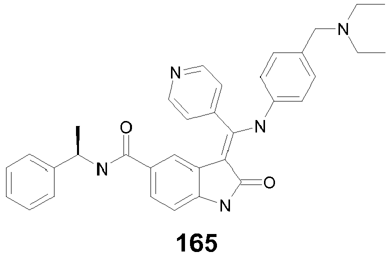

Compound **165** was obtained by reaction of (3Z)-3-[({4-[(diethylamino)methyl]phenyl}amino) (pyridin-4-yl)methylene]-2-oxoindoline-5-carboxylic acid and (1 R)-1-phenylethanamine according to **Step E.**
C34H35N5O2 calc.: 545.69
MS (ESI) [M+H]⁺: 546 MS (ESI) [M+H]⁻ : 544

### (3Z)-3-[({4-[(diethylamino)methyl]phenyl}amino)(pyridin-4-yl)methylene]-2-oxo-N-[(1 R)-1-phenylpropyl]indoline-5-carboxamide

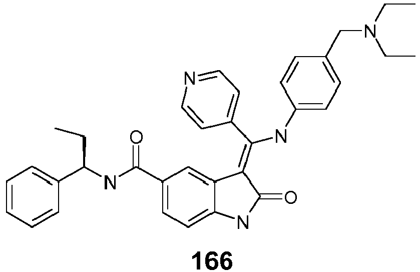

Compound **166** was obtained by reaction of (3Z)-3-[({4-[(diethylamino)methyl]phenyl}amino)(pyridin-4-yl)methylene]-2-oxoindoline-5-carboxylic acid and (1R)-1-phenylpropan-1-amine according to **Step E.**
C35H37N5O2 calc. 559.72
MS (ESI) [M+H]⁺: 560 MS (ESI) [M+H]⁻ : 558

### (3Z)-3-[{[4-(morpholin-4-ylmethyl)phenyl]amino}(pyridin-4-yl)methylene]-2-oxo-N-[(1 R)-1-phenylethyl]indoline-5-carboxamide

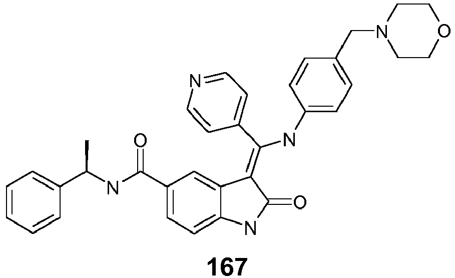

Compound **167** was obtained by reaction of (3*Z*)-3-[{[4-(morpholin-4-ylmethyl)phenyl]amino}(pyridin-4-yl)methylene]-2-oxoindoline-5-carboxylic acid and (1 R)-1-phenylethanamine according to **Step E.**
C34H33N5O3 calc.: 559.67
MS (ESI) [M+H]⁺: 560 MS (ESI) [M+H]⁻ : 558

### (3Z)-3-[{[4-(morpholin-4-ylmethyl)phenyl]amino}(pyridin-4-yl)methylene]-2-oxo-N-[(1 R)-1-phenylpropyl]indoline-5-carboxamide

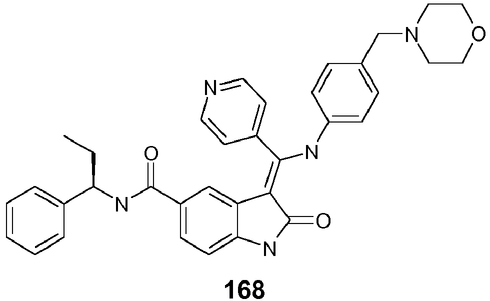

Compound **168** was obtained by reaction of (3*Z*)-3-[{[4-(morpholin-4-ylmethyl)phenyl]amino}(pyridin-4-yl)methylene]-2-oxoindoline-5-carboxylic acid and (1R)-1-phenylpropan-1-amine according to **Step E.**
C35H35N5O3 calc.: 573.70
MS (ESI) [M+H]⁺: 574 MS (ESI) [M+H]⁻ :572

### (3Z)-2-oxo-N-[(1R)-1-phenylpropyl]-3-(pyridin-4-yl{[4-(pyrrolidin-1-ylmethyl)phenyl]amino}methylene)indoline-5-carboxamide

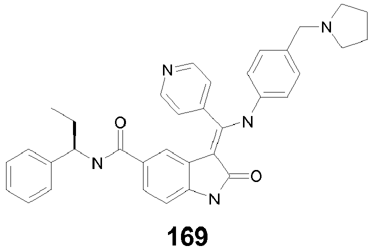

Compound **169** was obtained by reaction of (3*Z*)-2-oxo-3-(pyridin-4-yl{[4-(pyrrolidin-1-ylmethyl)phenyl]amino}methylene)indoline-5-carboxylic acid and (1 R)-1-phenylpropan-1-amine according to **Step E.**
C35H35N5O2 calc.: 557.70
MS (ESI) [M+H]⁺: 558 MS (ESI) [M+H]⁻ : 556

### (3Z)-2-oxo-N-[(1R)-1-phenylethyl]-3-(pyridin-4-yl{[4-(pyrrolidin-1-ylmethyl)phenyl]amino}methylene)indoline-5-carboxamide

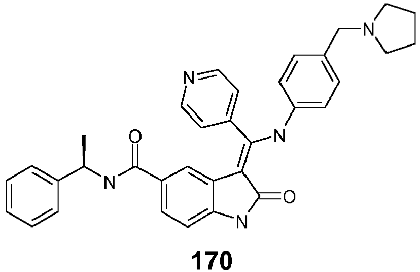

Compound **170** was obtained by reaction of (3*Z*)-2-oxo-3-(pyridin-4-yl{[4-(pyrrolidin-1-ylmethyl)phenyl]amino}methylene)indoline-5-carboxylic acid and (1 R)-1-phenylethanamine according to **Step E.**
C34H33N5O2 calc.: 543.67
MS (ESI) [M+H]⁺: 544 MS (ESI) [M+H]⁻ : 542

### (3Z)-2-oxo-N-[(1R)-1-phenylethyl]-3-[{[4-(pyrrolidin-1-ylmethyl)phenyl]amino}(3-thienyl)methylene]indoline-5-carboxamide

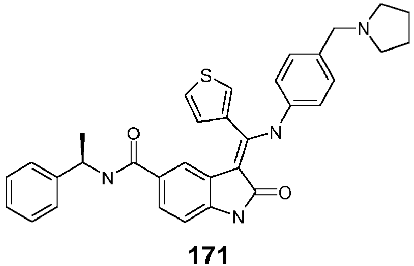

Compound **171** was obtained by reaction of (3*Z*)-2-oxo-3-[{[4-(pyrrolidin-1-ylmethyl)phenyl]amino}(3-thienyl)methylene]indoline-5-carboxylic acid and (1 R)-1-phenylethanamine according to **Step E.**
C33H32N4O2S calc.: 548.71
MS (ESI) [M+H]⁺: 549 MS (ESI) [M+H]⁻ : 547

### (3Z)-2-oxo-N-[(1R)-1-phenylpropyl]-3-[{[4-(pyrrolidin-1-ylmethyl)phenyl]amino}(3-thienyl)methylene]indoline-5-carboxamide

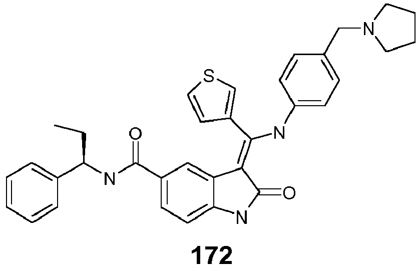

Compound **172** was obtained by reaction of (3*Z*)-2-oxo-3-[{[4-(pyrrolidin-1-ylmethyl)phenyl]amino}(3-thienyl)methylene]indoline-5-carboxylic acid and (1 R)-1-phenylpropan-1-amine according to **Step E.**
C34H34N4O2S calc.:562.74
MS (ESI) [M+H]⁺: 563 MS (ESI) [M+H]⁻ : 561

### (3Z)-2-oxo-N-[(1R)-1-phenylethyl]-3-[{[4-(piperidin-1-ylmethyl)phenyl]amino}(3-thienyl)methylene]indoline-5-carboxamide

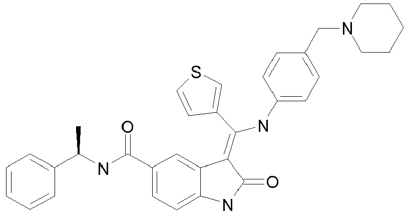

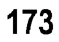

Compound **173** was obtained by reaction of (3*Z*)-2-oxo-3-[{[4-(piperidin-1-ylmethyl)phenyl]amino}(3-thienyl)methylene]indoline-5-carboxylic acid and (1 R)-1-phenylethanamine according to **Step E.**
C34H34N4O2S calc.: 562.74
MS (ESI) [M+H]⁺: 563 MS (ESI) [M+H]⁻ : 561

### (3Z)-2-oxo-N-[(1R)-1-phenylpropyl]-3-[{[4-(piperidin-1-ylmethyl)phenyl]amino}(3-thienyl)methylene]indoline-5-carboxamide

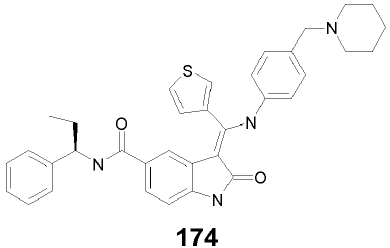

Compound **174** was obtained by reaction of (3*Z*)-2-oxo-3-[{[4-(piperidin-1-ylmethyl)phenyl]amino}(3-thienyl)methylene]indoline-5-carboxylic acid and(1R)-1-phenylpropan-1-amine according to **Step E.**
C35H36N4O2S calc.: 576.77
MS (ESI) [M+H]⁺: 577 MS (ESI) [M+H]⁻ : 575

### (3Z)-3-[{[4-(morpholin-4-ylmethyl)phenyl]amino}(3-thienyl)methylene]-2-oxo-N-[(1R)-1-phenylethyl]indoline-5-carboxamide

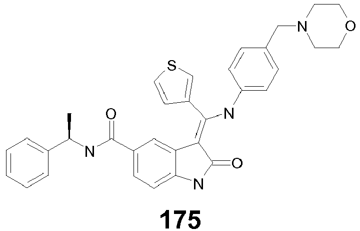

Compound **175** was obtained by reaction of (3*Z*)-3-[{[4-(morpholin-4-ylmethyl)phenyl]amino}(3-thienyl)methylene]-2-oxoindoline-5-carboxylic acid and (1 R)-1-phenylethanamine according to **Step E.**
C33H32N4O3S calc.: 564.71
MS (ESI) [M+H]⁺: 565 MS (ESI) [M+H]⁻ : 563

### (3Z)-3-[{[4-(morpholin-4-ylmethyl)phenyl]amino}(3-thienyl)methylene]-2-oxo-N-[(1 R)-1-phenylpropyl]indoline-5-carboxamide

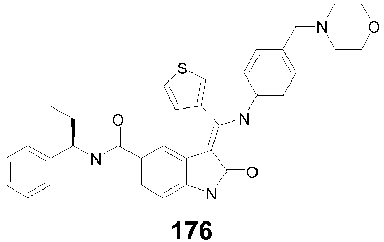

Compound **176** was obtained by reaction of (3*Z*)-3-[{[4-(morpholin-4-ylmethyl)phenyl]amino}(3-thienyl)methylene]-2-oxoindoline-5-carboxylic acid and (1R)-1-phenylpropan-1-amine according to **Step E.**
C34H34N4O3S calc.: 578.74
MS (ESI) [M+H]⁺: 579 MS (ESI) [M+H]⁻ : 577

### (3Z)-3-[({4-[(4-methylpiperazin-1-yl)methyl]phenyl}amino)(3-thienyl)methylene]-2-oxo-N-[(1R)-1-phenylethyl]indoline-5-carboxamide

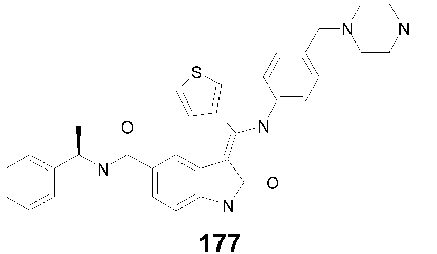

Compound **177** was obtained by reaction of (3*Z*)-3-[({4-[(4-methylpiperazin-1-yl)methyl]phenyl}amino)(3-thienyl)methylene]-2-oxoindoline-5-carboxylic acid and (1 R)-1-phenylethanamine according to **Step E.**
C34H35N5O2S calc.: 577.75
MS (ESI) [M+H]⁺: 578 MS (ESI) [M+H]⁻ : 576

### (3Z)-3-[({4-[(4-methylpiperazin-1-yl)methyl]phenyl}amino)(3-thienyl)methylene]-2-oxo-N-[(1R)-1-phenylpropyl]indoline-5-carboxamide

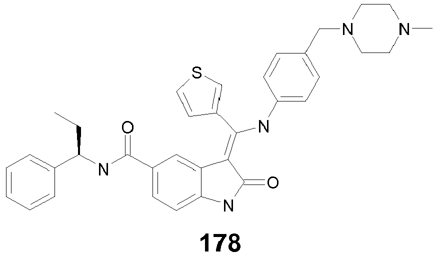

Compound **178** was obtained by reaction of (3*Z*)-3-[({4-[(4-methylpiperazin-1-yl)methyl]phenyl}amino)(3-thienyl)methylene]-2-oxoindoline-5-carboxylic acid and (1R)-1-phenylpropan-1-amine according to **Step E.**
C35H37N5O2S calc.: 591.78
MS (ESI) [M+H]⁺: 592 MS (ESI) [M+H]⁻ : 590

### (3Z)-2-oxo-N-[(1R)-1-phenylethyl]-3-[{[3-(pyrrolidin-1-ylmethyl)phenyl]amino}(3-thienyl)methylene]indoline-5-carboxamide

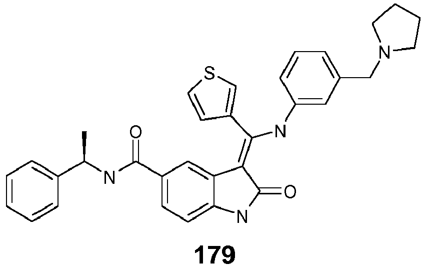

Compound **179** was obtained by reaction of (3*Z*)-2-oxo-3-[{[3-(pyrrolidin-1-ylmethyl)phenyl]amino}(3-thienyl)methylene]indoline-5-carboxylic acid and (1 R)-1-phenylethanamine according to **Step E.**
C33H32N4O2S calc.: 548.71
MS (ESI) [M+H]⁺: 549 MS (ESI) [M+H]⁻ : 547

### (3Z)-2-oxo-N-[(1R)-1-phenylpropyl]-3-[{[3-(pyrrolidin-1-ylmethyl)phenyl]amino}(3-thienyl)methylene]indoline-5-carboxamide

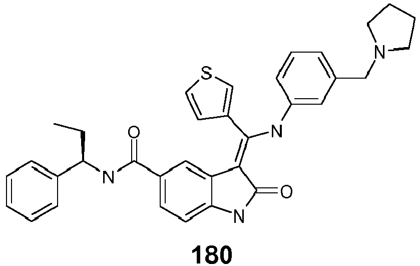

Compound **180** was obtained by reaction of (3*Z*)-2-oxo-3-[{[3-(pyrrolidin-1-ylmethyl)phenyl]amino}(3-thienyl)methylene]indoline-5-carboxylic acid and (1 R)-1-phenylpropan-1-amine according to **Step E.**
C34H34N4O2S calc.: 562.74
MS (ESI) [M+H]⁺: 563 MS (ESI) [M+H]⁻ : 561

### (3Z)-2-oxo-N-[(1R)-1-phenylethyl]-3-[{[3-(piperidin-1-ylmethyl)phenyl]amino}(3-thienyl)methylene]indoline-5-carboxamide

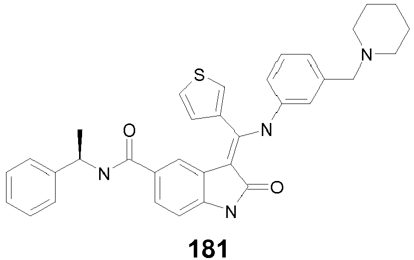

Compound **181** was obtained by reaction of (3*Z*)-2-oxo-3-[{[3-(piperidin-1-ylmethyl)phenyl]amino}(3-thienyl)methylene]indoline-5-carboxylic acid and (1 R)-1-phenylethanamine according to **Step E.**
C34H34N4O2S calc.: 562.74
MS (ESI) [M+H]⁺: 563 MS (ESI) [M+H]⁻ : 561

### (3Z)-2-oxo-N-[(1R)-1-phenylpropyl]-3-[{[3-(piperidin-1-ylmethyl)phenyl]amino}(3-thienyl)methylene]indoline-5-carboxamide

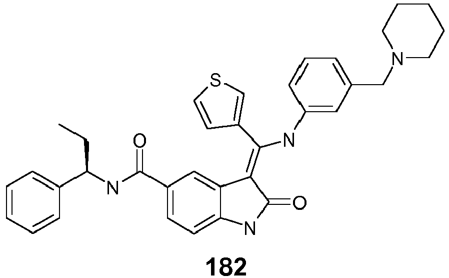

Compound **182** was obtained by reaction of (3*Z*)-2-oxo-3-[{[3-(piperidin-1-ylmethyl)phenyl]amino}(3-thienyl)methylene]indoline-5-carboxylic acid and (1 R)-1-phenylpropan-1-amine according to **Step E.**
C35H36N4O2S calc.: 576.77
MS (ESI) [M+H]⁺: 577 MS (ESI) [M+H]⁻ : 575

### (3Z)-3-[({3-[(4-methylpiperazin-1-yl)methyl]phenyl}amino)(3-thienyl)methylene]-2-oxo-N-[(1R)-1-phenylethyl]indoline-5-carboxamide

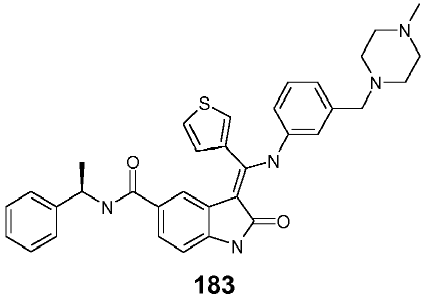

Compound **183** was obtained by reaction of (3*Z*)-3-[({3-[(4-methylpiperazin-1-yl)methyl]phenyl}amino)(3-thienyl)methylene]-2-oxoindoline-5-carboxylic acid and (1 R)-1-phenylethanamine according to **Step E.**
C34H35N5O2S calc.: 577.75
MS (ESI) [M+H]⁺: 578 MS (ESI) [M+H]⁻ : 576

### (3Z)-3-[({3-[(4-methylpiperazin-1-yl)methyl]phenyl}amino)(3-thienyl)methylene]-2-oxo-N-[(1R)-1-phenylpropyl]indoline-5-carboxamide

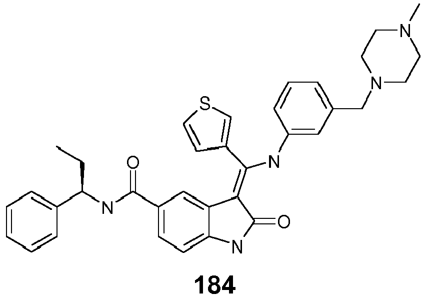

Compound **184** was obtained by reaction of (3*Z*)-3-[({3-[(4-methylpiperazin-1-yl)methyl]phenyl}amino)(3-thienyl)methylene]-2-oxoindoline-5-carboxylic acid and (1R)-1-phenylpropan-1-amine according to **Step E.**
C35H37N5O2S calc.: 591.78
MS (ESI) [M+H]⁺: 592 MS (ESI) [M+H]⁻ : 590

### (3Z)-3-[{[3-(morpholin-4-ylmethyl)phenyl]amino}(3-thienyl)methylene]-2-oxo-N-[(1R)-1-phenylethyl]indoline-5-carboxamide

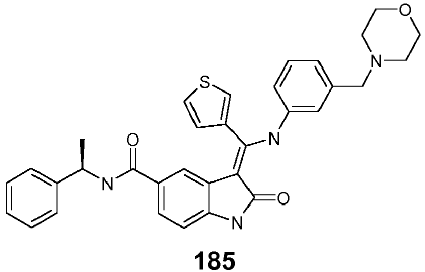

Compound **185** was obtained by reaction of (3*Z*)-3-[{[3-(morpholin-4-ylmethyl)phenyl]amino}(3-thienyl)methylene]-2-oxoindoline-5-carboxylic acid and (1 R)-1-phenylethanamine according to **Step E.**
C33H32N4O3S calc.: 564.71
MS (ESI) [M+H]⁺: 565 MS (ESI) [M+H]⁻ : 563

### (3Z)-3-[{[3-(morpholin-4-ylmethyl)phenyl]amino}(3-thienyl)methylene]-2-oxo-N-[(1 R)-1-phenylpropyl]indoline-5-carboxamide

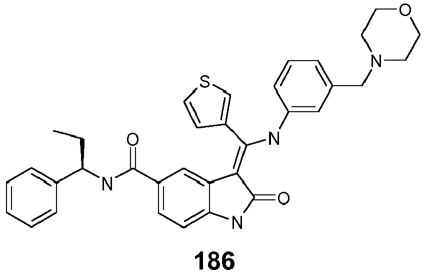

Compound **186** was obtained by reaction of (3*Z*)-3-[{[3-(morpholin-4-ylmethyl)phenyl]amino}(3-thienyl)methylene]-2-oxoindoline-5-carboxylic acid and (1R)-1-phenylpropan-1-amine according to **Step E.**
C34H34N4O3S calc.: 578.74
MS (ESI) [M+H]⁺: 579 MS (ESI) [M+H]⁻ : 577

### (3Z)-3-(3-furyl{[4-(pyrrolidin-1-ylmethyl)phenyl]amino}methylene)-2-oxo-N-[(1R)-1-phenylethyl]indoline-5-carboxamide

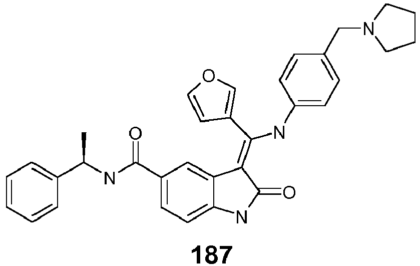

Compound **187** was obtained by reaction of (3*Z*)-3-(3-furyl{[4-(pyrrolidin-1-ylmethyl)phenyl]amino}methylene)-2-oxoindoline-5-carboxylic acid and (1 R)-1-phenylethanamine according to **Step E.**
C34H34N4O3 calc.: 532.65
MS (ESI) [M+H]+: 533 MS (ESI) [M+H]⁻: 531

### (3Z)-3-(3-furyl{[4-(piperidin-1-ylmethyl)phenyl]amino}methylene)-2-oxo-N-[(1R)-1-phenylpropyl]indoline-5-carboxamide

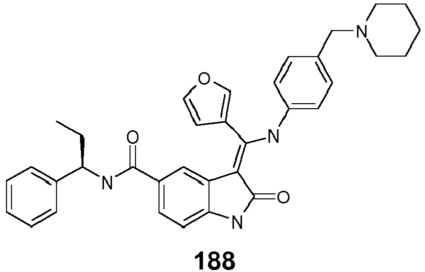

Compound **188** was obtained by reaction of (3*Z*)-3-(3-furyl{[4-(piperidin-1-ylmethyl)phenyl]amino}methylene)-2-oxoindoline-5-carboxylic acid and (1 R)-1-phenylpropan-1-amine according to **Step E.**
C35H36N4O3 calc.: 560.70
MS (ESI) [M+H]+: 561 MS (ESI) [M+H]⁻ : 559

### (3Z)-3-(2-furyl{[4-(piperidin-1-ylmethyl)phenyl]amino}methylene)-2-oxo-N-[(1R)-1-phenylpropyl]indoline-5-carboxamide

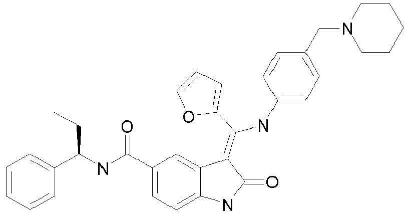

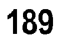

Compound **189** was obtained by reaction of (3*Z*)-3-(2-furyl{[4-(piperidin-1-ylmethyl)phenyl]amino}methylene)-2-oxoindoline-5-carboxylic acid and according to (1R)-1-phenylpropan-1-amine preparation of according to, **Step E.**
C35H36N4O3 calc.: 560.70
MS (ESI) [M+H]+: 561 MS (ESI) [M+H]⁻ : 559

### (3Z)-3-[(1-methyl-1H-pyrazol-4-yl){[4-(pyrrolidin-1-ylmethyl)phenyl]amino}methylene]-2-oxo-N-[(1R)-1-phenylethyl]indoline-5-carboxamide

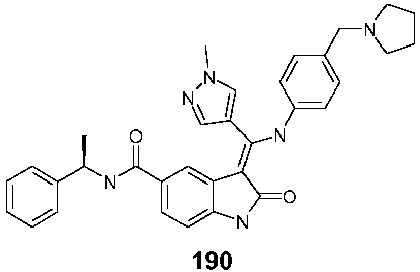

Compound **190** was obtained by reaction of and according to preparation of (3Z)-3-[(1-methyl-1*H*-pyrazol-4-yl){[4-(pyrrolidin-1-ylmethyl)phenyl]aminolmethylene]-2-oxoindoline-5-carboxylic acid and (1R)-1-phenylethanamine, according to **Step E.**
C33H34N6O2 calc.: 546.68
MS (ESI) [M+H]+: 547 MS (ESI) [M+H]⁻ : 545

### (3Z)-3-[(1-methyl-1H-pyrazol-4-yl){[4-(pyrrolidin-1-ylmethyl)phenyl]amino}methylene]-2-oxo-N-[(1R)-1-phenylpropyl]indoline-5-carboxamide

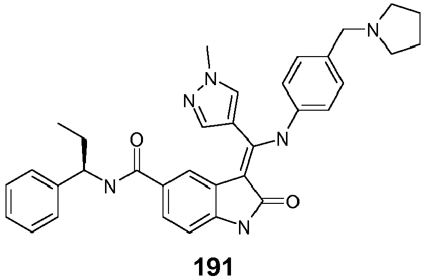

Compound **191** was obtained by reaction of (3*Z*)-3-[(1-methyl-1*H*-pyrazol-4-yl){[4-(pyrrolidin-1-ylmethyl)phenyl]amino}methylene]-2-oxoindoline-5-carboxylic acid and (1R)-1-phenylpropan-1-amine according to **Step E.**
C34H36N6O2 calc.: 560.71
MS (ESI) [M+H]+: 560 MS (ESI) [M+H]⁻ : 558

### (3Z)-3-[(1-methyl-1H-pyrazol-4-yl){[4-(piperidin-1-ylmethyl)phenyl]amino}methylene]-2-oxo-N-[(1R)-1-phenylethyl]indoline-5-carboxamide

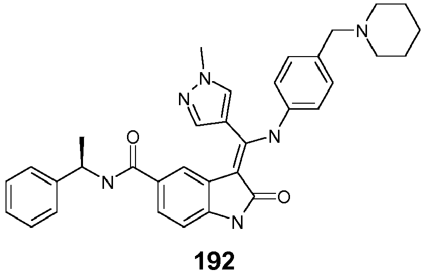

Compound **192** was obtained by reaction of (3*Z*)-3-[(1-methyl-1*H*-pyrazol-4-yl){[4-(piperidin-1-ylmethyl)phenyl]amino}methylene]-2-oxoindoline-5-carboxylic acid and (1 R)-1-phenylethanamine according to **Step E.**
C34H36N6O2 calc.: 560.71
MS (ESI) [M+H]+: 561 MS (ESI) [M+H]⁻ : 559

### (3Z)-3-[(1-methyl-1H-pyrazol-4-yl){[4-(piperidin-1-ylmethyl)phenyl]amino}methylene]-2-oxo-N-[(1R)-1-phenylpropyl]indoline-5-carboxamide

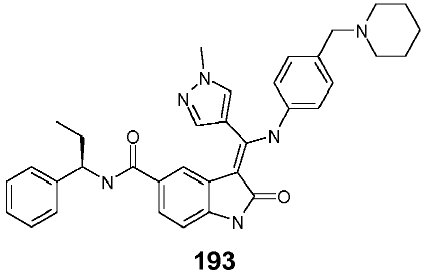

Compound **193** was obtained by reaction of (3*Z*)-3-[(1-methyl-1*H*-pyrazol-4-yl){[4-(piperidin-1-ylmethyl)phenyl]amino}methylene]-2-oxoindoline-5-carboxylic acid and (1R)-1-phenylpropan-1-amine according to **Step E.**
C35H38N6O2 calc.: 574.73
MS (ESI) [M+H]+: 575 MS (ESI) [M+H]⁻ : 573

### (3Z)-3-[(1-methyl-1H-pyrazol-4-yl){[3-(pyrrolidin-1-ylmethyl)phenyl]amino}methylene]-2-oxo-N-[(1R)-1-phenylethyl]indoline-5-carboxamide

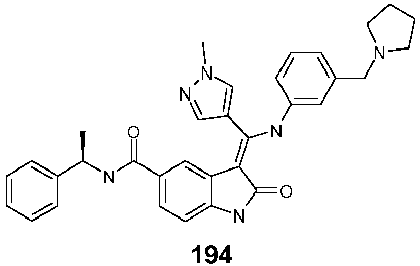

Compound **194** was obtained by reaction of (3*Z*)-3-[(1-methyl-1*H*-pyrazol-4-yl){[3-(pyrrolidin-1-ylmethyl)phenyl]amino}methylene]-2-oxoindoline-5-carboxylic acid and (1 R)-1-phenylethanamine according to **Step E.**
C33H34N6O2 calc.: 546.68
MS (ESI) [M+H]+: 547 MS (ESI) [M+H]⁻ : 545

### (3Z)-3-[(1-methyl-1H-pyrazol-4-yl){[3-(pyrrolidin-1-ylmethyl)phenyl]amino}methylene]-2-oxo-N-[(1R)-1-phenylpropyl]indoline-5-carboxamide

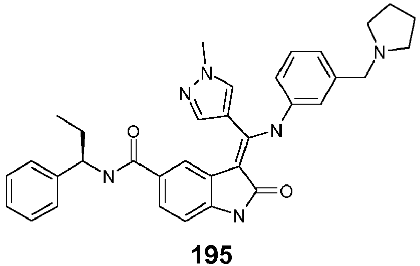

Compound **195** was obtained by reaction of (3*Z*)-3-[(1-methyl-1*H*-pyrazol-4-yl){[3-(pyrrolidin-1-ylmethyl)phenyl]amino}methylene]-2-oxoindoline-5-carboxylic acid and (1R)-1-phenylpropan-1-amine according to **Step E.**
C34H36N6O2 calc.: 560.71
MS (ESI) [M+H]+: 561 MS (ESI) [M+H]⁻ : 559

### (3Z)-3-[(1-methyl-1H-pyrazol-4-yl){[3-(piperidin-1-ylmethyl)phenyl]amino}methylene]-2-oxo-N-[(1R)-1-phenylethyl]indoline-5-carboxamide

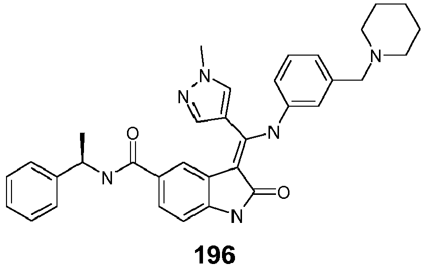

Compound **196** was obtained by reaction of (3*Z*)-3-[(1-methyl-1*H*-pyrazol-4-yl){[3-(piperidin-1-ylmethyl)phenyl]amino}methylene]-2-oxoindoline-5-carboxylic acid (1 R)-1-phenylethanamine and according to **Step E.**
C34H36N6O2 calc.: 560.71
MS (ESI) [M+H]+: 561 MS (ESI) [M+H]⁻ : 559

### (3Z)-3-[(1-methyl-1H-pyrazol-4-yl){[3-(piperidin-1-ylmethyl)phenyl]amino}methylene]-2-oxo-N-[(1R)-1-phenylpropyl]indoline-5-carboxamide

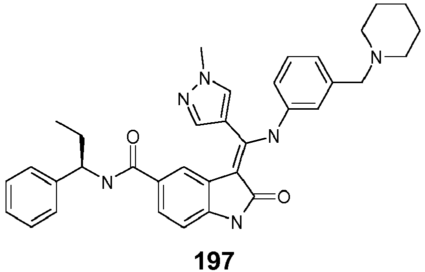

Compound **197** was obtained by reaction of (3*Z*)-3-[(1-methyl-1*H*-pyrazol-4-yl){[3-(piperidin-1-ylmethyl)phenyl]amino}methylene]-2-oxoindoline-5-carboxylic acid and (1R)-1-phenylpropan-1-amine according to **Step E.**
C35H38N6O2 calc.: 574.73
MS (ESI) [M+H]+: 575 MS (ESI) [M+H]⁻ : 573

### (3Z)-3-[(1-methyl-1H-indol-5-yl){[4-(pyrrolidin-1-ylmethyl)phenyl]amino}methylene]-2-oxo-N-[(1R)-1-phenylethyl]indoline-5-carboxamide

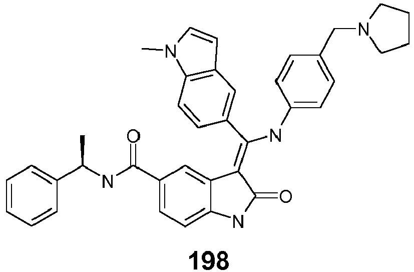

Compound **198** was obtained by reaction of (3*Z*)-3-[(1-methyl-1*H*-indol-5-yl){[4-(pyrrolidin-1-ylmethyl)phenyl]amino}methylene]-2-oxoindoline-5-carboxylic acid and (1 R)-1-phenylethanamine according to **Step E.**
C38H37N5O2 calc.: 595.75
MS (ESI) [M+H]+: 596 MS (ESI) [M+H]⁻ : 594

### (3Z)-3-[(1-methyl-1H-indol-5-yl){[4-(pyrrolidin-1-ylmethyl)phenyl]amino}methylene]-2-oxo-N-[(1R)-1-phenylpropyl]indoline-5-carboxamide

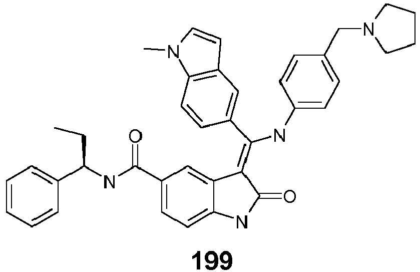

Compound **199** was obtained by reaction of (3*Z*)-3-[(1-methyl-1*H*-indol-5-yl){[4-(pyrrolidin-1-ylmethyl)phenyl]amino}methylene]-2-oxoindoline-5-carboxylic acid and (1R)-1-phenylpropan-1-amine according to **Step E.**
C39H39N5O2 calc.: 609.78
MS (ESI) [M+H]+: 610 MS (ESI) [M+H]⁻ : 608

### (3Z)-3-[(1-methyl-1H-indol-5-yl){[4-(piperidin-1-ylmethyl)phenyl]aminolmethylene]-2-oxo-N-[(1R)-1-phenylethyl]indoline-5-carboxamide

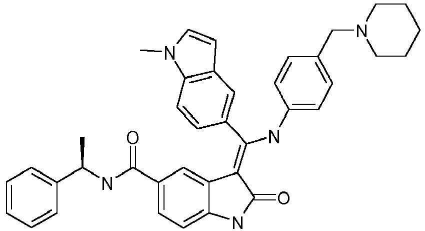

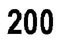

Compound **200** was obtained by reaction of (3*Z*)-3-[(1-methyl-1*H*-indol-5-yl){[4-(piperidin-1-ylmethyl)phenyl]amino}methylene]-2-oxoindoline-5-carboxylic acid and (1 R)-1-phenylethanamine according to **Step E.**
C39H39N5O2 calc.: 609.78
MS (ESI) [M+H]+: 610 MS (ESI) [M+H]⁻ : 608

### (3Z)-3-[(1-methyl-1H-indol-5-yl){[4-(piperidin-1-ylmethyl)phenyl]amino}methylene]-2-oxo-N-[(1R)-1-phenylpropyl]indoline-5-carboxamide

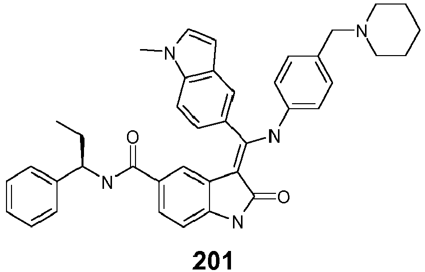

Compound **201** was obtained by reaction of (3*Z*)-3-[(1-methyl-1*H*-indol-5-yl){[4-(piperidin-1-ylmethyl)phenyl]amino}methylene]-2-oxoindoline-5-carboxylic acid and (1R)-1-phenylpropan-1-amine according to **Step E.**
C40H41N5O2 calc.: 623.81
MS (ESI) [M+H]+: 624 MS (ESI) [M+H]⁻ : 622

### (3Z)-3-[(1-methyl-1H-indol-5-yl){[3-(pyrrolidin-1-ylmethyl)phenyl]amino}methylene]-2-oxo-N-[(1R)-1-phenylethyl]indoline-5-carboxamide

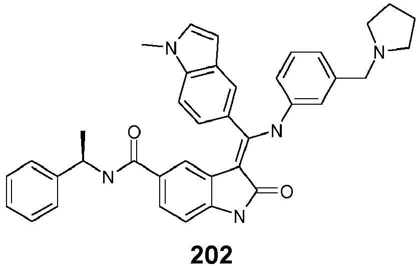

Compound **202** was obtained by reaction of (3*Z*)-3-[(1-methyl-1*H*-indol-5-yl){[3-(pyrrolidin-1-ylmethyl)phenyl]amino}methylene]-2-oxoindoline-5-carboxylic acid and (1 R)-1-phenylethanamine according to **Step E.**
C38H37N5O2 calc.: 595.75
MS (ESI) [M+H]+: 596 [M+H]⁻ : 594

### (3Z)-3-[(1-methyl-1H-indol-5-yl){[3-(pyrrolidin-1-ylmethyl)phenyl]amino}methylene]-2-oxo-N-[(1R)-1-phenylpropyl]indoline-5-carboxamide

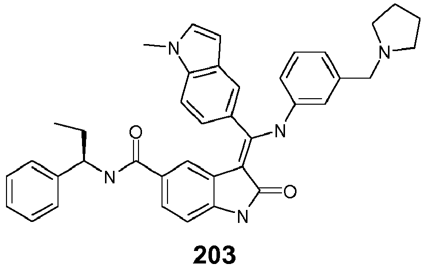

Compound **203** was obtained by reaction of (3*Z*)-3-[(1-methyl-1*H*-indol-5-yl){[3-(pyrrolidin-1-ylmethyl)phenyl]amino}methylene]-2-oxoindoline-5-carboxylic acid and (1R)-1-phenylpropan-1-amine according to **Step E.**
C39H39N5O2 calc.: 609.78
MS (ESI) [M+H]+: 610 [M+H]⁻ : 608

### (3Z)-3-[(1-methyl-1H-indol-5-yl){[3-(piperidin-1-ylmethyl)phenyl]aminolmethylene]-2-oxo-N-[(1R)-1-phenylethyl]indoline-5-carboxamide

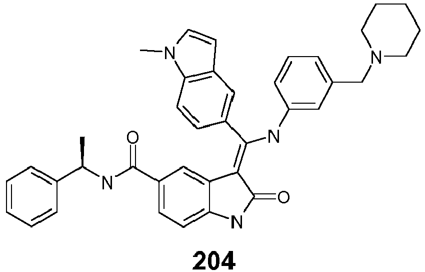

Compound **204** was obtained by reaction of (3*Z*)-3-[(1-methyl-1*H*-indol-5-yl){[3-(piperidin-1-ylmethyl)phenyl]amino}methylene]-2-oxoindoline-5-carboxylic acid and (1 R)-1-phenylethanamine according to **Step E.**
C39H39N5O2 calc.: 609.78
MS (ESI) [M+H]+: 610 [M+H]⁻ : 608

### (3Z)-3-[(1-methyl-1H-indol-5-yl){[3-(piperidin-1-ylmethyl)phenyl]amino}methylene]-2-oxo-N-[(1R)-1-phenylpropyl]indoline-5-carboxamide

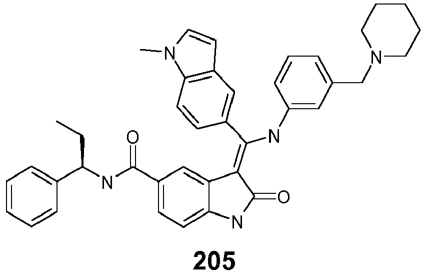

Compound 205 was obtained by reaction of (3*Z*)-3-[(1-methyl-1*H*-indol-5-yl){[3-(piperidin-1-ylmethyl)phenyl]amino}methylene]-2-oxoindoline-5-carboxylic acid (1R)-1-phenylpropan-1-amine according to **Step E.**
C40H41N5O2 calc.: 623.81
MS (ESI) [M+H]+: 624 [M+H]- : 622

### (3Z)-3-(cyclopropyl{[4-(pyrrolidin-1-ylmethyl)phenyl]amino}methylene)-2-oxo-N-[(1R)-1-phenylethyl]indoline-5-carboxamide

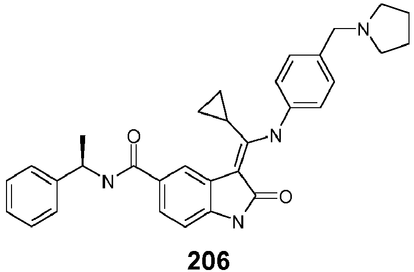

Compound **206** was obtained by reaction of (3*Z*)-3-(cyclopropyl{[4-(pyrrolidin-1-ylmethyl)phenyl]amino}methylene)-2-oxoindoline-5-carboxylic acid and (1 R)-1-phenylethanamine according to **Step E.**
C32H34N4O2 calc.: 506.65
MS (ESI) [M+H]+: 507 [M+H]⁻ : 505

### (3Z)-3-(cyclopropyl{[4-(pyrrolidin-1-ylmethyl)phenyl]amino}methylene)-2-oxo-N-[(1 R)-1-phenylpropyl]indoline-5-carboxamide

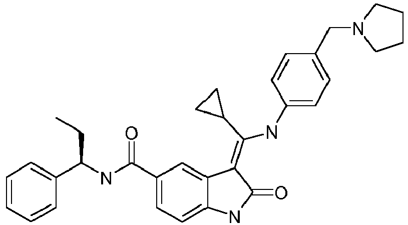

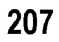

Compound **207** was obtained by reaction of (3*Z*)-3-(cyclopropyl{[4-(pyrrolidin-1-ylmethyl)phenyl]amino}methylene)-2-oxoindoline-5-carboxylic acid and (1 R)-1-phenylpropan-1-amine according to **Step E.**
C33H36N4O2 calc.: 520.68
MS (ESI) [M+H]+: 521 [M+H]⁻ : 519

### (3Z)-3-(cyclopropyl{[4-(piperidin-1-ylmethyl)phenyl]amino}methylene)-2-oxo-N-[(1R)-1-phenylethyl]indoline-5-carboxamide

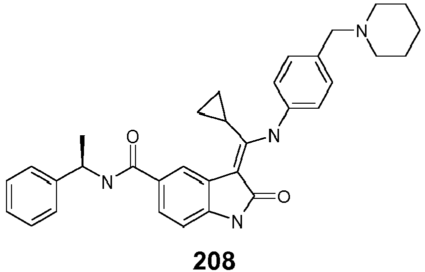

Compound **208** was obtained by reaction of (3*Z*)-3-(cyclopropyl{[4-(piperidin-1-ylmethyl)phenyl]amino}methylene)-2-oxoindoline-5-carboxylic acid and (1 R)-1-phenylethanamine according to **Step E.**
C33H36N4O2 calc.: 520.68
MS (ESI) [M+H]+: 521 [M+H]⁻ : 519

### (3Z)-3-(cyclopropyl{[4-(piperidin-1-ylmethyl)phenyl]amino}methylene)-2-oxo-N-[(1 R)-1-phenylpropyl]indoline-5-carboxamide

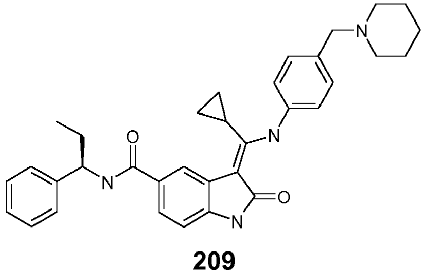

Compound **209** was obtained by reaction of (3*Z*)-3-(cyclopropyl{[4-(piperidin-1-ylmethyl)phenyl]amino}methylene)-2-oxoindoline-5-carboxylic acid and (1 R)-1-phenylpropan-1-amine according to **Step E.**
C34H38N4O2 calc.: 534.71
MS (ESI) [M+H]+: 535 [M+H]⁻ : 533

### (3Z)-3-(cyclohexyl{[4-(piperidin-1-ylmethyl)phenyl]amino}methylene)-2-oxo-N-[(1R)-1-phenylethyl]indoline-5-carboxamide

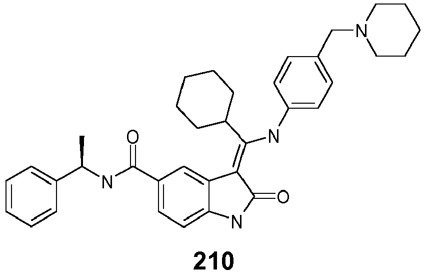

Compound **210** was obtained by reaction of (3*Z*)-3-(cyclohexyl{[4-(piperidin-1-ylmethyl)phenyl]amino}methylene)-2-oxoindoline-5-carboxylic acid and (1 R)-1-phenylethanamine according to **Step E.**
C36H42N4O2 calc.: 562.76
MS (ESI) [M+H]+: 563 [M+H]⁻ : 561

### (3Z)-3-(cyclohexyl{[4-(piperidin-1-ylmethyl)phenyl]amino}methylene)-2-oxo-N-[(1 R)-1-phenylpropyl]indoline-5-carboxamide

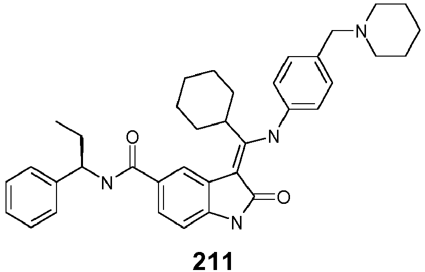

Compound **211** was obtained by reaction of (3*Z*)-3-(cyclohexyl{[4-(piperidin-1-ylmethyl)phenyl]amino}methylene)-2-oxoindoline-5-carboxylic acid and (1 R)-1-phenylpropan-1-amine according to **Step E.**
C37H44N4O2 calc.: 576.79
MS (ESI) [M+H]+: 577 [M+H]⁻ : 575

### (3Z)-2-oxo-N-[(1R)-1-phenylethyl]-3-(1-{[4-(piperidin-1-ylmethyl)phenyl]amino}propylidene) indoline-5-carboxamide

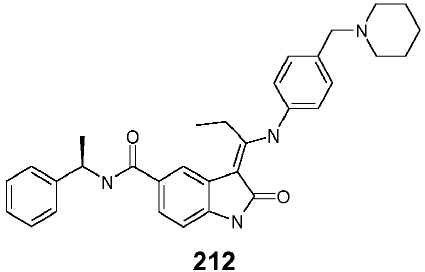

Compound **212** was obtained by reaction of (3*Z*)-2-oxo-3-(1-{[4-(piperidin-1-ylmethyl)phenyl]amino}propylidene)indoline-5-carboxylic acid and (1 R)-1-phenylethanamine according to **Step E.**
C32H36N4O2 calc. 508.67
MS (ESI) [M+H]+ 509 [M+H]⁻ :507

### (3Z)-2-oxo-N-[(1R)-1-phenylpropyl]-3-(1-{[4-(piperidin-1-ylmethyl)phenyl]amino}propylidene)indoline-5-carboxamide

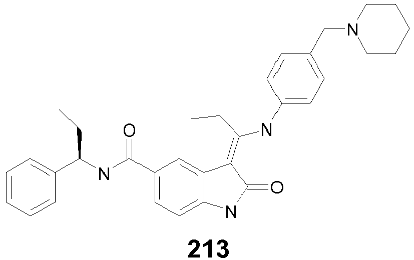

Compound **213** was obtained by reaction of (3*Z*)-2-oxo-3-(1-{[4-(piperidin-1-ylmethyl)phenyl]amino}propylidene)indoline-5-carboxylic acid and (1 R)-1-phenylpropan-1-amine according to **Step E.**
C33H38N4O2, calc: 522.70
MS (ESI) [M+H]+: 523 [M+H]⁻ : 521

### (3Z)-3-(2-methyl-1-{[4-(piperidin-1-ylmethyl)phenyl]amino}propylidene)-2-oxo-N-[(1R)-1-phenylethyl]indoline-5-carboxamide

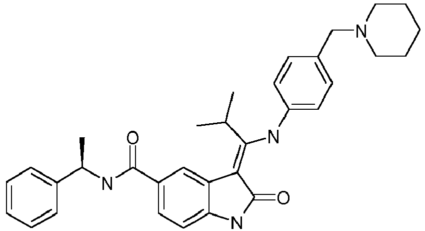

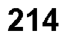

Compound **214** was obtained by reaction of (3*Z*)-3-(2-methyl-1-{[4-(piperidin-1-ylmethyl)phenyl]amino}propylidene)-2-oxoindoline-5-carboxylic acid and (1 R)-1-phenylethanamine according to **Step E.**
C33H38N4O2, calc. 522.70
MS (ESI) [M+H]⁺: 523 [M+H]⁻ : 521

### (3Z)-3-(2-methyl-1-{[4-(piperidin-1-ylmethyl)phenyl]amino}propylidene)-2-oxo-N-[(1 R)-1-phenylpropyl]indoline-5-carboxamide

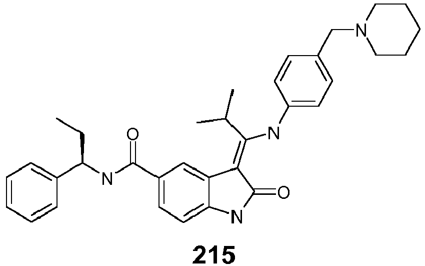

Compound **215** was obtained by reaction of (3*Z*)-3-(2-methyl-1-{[4-(piperidin-1-ylmethyl)phenyl]amino}propylidene)-2-oxoindoline-5-carboxylic acid and (1 R)-1-phenylpropan-1-amine according to **Step E.**
C34H40N4O2, calc. 536.72
MS (ESI) [M+H]+: 537 [M+H]⁻ : 535

### (3Z)-3-[(4-fluorophenyl){[3-(piperidin-1-ylmethyl)phenyl]amino}methylene]-2-oxo-N-[(1R)-1-phenylethyl]indoline-5-carboxamide

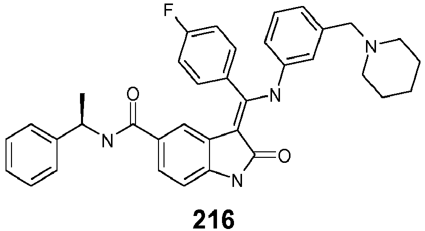

C₃₆H₃₅FN₄O₂, Mw calc. :574.3
MS(ESI): m/z (M+H)⁺575

### (3Z)-3-[(4-fluorophenyl){[3-(piperidin-1-ylmethyl)phenyl]amino}methylene]-2-oxo-N-[(1 R)-1-phenylpropyl]indoline-5-carboxamide

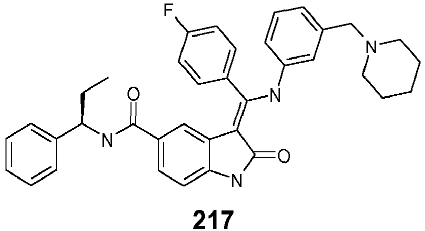

C₃₇H₃₇FN₄O₂, Mw cal. :588.3
MS(ESI): m/z (M+H)⁺589

### (3Z)-3-[(4-fluorophenyl){[3-(piperidin-1-ylmethyl)phenyl]amino}methylene]-N-[(1R)-1-(4-methoxyphenyl)ethyl]-2-oxoindoline-5-carboxamide

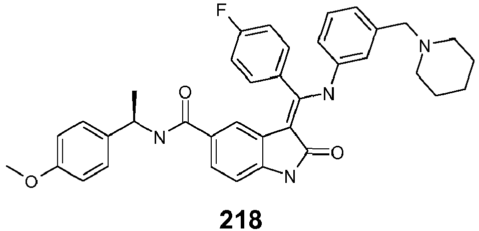

C₃₇H₃₇FN₄O₃, Mw calc. :604.3
MS(ESI): m/z (M+H)⁺605

### (3Z)-3-[(4-fluorophenyl){[3-(piperidin-1-ylmethyl)phenyl]amino}methylene]-N-[(1R)-1-(3-methoxyphenyl)ethyl]-2-oxoindoline-5-carboxamide

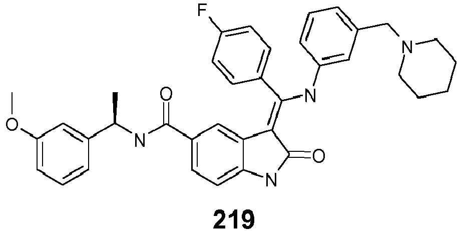

C₃₇H₃₇FN₄O₃, Mw calc.:604.3
MS(ESI): m/z (M+H)⁺605

### (3Z)-N-[(1R)-1-(3-chlorophenyl)ethyl]-3-[(4-fluorophenyl){[3-(piperidin-1-ylmethyl)phenyl]amino}methylene]-2-oxoindoline-5-carboxamide

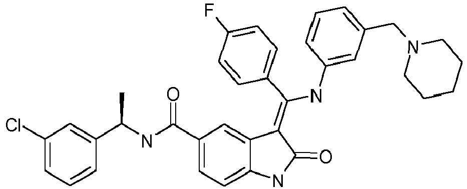

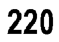

C₃₆H₃₄ClFN₄O₂, Mw calc. :608.2
MS(ESI): m/z (M+H)⁺609

### (3Z)-N-[(1R)-1-(4-chlorophenyl)ethyl]-3-[(4-fluorophenyl){[3-(piperidin-1-ylmethyl)phenyl]amino}methylene]-2-oxoindoline-5-carboxamide

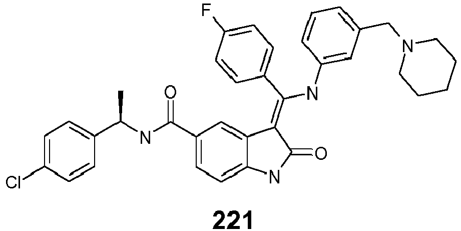

C₃₆H₃₄ClFN₄O₂, Mw calc. :608.2
MS(ESI): m/z (M+H)⁺609

### (3Z)-3-[(4-fluorophenyl){[3-(piperidin-1-ylmethyl)phenyl]amino}methylene]-N-[(1R)-1-(4-methylphenyl)ethyl]-2-oxoindoline-5-carboxamide

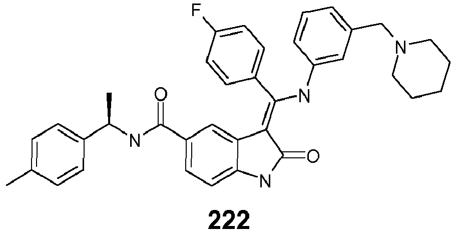

C₃₇H₃₇FN₄O₂, Mw calc. :588.3
MS(ESI): m/z (M+H)⁺589

### (3Z)-N-[1-(3,4-difluorophenyl)propyl]-3-[(4-fluorophenyl){[3-(piperidin-1-ylmethyl)phenyl]amino}methylene]-2-oxoindoline-5-carboxamide

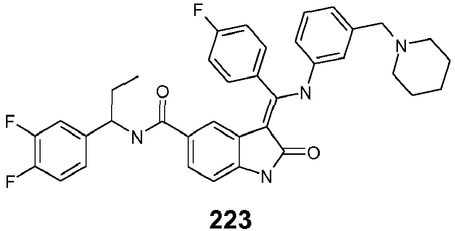

C₃₇H₃₇FN₄O₂, Mw calc. : 624.3
MS(ESI): m/z (M+H)⁺625

### (3Z)-N-[1-(3-chlorophenyl)propyl]-3-[(4-fluorophenyl){[3-(piperidin-1-ylmethyl)phenyl]amino}methylene]-2-oxoindoline-5-carboxamide

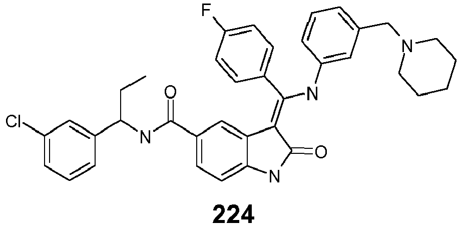

C₃₇H₃₆ClFN₄O₂, Mw calc. :622.2
MS(ESI): m/z (M+H)⁺623

### (3Z)-3-[({2-[(ethylcarbamoyl)amino]phenyl}amino)(phenyl)methylene]-2-oxo-N-[(1R)-1-phenylethyl]indoline-5-carboxamide

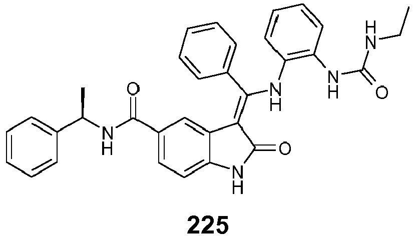

MS (ESI) [M+H]⁺: 547

### (3Z)-N-benzyl-3-{[(2-{[(ethylamino)carbonothioyl]amino}phenyl)amino] (phenyl)methylene}-2-oxoindoline-5-carboxamide

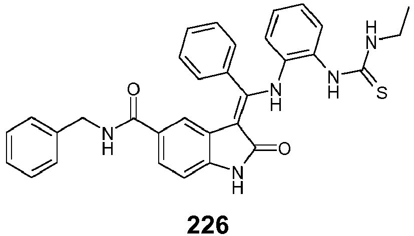

MS (ESI) [M+H]⁺: 549

### (3Z)-3-{[(2-{[(3-chlorophenyl)carbamoyl]amino}phenyl)amino](phenyl)methylene}-2-oxo-N-[(1R)-1-phenylethyl]indoline-5-carboxamide

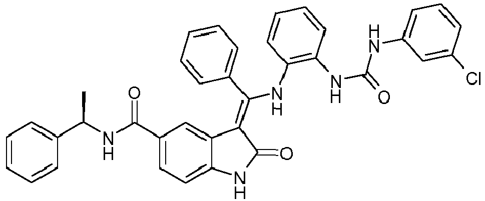

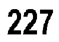

MS (ESI) [M+H]⁺: 629

### (3Z)-3-{[(2-{[(4-hydroxyphenyl)carbamoyl]amino}phenyl)amino](phenyl) methylene}-2-oxo-N-[(1R)-1-phenylpropyl]indoline-5-carboxamide

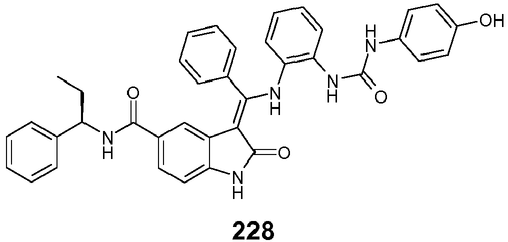

MS (ESI) [M+H]⁺: 625

### (3Z)-3-{[(2-{[(4-hydroxyphenyl)carbamoyl]amino}phenyl)amino](phenyl)methylene}-2-oxo-N-[(1S)-1-phenylpropyl]indoline-5-carboxamide

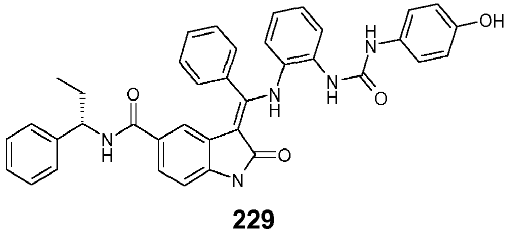

MS (ESI) [M+H]⁺: 625

### (3Z)-3-[{[2-(2,5-dioxoimidazolidin-1-yl)phenyl]amino}(phenyl)methylene]-2-oxo-N-[(1R)-1-phenylethyl]indoline-5-carboxamide

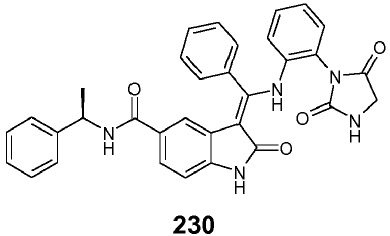

MS (ESI) [M+H]⁺: 559

### (3Z)-3-[({2-[(4Z)-2,5-dioxo-4-(pyridin-4-ylmethylene)imidazolidin-1-yl]phenyl}amino)(phenyl)methylene]-2-oxo-N-[(1R)-1-phenylethyl]indoline-5-carboxamide

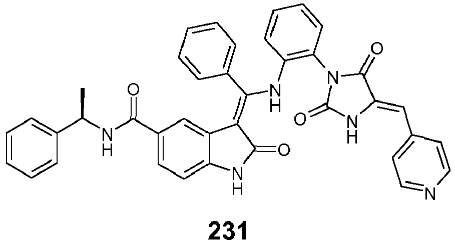

MS (ESI) [M+H]⁺: 648

### (3Z)-3-[({3-[(ethylcarbamoyl)amino]phenyl}amino)(phenyl)methylene]-2-oxo-N-[(1 R)-1-phenylpropyl]indoline-5-carboxamide

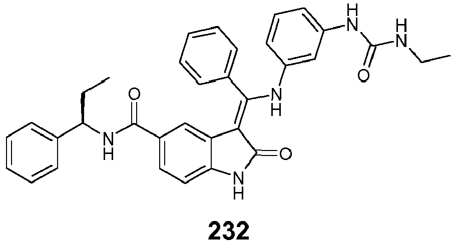

MS (ESI) [M+H]⁺: 561

### (3Z)-3-{[(3-{[(3-chlorophenyl)carbamoyl]amino}phenyl)amino](phenyl)methylene}-2-oxo-N-[(1R)-1-phenylpropyl]indoline-5-carboxamide

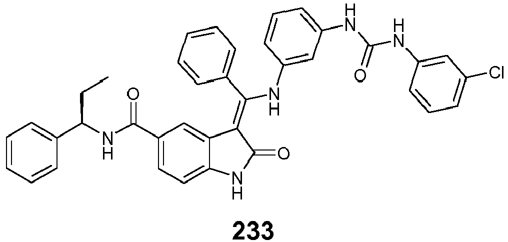

MS (ESI) [M+H]⁺: 643

### (3Z)-3-{[(3-{[(ethylamino)carbonothioyl]amino}phenyl)amino](phenyl)methylene}-2-oxo-N-[(1S)-1-phenylethyl]indoline-5-carboxamide

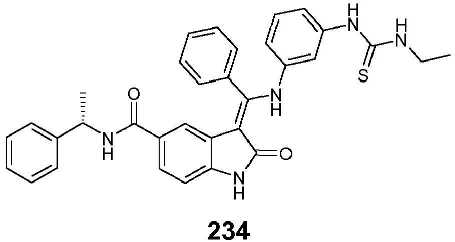

MS (ESI) [M+H]⁺: 563

### (3Z)-3-{[(3-{[(4-hydroxyphenyl)carbamoyl]amino}phenyl)amino](phenyl)methylene}-2-oxo-N-[(1R)-1-phenylpropyl]indoline-5-carboxamide

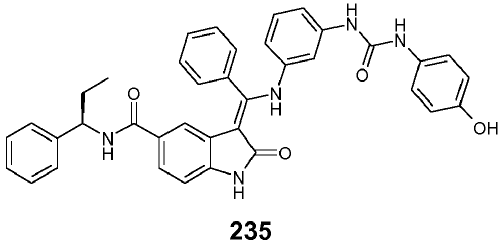

MS (ESI) [M+H]⁺: 625

### (3Z)-3-[{[3-({[4-(2,5-dioxoimidazolidin-1-yl)phenyl]carbamoyl}amino)phenyl]amino} (phenyl)methylene]-2-oxo-N-[(1S)-1-phenylethyl]indoline-5-carboxamide

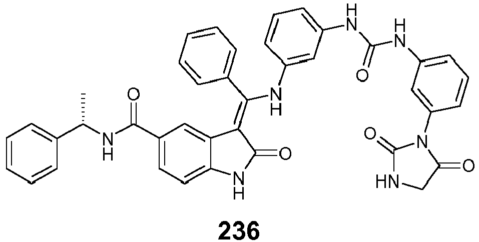

MS (ESI) [M+H]⁺: 693

### (3Z)-3-[({3-[({4-[(4Z)-2,5-dioxo-4-(pyridin-3-ylmethylene)imidazolidin-1-yl]phenyl}carbamoyl)amino]phenyl}amino)(phenyl)methylene]-2-oxo-N-[(1S)-1-phenylethyl]indoline-5-carboxamide

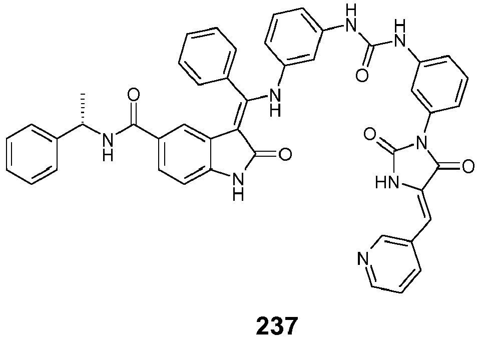

MS (ESI) [M+H]⁺: 782

### (3Z)-3-{[(4-{[(ethylamino)carbonothioyl]amino}phenyl)amino](phenyl)methylene}-2-oxo-N-[(1S)-1-phenylpropyl]indoline-5-carboxamide

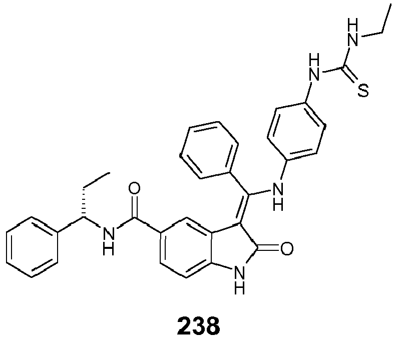

MS (ESI) [M+H]⁺: 577

### (3Z)-3-{[(4-{[(3-chlorophenyl)carbamoyl]amino}phenyl)amino](phenyl)methylene}-2-oxo-N-[(1R)-1-phenylpropyl]indoline-5-carboxamide

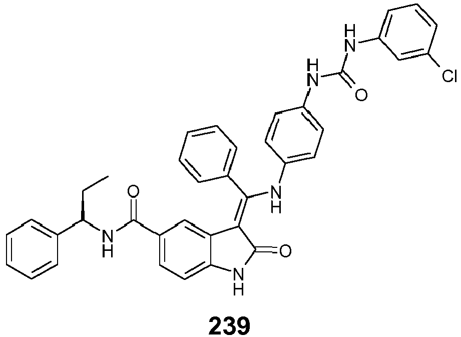

MS (ESI) [M+H]⁺: 643

### (3Z)-3-{[(4-{[(4-hydroxyphenyl)carbamoyl]amino}phenyl)amino](phenyl) methylene}-2-oxo-N-[(1R)-1-phenylpropyl]indoline-5-carboxamide

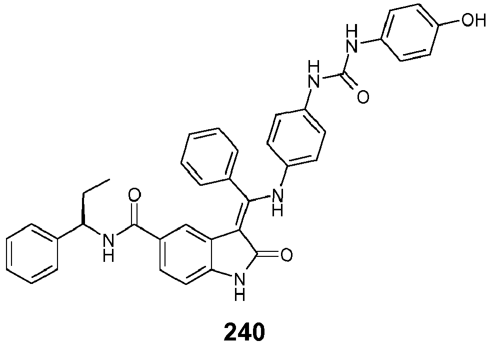

MS (ESI) [M+H]⁺: 625

### (3Z)-3-[{[4-(2,5-dioxoimidazolidin-1-yl)phenyl]amino}(pyridin-4-yl)methylene]-2-oxo-N-[(1R)-1-phenylethyl]indoline-5-carboxamide

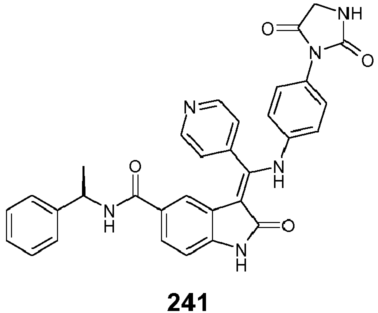

MS (ESI) [M+H]⁺: 560

### (3Z)-3-[({4-[(4Z)-4-benzylidene-2,5-dioxoimidazolidin-1-yl]phenyl}amino)(pyridin-4-yl)methylene]-2-oxo-N-[(1R)-1-phenylethyl]indoline-5-carboxamide

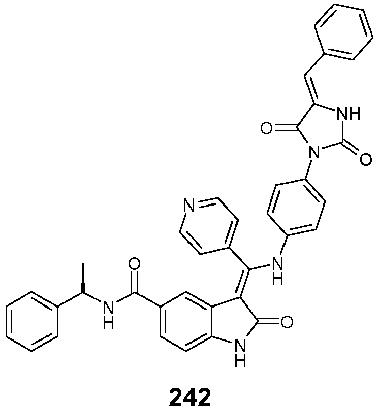

MS (ESI) [M+H]⁺: 648

### (3Z)-3-{[(2-{[(ethylamino)carbonothioyl]amino}benzyl)amino](3-thienyl)methylene}-2-oxo-N-[(1R)-1-phenylethyl]indoline-5-carboxamide

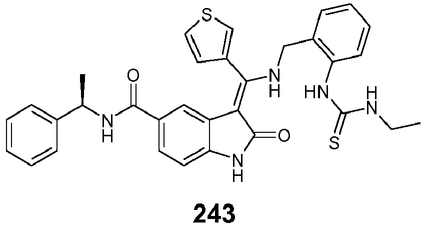

MS (ESI) [M+H]⁺: 583

### (3Z)-3-[({2-[(1,3-benzodioxol-5-ylcarbamoyl)amino]benzyl}amino)(phenyl)methylene]-2-oxo-N-[(1R)-1-phenyl propyl]indoline-5-carboxamide

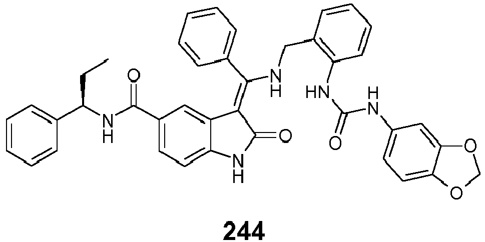

MS (ESI) [M+H]⁺: 667

### (3Z)-3-[({3-[(ethylcarbamoyl)amino]benzyl}amino)(pyridin-3-yl)methylene]-2-oxo-N-[(1R)-1-phenylethyl]indoline-5-carboxamide

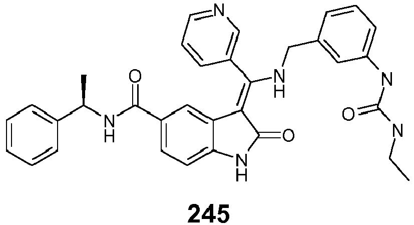

MS (ESI) [M+H]⁺: 562

### (3Z)-3-{[(3-{[(3-chlorophenyl)carbamoyl]amino}benzyl)amino](pyridin-3-yl)methylene}-2-oxo-N-[(1R)-1-phenylethyl]indoline-5-carboxamide

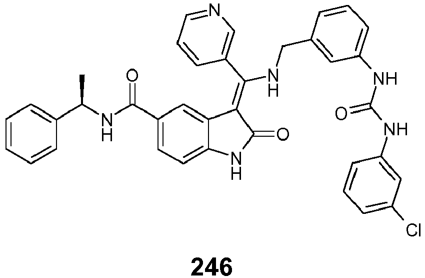

MS (ESI) [M+H]⁺: 644

### (3Z)-3-[{[3-(2,5-dioxoimidazolidin-1-yl)benzyl]amino}(pyridin-3-yl)methylene]-2-oxo-N-[(1R)-1-phenylethyl]indoline-5-carboxamide

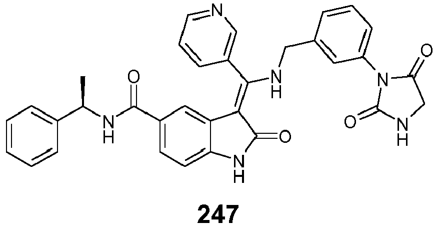

MS (ESI) [M+H]⁺: 574

### (3Z)-3-[({3-[(4Z)-2,5-dioxo-4-(pyridin-4-ylmethylene)imidazolidin-1-yl]benzyl}amino)(pyridin-3-yl)methylene]-2-oxo-N-[(1R)-1-phenylethyl]indoline-5-carboxamide

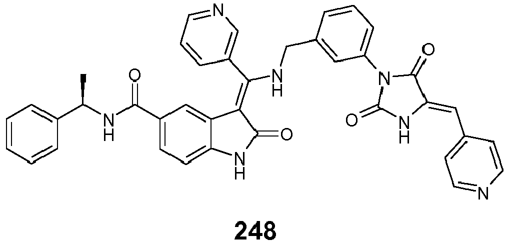

MS (ESI) [M+H]⁺: 663

### (3Z)-N-benzyl-3-[{[4-({[(3-chlorophenyl)amino]carbonothioyl}amino)benzyl]amino} (phenyl)methylene]-2-oxoindoline-5-carboxamide

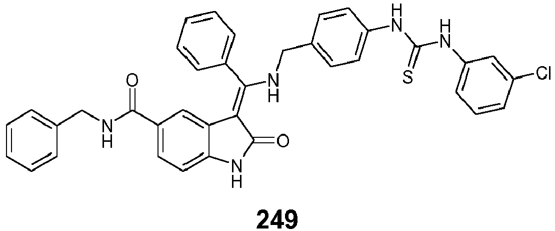

MS (ESI) [M+H]⁺: 645

### (3Z)-N-benzyl-3-[{[4-(2,5-dioxoimidazolidin-1-yl)benzyl]amino}(phenyl)methylene]-2-oxoindoline-5-carboxamide

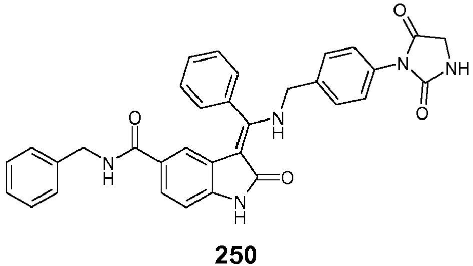

MS (ESI) [M+H]⁺: 559

### (3Z)-3-[{[4-(2,5-dioxoimidazolidin-1-yl)benzyl]amino}(phenyl)methylene]-2-oxo-N-[(1R)-1-phenylethyl]indoline-6-carboxamide

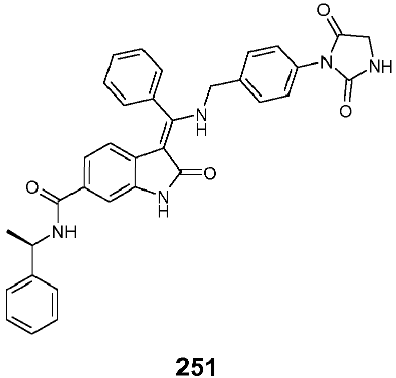

MS (ESI) [M+H]⁺: 573

### (3Z)-3-[({4-[(4Z)-2,5-dioxo-4-(pyridin-4-ylmethylene)imidazolidin-1-yl]benzyl}amino)(phenyl)methylene]-2-oxo-N-[(1R)-1-phenylethyl]indoline-6-carboxamide

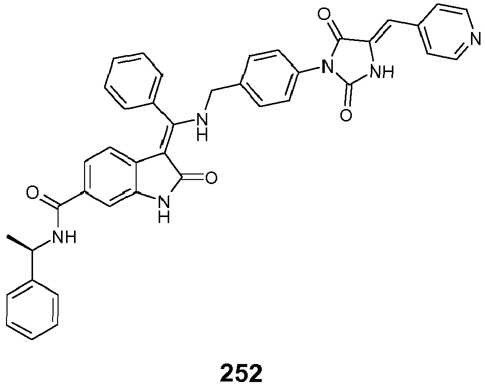

MS (ESI) [M+H]⁺: 662

### (3Z)-N-benzyl-3-[({4-[(4Z)-2,5-dioxo-4-(pyridin-4-ylmethylene)imidazolidin-1-yl]benzyl}amino)(phenyl)methylene]-2-oxoindoline-5-carboxamide

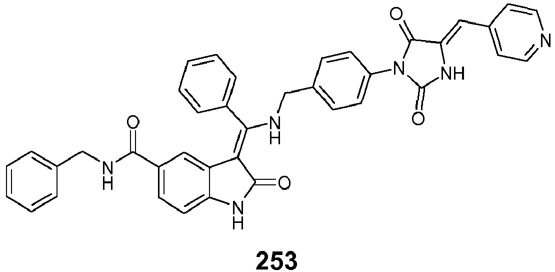

MS (ESI) [M+H]⁺: 648

### (3Z)-3-{[(2-{2-[(ethylcarbamoyl)amino]phenyl}ethyl)amino](phenyl)methylene}-2-oxo-N-[(1R)-1-phenylpropyl]indoline-7-carboxamide

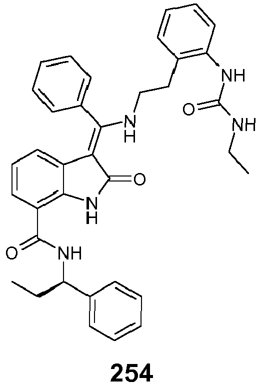

MS (ESI) [M+H]⁺: 589

### (3Z)-3-[{[2-(2-{[(3-chlorophenyl)carbamoyl]amino}phenyl)ethyl]amino}(pyridin-4-yl)methylene]-2-oxo-N-[(1S)-1-phenylpropyl]indoline-7-carboxamide

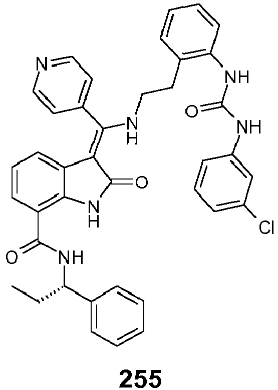

MS (ESI) [M+H]⁺: 672

### (3Z)-3-{[(2-{3-[(ethylcarbamoyl)amino]phenyl}ethyl)amino](phenyl)methylene}-2-oxo-N-[(1R)-1-phenylethyl]indoline-5-carboxamide

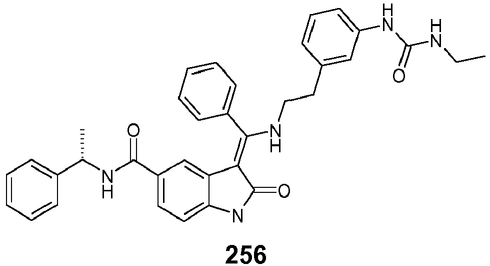

MS (ESI) [M+H]⁺: 575

### (3Z)-3-{[(2-{3-[(ethylcarbamoyl)amino]phenyl}ethyl)amino](phenyl)methylene}-2-oxo-N-[(1S)-1-phenylethyl]indoline-5-carboxamide

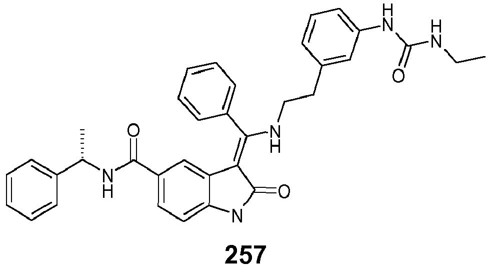

MS (ESI) [M+H]⁺: 575

### (3Z)-3-[{[2-(3-{[(3-chlorophenyl)carbamoyl]amino}phenyl)ethyl]amino}(phenyl)methylene]-2-oxo-N-[(1R)-1-phenylethyl]indoline-5-carboxamide

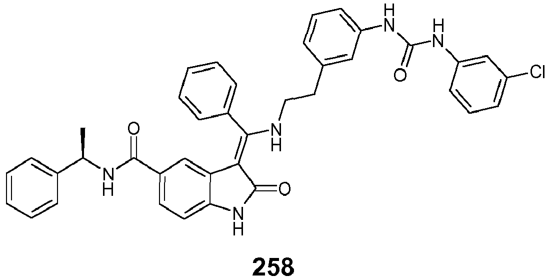

MS (ESI) [M+H]⁺: 657

### (3Z)-N-benzyl-3-{[(2-{3-[(4Z)-2,5-dioxo-4-(pyridin-4-ylmethylene)imidazolidin-1-yl]phenyl}ethyl)amino](phenyl)methylene}-2-oxoindoline-5-carboxamide

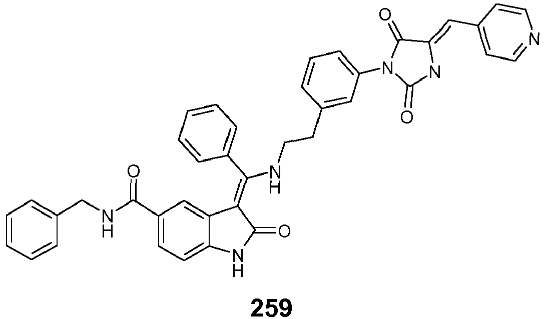

MS (ESI) [M+H]⁺: 662

### (3Z)-3-{[(2-{4-[(ethylcarbamoyl)amino]phenyl}ethyl)amino](phenyl)methylene}-2-oxo-N-[(1R)-1-phenylethyl]indoline-5-carboxamide

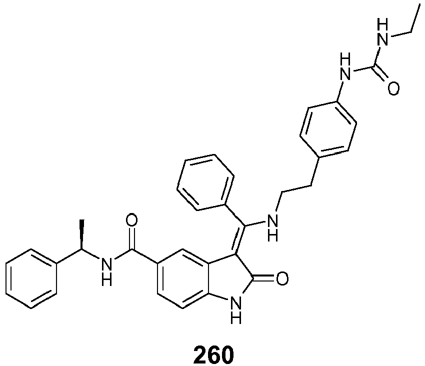

MS (ESI) [M+H]⁺: 575

### (3Z)-3-[{[2-(4-{[(3-chlorophenyl)carbamoyl]amino}phenyl)ethyl]amino} (phenyl)methylene]-2-oxo-N-[(1R)-1-phenylethyl]indoline-5-carboxamide

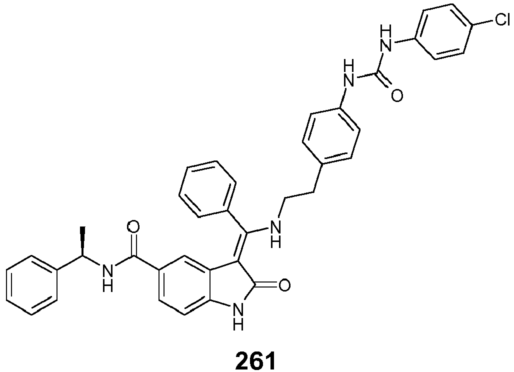

MS (ESI) [M+H]⁺:657

### (3Z)-3-{[(3S)-1-azabicyclo[2.2.2]oct-3-ylamino](phenyl)methylene}-2-oxo-N-[(1R)-1-phenylethyl]indoline-5-carboxamide

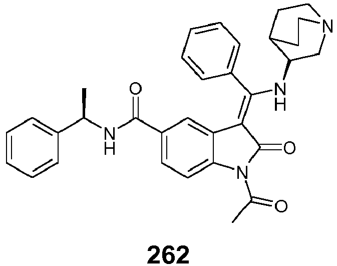

MS (ESI) [M+H]⁺: 536

### (3Z)-3-{[(3R)-1-azabicyclo[2.2.2]oct-3-ylamino](phenyl)methylene}-2-oxo-N-[(1R)-1-phenylethyl]indoline-5-carboxamide

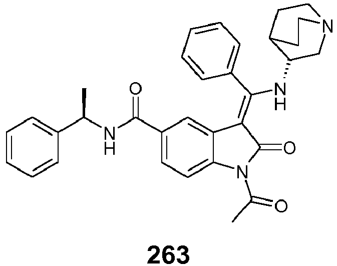

MS (ESI) [M+H]⁺: 536

### (3Z)-3-{[(3R)-1-azabicyclo[2.2.2]oct-3-ylamino](phenyl)methylene}-N-benzyl-2-oxoindoline-5-carboxamide

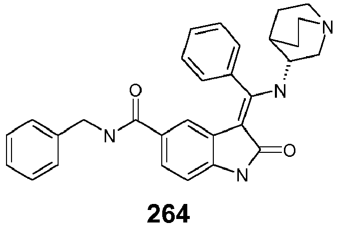

MS (ESI) [M+H]⁺:522

### (3Z)-3-{[(3S)-1-azabicyclo[2.2.2]oct-3-ylamino](phenyl)methylene}-2-oxo-N-[(1R)-1-phenylethyl]indoline-5-carboxamide

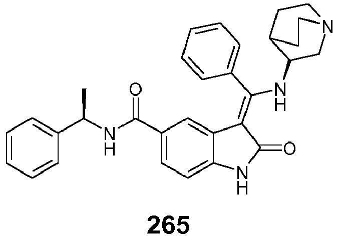

MS (ESI) [M+H]⁺: 494

### (3Z)-3-{[(3R)-1-azabicyclo[2.2.2]oct-3-ylamino](phenyl)methylene}-2-oxo-N-[(1R)-1-phenylethyl]indoline-5-carboxamide

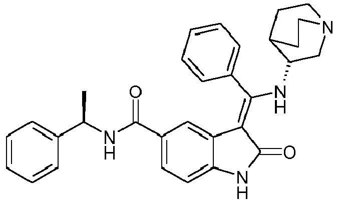

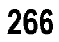

MS (ESI) [M+H]⁺: 494

### (3Z)-3-{[(3S)-1-azabicyclo[2.2.2]oct-3-ylamino](phenyl)methylene}-2-oxo-N-[(1R)-1-phenylpropyl]indoline-6-carboxamide

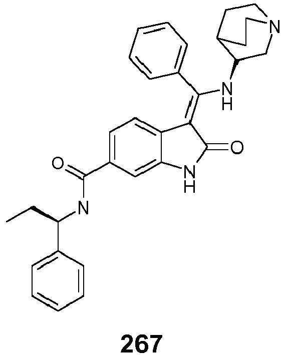

MS (ESI) [M+H]⁺: 508

### (3Z)-3-{[(3R)-1-azabicyclo[2.2.2]oct-3-ylamino](pyridin-4-yl)methylene}-2-oxo-N-[(1 R)-1-phenylpropyl]indoline-6-carboxamide

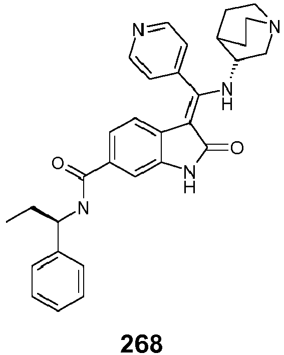

MS (ESI) [M+H]⁺: 509

### (3Z)-3-{[(3R)-1-azabicyclo[2.2.2]oct-3-ylamino](3-thienyl)methylene}-N-benzyl-2-oxoindoline-6-carboxamide

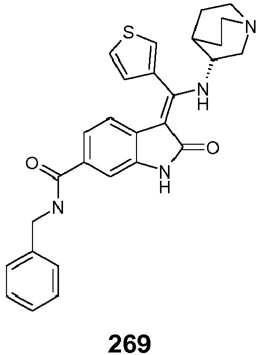

MS (ESI) [M+H]⁺: 486

### Part B: Biological evaluation

### Part B: Biological evaluation

### Methods and Material

The activity of the compounds described in the present invention was determined by the following kinase assays.

### In vitro Akt1 assay

The inhibition of Akt1 by the compounds described in the present invention were determined measuring the phosphorylation of a fluorescently-labeled peptide (TAMRA-peptide) by human Akt1 kinase by fluorescent polarization using a commercially available IMAP Screening Express Assay Kit (Molecular Devices).

Akt1 kinase assays were performed in low protein binding 384-well plates (Corning). Test compounds were diluted in 100% DMSO to 5 mM stock concentration and then further dilutions were made in H₂O or 100% DMSO to desirable concentrations.

Each reaction consisted of 5 nM enzyme: Akt1 kinase, 400 nM TAMRA-peptide (5-TAMRA-GRTGRRNSI-COOH; Genecust), 40 µM ATP and kinase buffer: 20 mM MES pH6, 1 mM DTT, 10 mM MgCl₂, 2 mM MnCl₂, 0.01 V/V% TritonX (all reagents from Sigma-Aldrich).

For each reaction, 4 or 6 µl containing TAMRA-peptide, ATP and kinase buffer were combined with 2 µl diluted compound in H₂O or 0.02 µl compound in 100% DMSO. The kinase reaction was started by the addition of 2 µl diluted enzyme. The reaction was allowed to run for 1 hour at room temperature. The reaction was stopped by adding 15 µl IMAP beads (1:400 beads in progressive (75% buffer A, 25% buffer B) 1x buffer). After an additional hour, fluorescent polarization (Ex: 550-10 nm, Em: 590-10 nm, Dich: 561 nm) was measured using an Analyst GT (Molecular Devices).

### In vitro Ack1 assay

The activity of the compounds described in the present invention can be determined by the following kinase assay, which measures the generation of ADP by human Ack1 kinase by fluorescent polarization using a commercially available TranScreener ADP2 FP Assay Kit (BellBrook Labs).

Ack1 kinase assays were performed in low protein binding 384-well plates (Corning). Test compounds were diluted in 100% DMSO to 5 mM stock concentration and then further dilutions were made in H₂O or 100% DMSO to desirable concentrations.

Each reaction consisted of 5 nM enzyme: Ack1 kinase, Poly Glu-Tyr (4:1) (Sigma), 3.5 µM ATP and kinase buffer: 20 mM HEPES pH7.5, 1 mM DTT, 2 mM MgCl₂, 0.4 mM MnCl2, 0.01 V/V% Tween20 (all reagents from Sigma-Aldrich)

For each reaction, 4 or 6 µl containing Poly Glu-Tyr (4:1), ATP and kinase buffer were combined with 2 µl diluted compound in H₂O or 0.02 µl compound in 100% DMSO. The kinase reaction was started by the addition of 2 µl diluted enzyme. The reaction was allowed to run for 1 hour at room temperature. The reaction was stopped by addition of 8 µl ADP detection mixture (1 x) (1 x Detection buffer + 4.78 µg/ml ADP2 Ab + 4 nM ADP Alexa633 Tracer). After an additional hour, fluorescent polarization (Ex: 630-35 nm, Em: 680-30 nm, Dich: 650 nm) was measured using an Analyst GT (Molecular Devices).

### In vitro Clk1 assay

The activity of the compounds described in the present invention can be determined by the following kinase assay, which measures the phosphorylation of a fluorescently-labeled peptide by human Clk1 kinase by fluorescent polarization using a commercially available IMAP Screening Express Assay Kit (Molecular Devices).

Clk1 kinase assays were performed in low protein binding 384-well plates (Corning). Test compounds were diluted in 100% DMSO to 5 mM stock concentration and then further dilutions were made in H₂O or 100% DMSO to desirable concentrations.

Each reaction consisted of 4 nM enzyme: Clk1 kinase domain, 400 nM TAMRA-AFRREWSPGKEAKK-NH2 (Genecust), 0.45 µM ATP (Sigma) and kinase buffer: 20 mM TRIS pH 8, 1 mM DTT, 0.4 mM MgCl₂, 0.4 mM MnCl₂, 0.01 V/V% Tween20 (all reagents from Sigma-Aldrich)

For each reaction, 4 or 6 µl containing TAMRA-peptide, ATP and kinase buffer were combined with 2 µl diluted compound in H₂O or 0.02 µl compound in 100% DMSO. The kinase reaction was started by the addition of 2 µl diluted enzyme. The reaction was allowed to run for 1 hour at room temperature. The reaction was stopped by adding 15 µl IMAP beads (1:600 beads in progressive (80 % buffer A, 20% buffer B) 1x buffer). After an additional hour, fluorescent polarization (Ex: 550-10 nm, Em: 590-10 nm, Dich: 561 nm) was measured using an Analyst GT (Molecular Devices).

### Example 3: High throughput screening of chemical compounds to identify small molecules that inhibit influenza virus replication

Human lung epithelial A549 cells were pre-treated on 384 well plates with the various substances in eight different 1:3 dilutions two hours before infection. Infection was performed using 130 plaque forming units (PFU) of the H1 N1 influenza virus strain A/WSN/33. Subsequently, compounds were re-added at the same concentrations. 36 hours post infection virus-containing supernatants were transferred to Madin-Darby Canine Kidney Epithelial (MDCK) cells, seeded the day before, to quantify the virus titer. The MDCK cells were fixed with formaldehyde 7 hours post infection and the percentage of infected to non-infected cells was quantified by staining cells with an influenza virus specific antibody (recognizing the viral nucleoprotein) and with Hoechst dye (to label all cell nuclei), followed by automated microscopy and single object analysis. Based on the results of the eight different dilutions dose-response-curves could be calculated and the IC50 values of each compound were determined. All experiments were performed within the BSL-3 lab using the FXP Laboratory Automation Workstation (Beckman Coulter). The whole screening process is illustrated in Fig. 1.

To be able to exclude possible cytotoxic effects all compounds were additionally tested using the 'Cell Proliferation Reagent WST-1' (Roche Diagnostics). Therefore, A549 cells were treated with the same concentrations of the various substances and 36 hours later the WST-1 was added to the cells. Finally, the read-out of this test was conducted at 48 hours post treatment using the EnVision Multilabel Reader (PerkinElmer).

### Cell line

A549 human lung carcinoma cells were cultured in RPMI 1640 medium (Sigma) supplemented with 10% fetal bovine serum (Sigma) and Antibiotic-Antimycotic (Sigma) in tissue culture flasks at 37°C in a humidified 5% CO₂ atmosphere. Cells were routinely assayed for mycoplasma contamination.

### Cell-based EC50 determination

Two thousand cells were plated in 384-well tissue culture plates, and treated after 24 hours with a 10-point dilution series of the compounds (1.5 nM-30 uM), using the vehicle, DMSO as control. Cells were incubated for 24 hours after which the relative cell density was determined by CellTiter-Glo Luminescent Cell Viability Assay (Promega). This method determines the number of metabolically active cells in the culture based on quantitation of the ATP present. The luminescent signal was measured with an Analyst GT multimode reader (Molecular Devices).

**Table 2: Inhibition of influenza virus replication and relevant kinase activity of different compounds of general formula (I).**

| **patent ID** | **Akt1 inh% @12.5 uM** | **Akt1 IC₅₀ (uM)** | **Ack1 inh% @12.5 uM** | **Clk1 IC₅₀** | **Influenza replication IC₅₀** | **A549 EC₅₀ 24h** |
|---|---|---|---|---|---|---|
| 12 | 75.1 | 7.98 | 18.1 | 0.21 | 0.01 | 6,89 |
| 13 | 60.6 | 5.94 | 13.7 | 0.03 | 0.16 | 9,34 |
| 18 | 92.1 | 0.57 | 85.9 | 0.03 | <0.01 | >30 |
| 20 | 91.6 | 0.91 | 72.7 | 0.01 | <0.01 | >30 |
| 21 | 78.2 | 1.70 | 87.0 | 0.02 | 1.48 | >30 |
| 22 | 88.0 | 0.11 | 83.8 | 0.02 | <0.01 | >30 |
| 23 | 92.1 | 0.18 | 91.1 | 0.02 | <0.01 | 15,27 |
| 24 | 76.8 | 0.88 | 13.7 | 0.08 | 0.42 | 11,79 |
| 27 | 81.3 | 2.96 | 9.9 | 0.20 | 2.46 | 25,35 |
| 30 | 93.0 | 1.30 | 80.5 | 0.01 | <0.01 | 5,98 |
| 32 | n.d. | >12.5 | n.d. | 0.10 | 10.06 | 6,38 |
| 35 | n.d. | 2.51 | n.d. | 0.01 | 0.02 | 4,99 |
| 36 | n.d. | 0.33 | n.d. | 0.02 | <0.01 | 1,77 |
| 37 | n.d. | 2.05 | n.d. | 0.01 | <0.01 | 2,01 |
| 38 | n.d. | 1.83 | n.d. | 0.01 | <0.01 | 3,67 |
| 39 | n.d. | 12.29 | n.d. | 0.01 | 0.10 | 18,76 |
| 40 | n.d. | 3.40 | n.d. | 0.01 | <0.01 | 14,64 |

### Example 4: Assessment of solubility

Samples were diluted in DMSO (control), phosphate buffer pH = 7.4 and pH = 2.0, from 5mM stock solutions (solved in DMSO), with a 120 µM final concentration. The samples were incubated for 24 hours at room temperature followed by 30 minutes centrifugation at 3700 rpm. 40 µl of the supernatants were injected into HPLC (Waters 2795 Alliance HPLC equipped with Waters 996 PDA Detector (HPLC column: Waters XBridge, RP C18, 3.5 µm, 4.6 mm x 50 mm; gradient MeCN/H2O, containing 0.1% HCOOH: 5% MeCN (0.5 min), 5% to 95% MeCN (5 min), 95% MeCN (0.5 min); flow rate: 2.0 ml/min)) and the AUC values (measured on sample specific wavelength) of the buffered samples were divided by the AUC values (same wavelength as buffered samples') of the DMSO control samples.

**Table 3: Solubility in water for several compounds according to general formula (I)**

| ID | Solubility pH = 7.4 (µM; max 120 µM) | Solubility pH = 2.0 (µM; max 120 µM) |
|---|---|---|
| 12 | < 10 | 110 - 120 |
| 13 | < 10 | 110 - 120 |
| 18 | < 10 | 110 - 120 |
| 20 | < 10 | 110 - 120 |
| 21 | < 10 | 100 - 110 |
| 22 | < 10 | 110 - 120 |
| 23 | < 10 | 110 - 120 |
| 24 | < 10 | 110 - 120 |
| 27 | < 10 | 110 - 120 |
| 30 | < 10 | 110 - 120 |
| 32 | < 10 | 110 - 120 |
| 35 | 10 - 20 | 110 - 120 |
| 36 | < 10 | 100 - 110 |
| 37 | < 10 | 110 - 120 |
| 38 | 10 - 20 | 110 - 120 |
| 39 | < 10 | 110 - 120 |
| 40 | < 10 | 110 - 120 |

### Example 5: In vitro growth inhibition of colon carcinoma cells

### Cell lines

HCT-116 and HKE3 human colon carcinoma cell lines (two completely identical cell lines that differ only in KRas mutation status) and HepG2 hepatoblastomaderived cell line were cultured in RPMI 1640 medium (Sigma) supplemented with 10% fetal bovine serum (Sigma) and Antibiotic-Antimycotic (Sigma) in tissue culture flasks at 37°C in a humidified 5% CO₂ atmosphere. Cell lines were routinely assayed for mycoplasma contamination.

### Cell-based EC₅₀ determination

One thousand cells were plated in 384-well tissue culture plates, and treated after 24 hours with a 10-point dilution series of the compounds (1.5 nM-30 µM), using the vehicle, DMSO as control. Cells were incubated for 72 hours after which the relative cell density was determined by CellTiter-Glo Luminescent Cell Viability Assay (Promega). This method determines the number of metabolically active cells in the culture based on quantitation of the ATP present. The luminescent signal was measured with an Analyst GT multimode reader (Molecular Devices).

**Table 4. Growth inhibition of human colon carcinoma cell lines**

| **compound ID** | **HCT116 EC₅₀ (uM)** | **HKE3 EC₅₀ (uM)** | **HepG2 EC₅₀ (uM)** |
|---|---|---|---|
| **12** | 0.03 | 0.02 | 3.52 |
| **13** | 0.17 | 0.14 | 5.66 |
| **18** | 0.01 | 0.004 | 0.31 |
| **20** | 0.02 | 0.01 | 0.79 |
| **21** | 0.41 | 0.32 | 5.30 |
| **22** | 0.04 | 0.02 | 1.06 |
| **23** | 0.04 | 0.04 | 0.92 |
| **24** | 0.38 | 0.29 | 6.09 |
| **27** | 1.41 | 1.32 | 5.24 |
| **30** | 0.01 | 0.01 | 0.58 |
| **35** | 0.03 | 0.02 | 2.12 |
| **36** | 0.02 | 0.02 | 0.64 |
| **37** | 0.001 | <0.00152 | 0.36 |
| **38** | 0.01 | 0.01 | 1.12 |
| **39** | 3.80 | 2.96 | 24.20 |
| **40** | 0.11 | 0.08 | 10.00 |

## Claims

1. The compound of general formula (I) 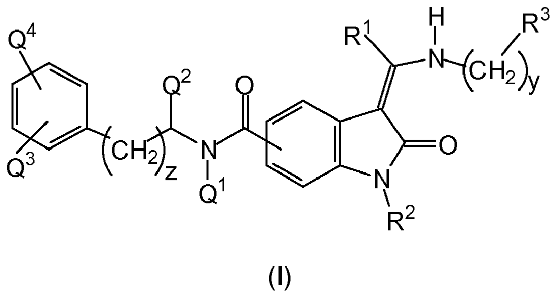 wherein,
y is an integer number selected from: 0, 1, and 2;
z is an integer number selected from: 0, 1, and 2;
Q¹ represents: -H or -CH₃;
Q² represents: -H, -CH₃ or -CH₂CH₃;
Q³ and Q⁴ are independently of each other selected from: -H, -F, -Cl, -CH₃, and -OCH₃;
R¹ represents: -CH₂CH₃, -CH(CH₃)₂, 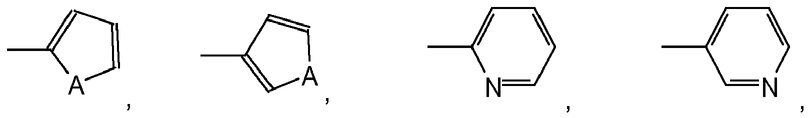 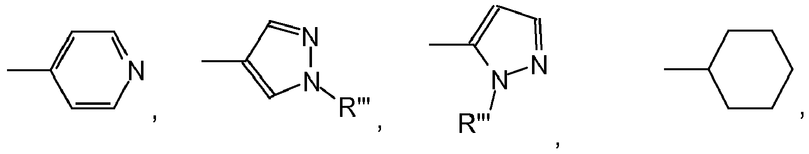 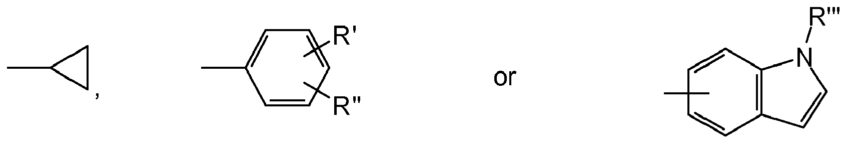
R' and R" are independently of each other selected from: -H, -F, -CH₃ and -OCH₃;
R'" represents: -H or -CH₃;
A represents -NH-, -O- or -S-;
R² represents: -H, -CH₃ or -C(=O)CH₃;
R³ represents: 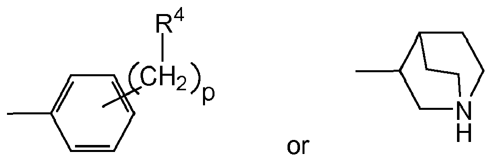
R⁴ represents: -R¹², -NR⁷R⁸, 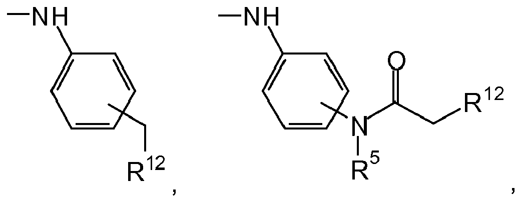 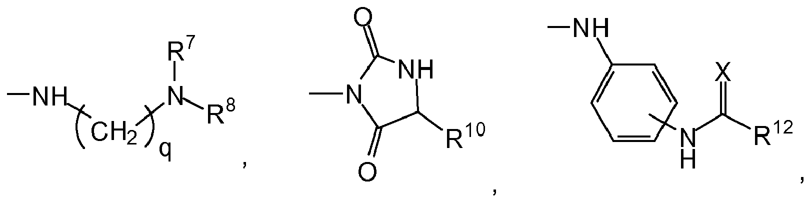 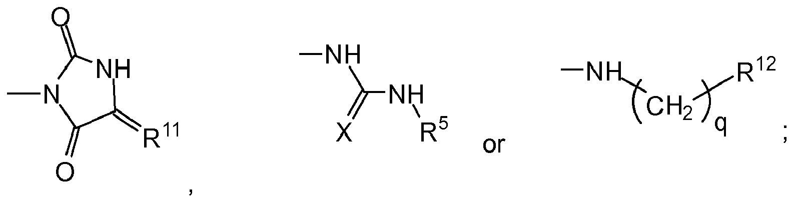
X represents: =O or =S;
R⁵ is: -R⁶, 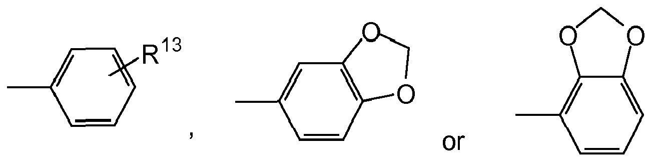
R⁶, R⁷, R⁸, R⁹ and R¹⁰ are independently of each other selected form : -H, -CH₃, -C₂H₅, -C₃H₇, -CH(CH₃)₂, -C(CH₃)₃-(CH₂)ₙ-CH₃, -(CH₂)ₙ-C(CH₃)₂, -CH[(CH₂)ₙCH₃]-(CH₂)ₘ-CH₃, -CH[(CH₂)ₙCH₃]-(CH₂)ₘ-C(CH₃)₂, -CH=CH₂, -(CH₂)ₙ-CH=CH-(CH₂)ₖ-CH₃, -CH≡CH, -(CH₂)ₙ-C≡C-(CH₂)ₖ-CH₃, -Ph, -(CH₂)ₙ-Ph, -(CH₂)ₙ-OH, -(CH₂)ₙ-NH₂, -(CH₂)ₙ-O-(CH₂)ₖ-CH₃, -(CH₂)ₙ-NH(CH₂)ₖ-CH₃, -(CH₂)ₙ-N(CH₃)₂, -(CH₂)ₙ-N(C₂H₅)₂ and -(CH₂)ₙ-N(CH₃)(C₂H₅);
R¹³ is: -OH, -Cl, 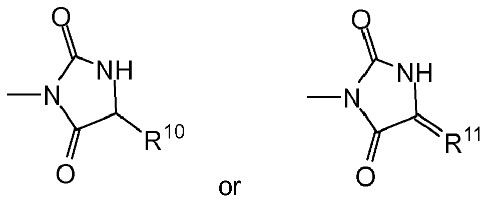
R¹¹ represents: =CH₂, =C(CH₃)₂, 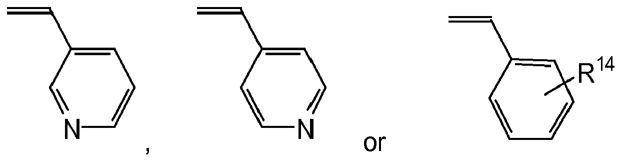
R¹⁴ represents: -H, -F, -Cl, -Br, -I, -OH or -OCH₃;
R¹² represents: 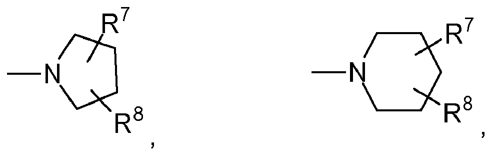 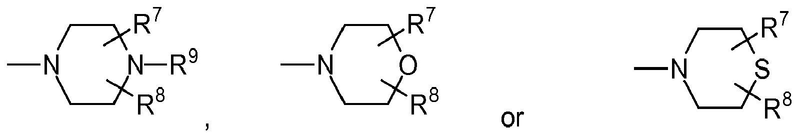
k and I are integer numbers and independently of each other selected from: 0, 1, 2, 3, 4 and 5;
m, n, and q are integer numbers and independently of each other selected from: 1, 2, 3, 4 and 5;
p is an integer number selected from: 0 and 1
and stereoisomeric forms, E/Z isomers, enantiomers, mixtures of enantiomers, anomers, deoxyforms, diastereomers, mixtures of diastereomers, prodrugs, tautomers, hydrates, solvates and racemates of the above mentioned compounds and pharmaceutically acceptable salts thereof, for the use in the treatment of infectious diseases or cancer.

2. Compound for use according to claim 1, wherein z represents 0.

3. Compound for use according to claims 1 and 2, wherein R¹ represents -Ph.

4. Compound for use according to anyone of claims 1 - 3, wherein y represents 0.

5. Compound for use according to anyone of claims 1 - 4, wherein Q² represents: -CH₃ or -C₂H₅ and the stereogenic center is (R) configurated.

6. Compound for use according to claim 1, wherein y is 0, R² is selected from: -H and -COCH₃ and R³ represents

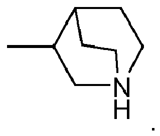

7. Compound for use according to claim 1, wherein the compound is selected from the following group:
| No. | Compound |
|---|---|
| 12 | (3*Z*)-2-oxo-3-(phenyl{[3-(piperidin-1-ylmethyl)phenyl]amino}methylene)-*N*-[(1*R*)-1-phenylpropyl]indoline-5-carboxamide |
| 13 | (3*Z*)-2-oxo-*N*-[(1*R*)-1-phenylethyl]-3-(phenyl{[3-(piperidin-1-ylmethyl)phenyl]amino}methylene)indoline-5-carboxamide |
| 14 | (3*Z*)-2-oxo-3-(phenyl{[3-(piperidin-1-ylmethyl)phenyl]amino}methylene)-*N-*[(1 S)-1-phenylpropyl]indoline-5-carboxamide |
| 15 | (3*Z*)-*N*-[(1*S*)-1-(4-chlorophenyl)ethyl]-2-oxo-3-(phenyl{[3-(piperidin-1-ylmethyl)phenyl]amino}methylene)indoline-5-carboxamide |
| 16 | (3*Z*)-*N*-[(1*R*)-1-(4-chlorophenyl)ethyl]-2-oxo-3-(phenyl{[3-(piperidin-1-ylmethyl)phenyl]amino}methylene)indoline-5-carboxamide |
| 17 | (3*Z*)-*N*-[(1*R*)-1-(4-fluorophenyl)ethyl]-2-oxo-3-(phenyl{[3-(piperidin-1-ylmethyl)phenyl]amino}methylene)indoline-5-carboxamide |
| 18 | (3*Z*)-2-oxo-3-(phenyl{[4-(piperidin-1-ylmethyl)phenyl]amino}methylene)-*N-*[(1 R)-1-phenylpropyl]indoline-5-carboxamide |
| 19 | (3*Z*)-*N*-methyl-2-oxo-*N*-[(1*R*)-1-phenylethyl]-3-(phenyl{[3-(piperidin-1-ylmethyl)phenyl]amino}methylene)indoline-5-carboxamide |
| 20 | (3*Z*)-*N*-benzyl-2-oxo-3-(phenyl{[4-(piperidin-1-ylmethyl)phenyl]amino} methylene)indoline-5-carboxamide |
| 21 | (3*Z*)-*N*-[(1*R*)-1*-*(4-methoxyphenyl)ethyl]-2-oxo-3-(phenyl{[4-(piperidin-1-ylmethyl)phenyl]amino}methylene)indoline-5-carboxamide |
| 22 | (3*Z*)-*N*-[(1*R*)-1-(3-methoxyphenyl)ethyl]-2-oxo-3-(phenyl{[4-(piperidin-1-ylmethyl)phenyl]amino}methylene)indoline-5-carboxamide |
| 23 | (3*Z*)-*N*-[(1*R*)-1-(4-fluorophenyl)ethyl]-2-oxo-3-(phenyl{[4-(piperidin-1-ylmethyl)phenyl]amino}methylene)indoline-5-carboxamide |
| 24 | (3*Z*)-*N*-[(1*R*)-1-(3-methoxyphenyl)ethyl]-2-oxo-3-(phenyl{[3-(piperidin-1-ylmethyl)phenyl]amino}methylene)indoline-5-carboxamide |
| 25 | (3*Z*)-2-oxo-*N*-[(1*S*)-1-phenylethyl]-3-(phenyl{[3-(piperidin-1-ylmethyl)phenyl]amino}methylene)indoline-5-carboxamide |
| 26 | (3*Z*)-*N*-[(1*S*)-1-(4-fluorophenyl)ethyl]-2-oxo-3-(phenyl{[3-(piperidin-1-ylmethyl)phenyl]amino}methylene)indoline-5-carboxamide |
| 27 | (3*Z*)-*N*-[(1*R*)-1-(4-methylphenyl)ethyl]-2-oxo-3-(phenyl{[3-(piperidin-1-ylmethyl)phenyl]amino}methylene)indoline-5-carboxamide |
| 28 | (3*Z*)-*N*-[(1*S*)-1-(4-methylphenyl)ethyl]-2-oxo-3-(phenyl{[3-(piperidin-1-ylmethyl)phenyl]amino}methylene)indoline-5-carboxamide |
| 29 | (3*Z*)-*N*-[(1*R*)-1-(4-methoxyphenyl)ethyl]-2-oxo-3-(phenyl{[3-(piperidin-1-ylmethyl)phenyl]amino}methylene)indoline-5-carboxamide |
| 30 | (3*Z*)-2-oxo-*N*-[(1*S*)-1-phenylethyl]-3-(phenyl{[4-(piperidin-1-ylmethyl)phenyl]amino}methylene)indoline-5-carboxamide |
| 31 | (3*Z*)-2-oxo-*N*-[(1*R*)-1-phenylethyl]-3-(phenyl{[4-(piperidin-1-ylmethyl)phenyl]amino}methylene)indoline-5-carboxamide |
| 32 | (3*Z*)-2-oxo-3-(phenyl{[4-(piperidin-1-ylmethyl)phenyl]amino}methylene)-*N-*[(1 S)-1-phenylpropyl]indoline-5-carboxamide |
| 33 | (3*Z*)-*N*-[(1*S*)-1-(4-methoxyphenyl)ethyl]-2-oxo-3-(phenyl{[4-(piperidin-1-ylmethyl)phenyl]amino}methylene)indoline-5-carboxamide |
| 34 | (3*Z*)-*N*-[(1*S*)-1-(3-methoxyphenyl)ethyl]-2-oxo-3-(phenyl{[4-(piperidin-1-ylmethyl)phenyl]amino}methylene)indoline-5-carboxamide |
| 35 | (3*Z*)-*N*-[(1*R*)-1-(3-methoxyphenyl)ethyl]-3-[({4-[(4-methylpiperazin-1-yl)methyl]phenyl}amino)(phenyl)methylene]-2-oxoindoline-5-carboxamide |
| 36 | (3*Z*)-*N*-[(1*R*)-1-(3-chlorophenyl)ethyl]-3-[({4-[(4-methylpiperazin-1-yl)methyl]phenyl}amino)(phenyl)methylene]-2-oxoindoline-5-carboxamide |
| 37 | (3*Z*)-3-[({4-[(4-methylpiperazin-1-yl)methyl]phenyl}amino)(phenyl)methylene]-2-oxo-*N*-[(1*R)-1-*phenylpropyl]indoline-5-carboxamide |
| 38 | (3*Z*)-3-[({4-[(4-methylpiperazin-1-yl)methyl]phenyl}amino)(phenyl)methylene]-2-oxo-*N*-[(1*R)-*1-phenylethyl]indoline-5-carboxamide |
| 39 | (3*Z*)-*N*-(3,4-dimethoxybenzyl)-3-[({4-[(4-methylpiperazin-1-yl)methyl]phenyl}amino)(phenyl)methylene]-2-oxoindoline-5-carboxamide |
| 40 | (3*Z*)-*N*-(3-methoxybenzyl)-3-[({4-[(4-methylpiperazin-1-yl)methyl]phenyl}amino)(phenyl)methylene]-2-oxoindoline-5-carboxamide |
| 41 | (3*Z*)-2-oxo-*N*-[(1*R*)-1-phenylethyl]-3-(phenyl{[4-(piperidin-1-ylmethyl)phenyl]amino}methylene)indoline-6-carboxamide |
| 42 | (3*Z*)-2-oxo-3-(phenyl{[4-(piperidin-1-ylmethyl)phenyl]amino}methylene)-*N-*[(1 R)-1-phenylpropyl]indoline-6-carboxamide |
| 43 | (3*Z*)-3-[({3-[(4-methylpiperazin-1-yl)methyl]phenyl}amino)(phenyl)methylene]-2-oxo-*N*-[(1*R*)-1-phenylethyl]indoline-5-carboxamide |
| 44 | (3*Z*)-3-[({3-[(4-methylpiperazin-1-yl)methyl]phenyl}amino)(phenyl)methylene]-2-oxo-*N*-[(1R)-1-phenylpropyl]indoline-5-carboxamide |
| 45 | (3*Z*)-*N*-benzyl-3-[({3-[(4-methylpiperazin-1-yl)methyl]phenyl}amino)(phenyl)methylene]-2-oxoindoline-5-carboxamide |
| 46 | (3*Z*)-*N*-[(1*R*)-1-(3-chlorophenyl)ethyl]-3-[({3-[(4-methylpiperazin-1-yl)methyl]phenyl}amino)(phenyl)methylene]-2-oxoindoline-5-carboxamide |
| 47 | (3*Z*)-*N*-[(1*R*)-1-(3-methoxyphenyl)ethyl]-3-[({3-[(4-methylpiperazin-1-yl)methyl]phenyl}amino)(phenyl)methylene]-2-oxoindoline-5-carboxamide |
| 48 | (3*Z*)-*N*-(3-methoxybenzyl)-3-[({3-[(4-methylpiperazin-1-yl)methyl]phenyl}amino) (phenyl)methylene]-2-oxoindoline-5-carboxamide |
| 49 | (3*Z*)-*N*-(3,4-dimethoxybenzyl)-3-[({3-[(4-methylpiperazin-1-yl)methyl]phenyl}amino)(phenyl)methylene]-2-oxoindoline-5-carboxamid |
| 135 | (3*Z*)-3-[({4-[(4-methylpiperazin-1-yl)methyl]phenyl}amino)(pyridin-4-yl)methylene]-2-oxo-*N*-[(1*R*)-1-phenylpropyl]indoline-5-carboxamide |
| 136 | (3*Z*)-*N*-benzyl-3-[({4-[(4-methylpiperazin-1-yl)methyl]phenyl}amino)(pyridin-3-yl)methylene]-2-oxoindoline-5-carboxamide |
| 137 | (3*Z*)-*N*-(4-fluorobenzyl)-3-[({4-[(4-methylpiperazin-1-yl)methyl]phenyl}amino)(pyridin-3-yl)methylene]-2-oxoindoline-5-carboxamide |
| 138 | (3*Z*)-3-[({4-[(4-methylpiperazin-1-yl)methyl]phenyl}amino)(pyridin-3-yl)methylene]-2-oxo-*N*-[(1*R*)-1-phenylethyl]indoline-5-carboxamide |
| 139 | (3*Z*)-3-[({4-[(4-methylpiperazin-1-yl)methyl]phenyl}amino)(pyridin-3-yl)methylene]-2-oxo-*N*-[(1*R*)-1-phenylpropyl]indoline-5-carboxamide |
| 140 | (3*Z*)-2-oxo-*N*-[(1*R*)-1-phenylethyl]-3-[{[4-(piperidin-1-ylmethyl)phenyl]amino}(pyridin-3-yl)methylene]indoline-5-carboxamide |
| 141 | (3*Z*)-2-oxo-*N*-[(1*S*)-1-phenylethyl]-3-[{[4-(piperidin-1-ylmethyl)phenyl]amino}(pyridin-3-yl)methylene]indoline-5-carboxamide |
| 142 | (3*Z*)-2-oxo-*N*-[(1*R*)-1-phenylpropyl]-3-[{[4-(piperidin-1-ylmethyl)phenyl]amino}(pyridin-3-yl)methylene]indoline-5-carboxamide |
| 143 | (3*Z*)-3-[({4-[(diethylamino)methyl]phenyl}amino)(pyridin-3-yl)methylene]-2-oxo-*N*-[(1*R*)-1-phenylpropyl]indoline-5-carboxamide |
| 144 | (3*Z*)-3-[({4-[(diethylamino)methyl]phenyl}amino)(pyridin-3-yl)methylene]-2-oxo-*N*-[(1*R*)-1-phenylethyl]indoline-5-carboxamide |
| 145 | (3*Z*)-2-oxo-*N*-[(1*S*)-1-phenylpropyl]-3-[{[4-(piperidin-1-ylmethyl)phenyl]amino}(pyridin-3-yl)methylene]indoline-5-carboxamide |
| 146 | (3*Z*)-3-[{[4-(morpholin-4-ylmethyl)phenyl]amino}(pyridin-3-yl)methylene]-2-oxo-*N*-[(1*R*)-1-phenylethyl]indoline-5-carboxamide |
| 147 | (3*Z*)-3-[{[4-(morpholin-4-ylmethyl)phenyl]amino}(pyridin-3-yl)methylene]-2-oxo-*N*-[(1*R*)-1-phenylpropyl]indoline-5-carboxamide |
| 148 | (3*Z*)-3-[({3-[(4-methylpiperazin-1-yl)methyl]phenyl}amino)(pyridin-3-yl)methylene]-2-oxo-*N*-[(1*R*)-1-phenylethyl]indoline-5-carboxamide |
| 149 | (3*Z*)-3-[({3-[(4-methylpiperazin-1-yl)methyl]phenyl}amino)(pyridin-3-yl)methylene]-2-oxo-*N*-[(1*R*)-1-phenylpropyl]indoline-5-carboxamide |
| 150 | (3*Z*)-2-oxo-*N*-[(1*R*)-1-phenylpropyl]-3-[{[3-(piperidin-1-ylmethyl)phenyl]amino}(pyridin-3-yl)methylene]indoline-5-carboxamide |
| 151 | (3*Z*)-2-oxo-*N*-[(1*S*)-1-phenylpropyl]-3-[{[3-(piperidin-1-ylmethyl)phenyl]amino}(pyridin-3-yl)methylene]indoline-5-carboxamide |
| 152 | (3*Z*)-2-oxo-*N*-[(1*S*)-1-phenylethyl]-3-[{[3-(piperidin-1-ylmethyl)phenyl]amino}(pyridin-3-yl)methylene]indoline-5-carboxamide |
| 153 | (3*Z*)-2-oxo-*N*-[(1*R*)-1-phenylethyl]-3-[{[3-(piperidin-1-ylmethyl)phenyl]amino}(pyridin-3-yl)methylene]indoline-5-carboxamide |
| 154 | (3*Z*)-2-oxo-*N*-[(1*R*)-1-phenylethyl]-3-[{[4-(piperidin-1-ylmethyl)phenyl]amino}(pyridin-2-yl)methylene]indoline-5-carboxamide |
| 155 | (3*Z*)-2-oxo-*N*-[(1*R*)-1-phenylpropyl]-3-[{[4-(piperidin-1-ylmethyl)phenyl]amino}(pyridin-2-yl)methylene]indoline-5-carboxamide |
| 156 | (3*Z*)-2-oxo-*N*-[(1*R*)-1-phenylethyl]-3-(pyridin-2-yl{[3-(pyrrolidin-1-ylmethyl)phenyl]amino}methylene)indoline-5-carboxamide |
| 157 | (3*Z*)-2-oxo-*N*-[(1*R*)-1-phenylpropyl]-3-(pyridin-2-yl{[3-(pyrrolidin-1-ylmethyl)phenyl]amino}methylene)indoline-5-carboxamide |
| 158 | (3*Z*)-2-oxo-*N*-[(1*R*)-1-phenylethyl]-3-[{[3-(piperidin-1-ylmethyl)phenyl]amino}(pyridin-2-yl)methylene]indoline-5-carboxamide |
| 159 | (3*Z*)-2-oxo-*N*-[(1*R*)-1-phenylpropyl]-3-[{[3-(piperidin-1-ylmethyl)phenyl]amino}(pyridin-2-yl)methylene]indoline-5-carboxamide |
| 160 | (3*Z*)-3-[({4-[(4-methylpiperazin-1-yl)methyl]phenyl}amino)(pyridin-4-yl)methylene]-2-oxo-*N*-[(1*R*)-1-phenylethyl]indoline-5-carboxamide |
| 161 | (3*Z*)-2-oxo-*N*-[(1*R*)-1-phenylpropyl]-3-[{[4-(piperidin-1-ylmethyl)phenyl]amino}(pyridin-4-yl)methylene]indoline-5-carboxamide |
| 162 | (3*Z*)-2-oxo-*N*-[(1*S*)-1-phenylpropyl]-3-[{[4-(piperidin-1-ylmethyl)phenyl]amino}(pyridin-4-yl)methylene]indoline-5-carboxamide |
| 163 | (3*Z*)-2-oxo-*N*-[(1*R*)-1-phenylethyl]-3-[{[4-(piperidin-1-ylmethyl)phenyl]amino}(pyridin-4-yl)methylene]indoline-5-carboxamide |
| 164 | (3*Z*)-2-oxo-*N*-[(1*S*)-1-phenylethyl]-3-[{[4-(piperidin-1-ylmethyl)phenyl]amino}(pyridin-4-yl)methylene]indoline-5-carboxamide |
| 165 | (3*Z*)-3-[({4-[(diethylamino)methyl]phenyl}amino)(pyridin-4-yl)methylene]-2-oxo-*N*-[(1*R*)-1-phenylethyl]indoline-5-carboxamide |
| 166 | (3*Z*)-3-[({4-[(diethylamino)methyl]phenyl}amino)(pyridin-4-yl)methylene]-2-oxo-*N*-[(1*R*)-1-phenylpropyl]indoline-5-carboxamide |
| 167 | (3*Z*)-3-[{[4-(morpholin-4-ylmethyl)phenyl]amino}(pyridin-4-yl)methylene]-2-oxo-*N*-[(1*R*)-1-phenylethyl]indoline-5-carboxamide |
| 168 | (3*Z*)-3-[{[4-(morpholin-4-ylmethyl)phenyl]amino}(pyridin-4-yl)methylene]-2-oxo-*N*-[(1*R*)-1-phenylpropyl]indoline-5-carboxamide |
| 169 | (3*Z*)-2-oxo-*N*-[(1*R*)-1-phenylpropyl]-3-(pyridin-4-yl{[4-(pyrrolidin-1-ylmethyl)phenyl]amino}methylene)indoline-5-carboxamide |
| 170 | (3*Z*)-2-oxo-*N*-[(1*R*)-1-phenylethyl]-3-(pyridin-4-yl{[4-(pyrrolidin-1-ylmethyl)phenyl]amino}methylene)indoline-5-carboxamide |
| 171 | (3*Z*)-2-oxo-*N*-[(1*R*)-1-phenylethyl]-3-[{[4-(pyrrolidin-1-ylmethyl)phenyl]amino}(3-thienyl)methylene]indoline-5-carboxamide |
| 172 | (3*Z*)-2-oxo-*N*-[(1*R*)-1-phenylpropyl]-3-[{[4-(pyrrolidin-1-ylmethyl)phenyl]amino}(3-thienyl)methylene]indoline-5-carboxamide |
| 173 | (3*Z*)-2-oxo-*N*-[(1*R*)-1-phenylethyl]-3-[{[4-(piperidin-1-ylmethyl)phenyl]amino}(3-thienyl)methylene]indoline-5-carboxamide |
| 174 | (3*Z*)-2-oxo-*N*-[(1*R*)-1-phenylpropyl]-3-[{[4-(piperidin-1-ylmethyl)phenyl]amino}(3-thienyl)methylene]indoline-5-carboxamide |
| 175 | (3*Z*)-3-[{[4-(morpholin-4-ylmethyl)phenyl]amino}(3-thienyl)methylene]-2-oxo-*N*-[(1*R*)-1-phenylethyl]indoline-5-carboxamide |
| 176 | (3*Z*)-3-[{[4-(morpholin-4-ylmethyl)phenyl]amino}(3-thienyl)methylene]-2-oxo-*N*-[(1*R*)-1-phenylpropyl]indoline-5-carboxamide |
| 177 | (3*Z*)-3-[({4-[(4-methylpiperazin-1-yl)methyl]phenyl}amino)(3-thienyl)methylene]-2-oxo-*N*-[(1*R*)-1-phenylethyl]indoline-5-carboxamide |
| 178 | (3*Z*)-3-[({4-[(4-methylpiperazin-1-yl)methyl]phenyl}amino)(3-thienyl)methylene]-2-oxo-*N*-[(1*R*)-1-phenylpropyl]indoline-5-carboxamide |
| 179 180 | (3*Z*)-2-oxo-*N*-[(1*R*)-1-phenylethyl]-3-[{[3-(pyrrolidin-1-ylmethyl)phenyl]amino}(3-thienyl)methylene]indoline-5-carboxamide (3*Z*)-2-oxo-*N*-[(1*R*)-1-phenylpropyl]-3-[{[3-(pyrrolidin-1-ylmethyl)phenyl]amino}(3-thienyl)methylene]indoline-5-carboxamide |
| 181 | (3*Z*)-2-oxo-*N*-[(1*R*)-1-phenylethyl]-3-[{[3-(piperidin-1-ylmethyl)phenyl]amino}(3-thienyl)methylene]indoline-5-carboxamide |
| 182 | (3*Z*)-2-oxo-*N*-[(1*R*)-1-phenylpropyl]-3-[{[3-(piperidin-1-ylmethyl)phenyl]amino}(3-thienyl)methylene]indoline-5-carboxamide |
| 183 | (3*Z*)-3-[({3-[(4-methylpiperazin-1-yl)methyl]phenyl}amino)(3-thienyl)methylene]-2-oxo-*N*-[(1*R*)-1-phenylethyl]indoline-5-carboxamide |
| 184 | (3*Z*)-3-[({3-[(4-methylpiperazin-1-yl)methyl]phenyl}amino)(3-thienyl)methylene]-2-oxo-*N*-[(1*R*)-1-phenylpropyl]indoline-5-carboxamide |
| 185 | (3*Z*)-3-[{[3-(morpholin-4-ylmethyl)phenyl]amino}(3-thienyl)methylene]-2-oxo-*N*-[(1*R*)-1-phenylethyl]indoline-5-carboxamide |
| 186 | (3*Z*)-3-[{[3-(morpholin-4-ylmethyl)phenyl]amino}(3-thienyl)methylene]-2-oxo-*N*-[(1*R*)-1-phenylpropyl]indoline-5-carboxamide |
| 187 | (3*Z*)-3-(3-furyl{[4-(pyrrolidin-1-ylmethyl)phenyl]amino}methylene)-2-oxo-*N-*[(1*R*)-1-phenylethyl]indoline-5-carboxamide |
| 188 | (3*Z*)-3-(3-furyl{[4-(piperidin-1-ylmethyl)phenyl]amino}methylene)-2-oxo-*N-*[(1 *R*)-1-phenylpropyl]indoline-5-carboxamide |
| 189 | (3*Z*)-3-(2-furyl{[4-(piperidin-1-ylmethyl)phenyl]amino}methylene)-2-oxo-*N-*[(1 R)-1-phenylpropyl]indoline-5-carboxamide |
| 190 | (3*Z*)-3-[(1-methyl-1*H*-pyrazol-4-yl){[4-(pyrrolidin-1-ylmethyl)phenyl]amino}methylene]-2-oxo-*N*-[(1*R*)-1-phenylethyl]indoline-5-carboxamide |
| 191 | (3*Z*)-3-[(1-methyl-1*H*-pyrazol-4-yl){[4-(pyrrolidin-1-ylmethyl)phenyl]amino}methylene]-2-oxo-*N*-[(1*R*)-1-phenylpropyl]indoline-5-carboxamide |
| 192 | (3*Z*)-3-[(1-methyl-1*H*-pyrazol-4-yl){[4-(piperidin-1-ylmethyl)phenyl]amino}methylene]-2-oxo-*N*-[(1*R*)-1-phenylethyl]indoline-5-carboxamide |
| 193 | (3*Z*)-3-[(1-methyl-1*H*-pyrazol-4-yl){[4-(piperidin-1-ylmethyl)phenyl]amino}methylene]-2-oxo-*N*-[(1*R*)-1-phenylpropyl]indoline-5-carboxamide |
| 194 | (3*Z*)-3-[(1-methyl-1*H*-pyrazol-4-yl){[3-(pyrrolidin-1-ylmethyl)phenyl]amino}methylene]-2-oxo-*N*-[(1*R*)-1-phenylethyl]indoline-5-carboxamide |
| 195 | (3*Z*)-3-[(1-methyl-1*H*-pyrazol-4-yl){[3-(pyrrolidin-1-ylmethyl)phenyl]amino}methylene]-2-oxo-*N*-[(1*R*)-1-phenylpropyl]indoline-5-carboxamide |
| 196 | (3*Z*)-3-[(1-methyl-1*H*-pyrazol-4-yl){[3-(piperidin-1-ylmethyl)phenyl]amino}methylene]-2-oxo-*N*-[(1*R*)-1-phenylethyl]indoline-5-carboxamide |
| 197 | (3*Z*)-3-[(1-methyl-1*H*-pyrazol-4-yl){[3-(piperidin-1-ylmethyl)phenyl]amino}methylene]-2-oxo-*N*-[(1*R*)-1-phenylpropyl]indoline-5-carboxamide |
| 198 | (3*Z*)-3-[(1-methyl-1*H*-indol-5-yl){[4-(pyrrolidin-1-ylmethyl)phenyl]amino}methylene]-2-oxo-*N*-[(1*R*)-1-phenylethyl]indoline-5-carboxamide |
| 199 | (3*Z*)-3-[(1-methyl-1*H*-indol-5-yl){[4-(pyrrolidin-1-ylmethyl)phenyl]amino}methylene]-2-oxo-*N*-[(1*R*)-1-phenylpropyl]indoline-5-carboxamide |
| 200 | (3*Z*)-3-[(1-methyl-1*H*-indol-5-yl){[4-(piperidin-1-ylmethyl)phenyl]amino}methylene]-2-oxo-*N*-[(1*R*)-1-phenylethyl]indoline-5-carboxamide |
| 201 | (3*Z*)-3-[(1-methyl-1*H*-indol-5-yl){[4-(piperidin-1-ylmethyl)phenyl]amino}methylene]-2-oxo-*N*-[(1*R*)-1-phenylpropyl]indoline-5-carboxamide |
| 202 | (3*Z*)-3-[(1-methyl-1*H*-indol-5-yl){[3-(pyrrolidin-1-ylmethyl)phenyl]amino}methylene]-2-oxo-*N*-[(1*R*)-1-phenylethyl]indoline-5-carboxamide |
| 203 | (3*Z*)-3-[(1-methyl-1*H*-indol-5-yl){[3-(pyrrolidin-1-ylmethyl)phenyl]amino}methylene]-2-oxo-*N*-[(1*R*)-1-phenylpropyl]indoline-5-carboxamide |
| 204 | (3*Z*)-3-[(1-methyl-1*H*-indol-5-yl){[3-(piperidin-1-ylmethyl)phenyl]amino}methylene]-2-oxo-*N*-[(1*R*)-1-phenylethyl]indoline-5-carboxamide |
| 205 | (3*Z*)-3-[(1-methyl-1*H*-indol-5-yl){[3-(piperidin-1-ylmethyl)phenyl]amino}methylene]-2-oxo-*N*-[(1*R*)-1-phenylpropyl]indoline-5-carboxamide |
| 206 | (3*Z*)-3-(cyclopropyl{[4-(pyrrolidin-1-ylmethyl)phenyl]amino}methylene)-2-oxo-*N*-[(1*R*)-1-phenylethyl]indoline-5-carboxamide |
| 207 | (3*Z*)-3-(cyclopropyl{[4-(pyrrolidin-1-ylmethyl)phenyl]amino}methylene)-2-oxo-*N*-[(1*R*)-1-phenylpropyl]indoline-5-carboxamide |
| 208 | (3*Z*)-3-(cyclopropyl{[4-(piperidin-1-ylmethyl)phenyl]amino}methylene)-2-oxo-*N*-[(1*R*)-1-phenylethyl]indoline-5-carboxamide |
| 209 | (3*Z*)-3-(cyclopropyl{[4-(piperidin-1-ylmethyl)phenyl]amino}methylene)-2-oxo-*N*-[(1*R*)-1-phenylpropyl]indoline-5-carboxamide |
| 210 | (3*Z*)-3-(cyclohexyl{[4-(piperidin-1-ylmethyl)phenyl]amino}methylene)-2-oxo-*N*-[(1*R*)-1-phenylethyl]indoline-5-carboxamide |
| 211 | (3*Z*)-3-(cyclohexyl{[4-(piperidin-1-ylmethyl)phenyl]amino}methylene)-2-oxo-*N*-[(1*R*)-1-phenylpropyl]indoline-5-carboxamide |
| 212 | (3*Z*)-2-oxo-*N*-[(1*R*)-1-phenylethyl]-3-(1-{[4-(piperidin-1-ylmethyl)phenyl]amino}propylidene)indoline-5-carboxamide |
| 213 | (3*Z*)-2-oxo-*N*-[(1*R*)-1-phenylpropyl]-3-(1-{[4-(piperidin-1-ylmethyl)phenyl]amino}propylidene)indoline-5-carboxamide |
| 214 | (3*Z*)-3-(2-methyl-1-{[4-(piperidin-1-ylmethyl)phenyl]amino}propylidene)-2-oxo-*N*-[(1*R*)-1-phenylethyl]indoline-5-carboxamide |
| 215 | (3*Z*)-3-(2-methyl-1-{[4-(piperidin-1-ylmethyl)phenyl]amino}propylidene)-2-oxo-*N*-[(1*R*)-1-phenylpropyl]indoline-5-carboxamide |
| 216 | (3*Z*)-3-[(4-fluorophenyl){[3-(piperidin-1-ylmethyl)phenyl]amino}methylene]-2-oxo-*N*-[(1*R*)-1-phenylethyl]indoline-5-carboxamide |
| 217 | (3*Z*)-3-[(4-fluorophenyl){[3-(piperidin-1-ylmethyl)phenyl]amino}methylene]-2-oxo-*N*-[(1*R*)-1-phenylpropyl]indoline-5-carboxamide |
| 218 | (3*Z*)-3-[(4-fluorophenyl){[3-(piperidin-1-ylmethyl)phenyl]amino}methylene]-*N*-[(1*R*)-1-(4-methoxyphenyl)ethyl]-2-oxoindoline-5-carboxamide |
| 219 | (3*Z*)-3-[(4-fluorophenyl){[3-(piperidin-1-ylmethyl)phenyl]amino}methylene]-*N*-[(1*R*)-1-(3-methoxyphenyl)ethyl]-2-oxoindoline-5-carboxamide |
| 220 | (3*Z*)-*N*-[(1*R*)-1-(3-chlorophenyl)ethyl]-3-[(4-fluorophenyl){[3-(piperidin-1-ylmethyl)phenyl]amino}methylene]-2-oxoindoline-5-carboxamide |
| 221 | (3*Z*)-*N*-[(1*R*)-1-(4-chlorophenyl)ethyl]-3-[(4-fluorophenyl){[3-(piperidin-1-ylmethyl)phenyl]amino}methylene]-2-oxoindoline-5-carboxamide |
| 222 | (3*Z*)-3-[(4-fluorophenyl){[3-(piperidin-1-ylmethyl)phenyl]amino}methylene]-*N*-[(1*R*)-1-(4-methylphenyl)ethyl]-2-oxoindoline-5-carboxamide |
| 223 | (3*Z*)-*N*-[1-(3,4-difluorophenyl)propyl]-3-[(4-fluorophenyl){[3-(piperidin-1-ylmethyl)phenyl]amino}methylene]-2-oxoindoline-5-carboxamide |
| 224 | (3*Z*)-*N*-[1-(3-chlorophenyl)propyl]-3-[(4-fluorophenyl){[3-(piperidin-1-ylmethyl)phenyl]amino}methylene]-2-oxoindoline-5-carboxamide |
| 225 | (3*Z*)-3-[({2-[(ethylcarbamoyl)amino]phenyl}amino)(phenyl)methylene]-2-oxo-*N*-[(1*R*)-1-phenylethyl]indoline-5-carboxamide |
| 226 | (3*Z*)-*N*-benzyl-3-{[(2-{[(ethylamino)carbonothioyl]amino}phenyl)amino] (phenyl)methylene}-2-oxoindoline-5-carboxamide |
| 227 | (3*Z*)-3-{[(2-{[(4-hydroxyphenyl)carbamoyl]amino}phenyl)amino](phenyl) methylene}-2-oxo-*N*-[(1*R*)-1-phenylpropyl]indoline-5-carboxamide |
| 228 | (3*Z*)-3-{[(2-{[(4-hydroxyphenyl)carbamoyl]amino}phenyl)amino](phenyl) methylene}-2-oxo-*N*-[(1*R*)-1-phenylpropyl]indoline-5-carboxamide |
| 229 | (3Z)-3-{[(2-{[(4-hydroxyphenyl)carbamoyl]amino}phenyl)amino](phenyl)methylene}-2-oxo-*N*-[(1*S*)-1-phenylpropyl]indoline-5-carboxamide |
| 230 | (3*Z*)-3-[{[2-(2,5-dioxoimidazolidin-1-yl)phenyl]amino}(phenyl)methylene]-2-oxo-*N*-[(1*R*)-1-phenylethyl]indoline-5-carboxamide |
| 231 | (3*Z*)-3-[({2-[(4*Z*)-2,5-dioxo-4-(pyridin-4-ylmethylene)imidazolidin-1-yl]phenyl}amino)(phenyl)methylene]-2-oxo-*N*-[(1*R*)-1-phenylethyl]indoline-5-carboxamide |
| 232 | (3*Z*)-3-[({3-[(ethylcarbamoyl)amino]phenyl}amino)(phenyl)methylene]-2-oxo-*N*-[(1*R*)-1-phenylpropyl]indoline-5-carboxamide |
| 233 | (3Z)-3-{[(3-{[(3-chlorophenyl)carbamoyl]amino}phenyl)amino](phenyl)methylene}-2-oxo-*N*-[(1*R*)-1-phenylpropyl]indoline-5-carboxamide |
| 234 | (3Z)-3-{[(3-{[(ethylamino)carbonothioyl]amino}phenyl)amino](phenyl)methylene}-2-oxo-*N*-[(1*S*)-1-phenylethyl]indoline-5-carboxamide |
| 235 | (3Z)-3-{[(3-{[(4-hydroxyphenyl)carbamoyl]amino}phenyl)amino](phenyl)methylene}-2-oxo-*N*-[(1*R*)-1-phenylpropyl]indoline-5-carboxamide |
| 236 | (3*Z*)-3-[{[3-({[4-(2,5-dioxoimidazolidin-1-yl)phenyl]carbamoyl}amino)phenyl]amino} (phenyl)methylene]-2-oxo-*N*-[(1*S*)-1-phenylethyl]indoline-5-carboxamide |
| 237 | (3*Z*)-3-[({3-[({4-[(4*Z*)-2,5-dioxo-4-(pyridin-3-ylmethylene)imidazolidin-1-yl]phenyl}carbamoyl)amino]phenyl}amino)(phenyl)methylene]-2-oxo-*N-*[(1*S*)-1-phenylethyl]indoline-5-carboxamide |
| 238 | (3Z)-3-{[(4-{[(ethylamino)carbonothioyl]amino}phenyl)amino](phenyl)methylene}-2-oxo-*N*-[(1*S*)-1-phenylpropyl]indoline-5-carboxamide |
| 239 | (3Z)-3-{[(4-{[(3-chlorophenyl)carbamoyl]amino}phenyl)amino](phenyl)methylene}-2-oxo-*N*-[(1*R*)-1-phenylpropyl]indoline-5-carboxamide |
| 240 | (3*Z*)-3-{[(4-{[(4-hydroxyphenyl)carbamoyl]amino}phenyl)amino](phenyl) methylene}-2-oxo-*N*-[(1*R*)-1-phenylpropyl]indoline-5-carboxamide |
| 241 | (3*Z*)-3-[{[4-(2,5-dioxoimidazolidin-1-yl)phenyl]amino}(pyridin-4-yl)methylene]-2-oxo-*N*-[(1*R*)-1-phenylethyl]indoline-5-carboxamide |
| 242 | (3*Z*)-3-[({4-[(4*Z*)-4-benzylidene-2,5-dioxoimidazolidin-1-yl]phenyl}amino)(pyridin-4-yl)methylene]-2-oxo-*N*-[(1*R*)-1-phenylethyl]indoline-5-carboxamide |
| 243 | (3*Z*)-3-{[(2-{[(ethylamino)carbonothioyl]amino}benzyl)amino](3-thienyl)methylene}-2-oxo-*N*-[(1*R*)-1-phenylethyl]indoline-5-carboxamide |
| 244 | (3*Z*)-3-[({2-[(1,3-benzodioxol-5-ylcarbamoyl)amino]benzyl}amino)(phenyl)methylene]-2-oxo-*N*-[(1*R*)-1-phenylpropyl]indoline-5-carboxamide |
| 245 | (3*Z*)-3-[({3-[(ethylcarbamoyl)amino]benzyl}amino)(pyridin-3-yl)methylene]-2-oxo-*N*-[(1*R*)-1-phenylethyl]indoline-5-carboxamide |
| 246 | (3*Z*)-3-{[(3-{[(3-chlorophenyl)carbamoyl]amino}benzyl)amino](pyridin-3-yl)methylene}-2-oxo-*N*-[(1*R*)-1-phenylethyl]indoline-5-carboxamide |
| 247 | (3*Z*)-3-[{[3-(2,5-dioxoimidazolidin-1-yl)benzyl]amino}(pyridin-3-yl)methylene]-2-oxo-*N*-[(1*R*)-1-phenylethyl]indoline-5-carboxamide |
| 248 | (3*Z*)-3-[({3-[(4*Z*)-2,5-dioxo-4-(pyridin-4-ylmethylene)imidazolidin-1-yl]benzyl}amino)(pyridin-3-yl)methylene]-2-oxo-*N*-[(1*R*)-1-phenylethyl]indoline-5-carboxamide |
| 249 | (3*Z*)-*N*-benzyl-3-[{[4-({[(3-chlorophenyl)amino]carbonothioyl}amino)benzyl]amino} (phenyl)methylene]-2-oxoindoline-5-carboxamide |
| 250 | (3*Z*)-*N*-benzyl-3-[{[4-(2,5-dioxoimidazolidin-1-y)benzyl]amino}(phenyl)methylene]-2-oxoindoline-5-carboxamide |
| 251 | (3*Z*)-3-[{[4-(2,5-dioxoimidazolidin-1-yl)benzyl]amino}(phenyl)methylene]-2-oxo-*N*-[(1*R*)-1-phenylethyl]indoline-6-carboxamide |
| 252 | (3*Z*)-3-[({4-[(4*Z*)-2,5-dioxo-4-(pyridin-4-ylmethylene)imidazolidin-1-yl]benzyl}amino)(phenyl)methylene]-2-oxo-*N*-[(1*R*)-1-phenylethyl]indoline-6-carboxamide |
| 253 | (3*Z*)-*N*-benzyl-3-[({4-[(4*Z*)-2,5-dioxo-4-(pyridin-4-ylmethylene)imidazolidin-1-yl]benzyl}amino)(phenyl)methylene]-2-oxoindoline-5-carboxamide |
| 254 | (3Z)-3-{[(2-{2-[(ethylcarbamoyl)amino]phenyl}ethyl)amino](phenyl)methylene}-2-oxo-*N-*[(1*R*)-1-phenylpropyl]indoline-7-carboxamide |
| 255 | (3Z)-3-[{[2-(2-{[(3-chlorophenyl)carbamoyl]amino}phenyl)ethyl]amino}(pyridin-4-yl)methylene]-2-oxo-*N*-[(1*S*)-1-phenylpropyl]indoline-7-carboxamide |
| 256 | (3Z)-3-{[(2-{3-[(ethylcarbamoyl)amino]phenyl}ethyl)amino](phenyl)methylene}-2-oxo-*N-*[(1*R*)-1-phenylethyl]indoline-5-carboxamide |
| 257 | (3Z)-3-{[(2-{3-[(ethylcarbamoyl)amino]phenyl}ethyl)amino](phenyl)methylene}-2-oxo-*N-*[(1*S*)-1-phenylethyl]indoline-5-carboxamide |
| 258 | (3Z)-3-[{[2-(3-{[(3-chlorophenyl)carbamoyl]amino}phenyl)ethyl]amino}(phenyl)methylene]-2-oxo-*N*-[(1*R*)-1-phenylethyl]indoline-5-carboxamide |
| 259 | (3*Z*)-*N*-benzyl-3-{[(2-{3-[(4Z)-2,5-dioxo-4-(pyridin-4-ylmethylene)imidazolidin-1-yl]phenyl}ethyl)amino](phenyl)methylene}-2-oxoindoline-5-carboxamide |
| 260 | (3Z)-3-{[(2-{4-[(ethylcarbamoyl)amino]phenyl}ethyl)amino](phenyl)methylene}-2-oxo-*N-*[(1*R*)-1-phenylethyl]indoline-5-carboxamide |
| 261 | (3*Z*)-3-[{[2-(4-{[(3-chlorophenyl)carbamoyl]amino}phenyl)ethyl]amino} (phenyl)methylene]-2-oxo-*N*-[(1*R*)-1-phenylethyl]indoline-5-carboxamide |
| 262 | (3*Z*)-3-{[(3*S*)-1-azabicyclo[2.2.2]oct-3-ylamino](phenyl)methylene}-2-oxo-*N*-[(1*R*)-1-phenylethyl]indoline-5-carboxamide |
| 263 | (3*Z*)-3-{[(3*R*)-1-azabicyclo[2.2.2]oct-3-ylamino](phenyl)methylene}-2-oxo-*N*-[(1*R*)-1-phenylethyl]indoline-5-carboxamide |
| 264 | (3*Z*)-3-{[(3*R*)-1-azabicyclo[2.2.2]oct-3-ylamino](phenyl)methylene}-*N-*benzyl-2-oxoindoline-5-carboxamide |
| 265 | (3*Z*)-3-{[(3*S*)-1-azabicyclo[2.2.2]oct-3-ylamino](phenyl)methylene}-2-oxo-*N*-[(1*R*)-1-phenylethyl]indoline-5-carboxamide |
| 266 | (3*Z*)-3-{[(3*R*)-1-azabicyclo[2.2.2]oct-3-ylamino](phenyl)methylene}-2-oxo-*N*-[(1*R*)-1-phenylethyl]indoline-5-carboxamide |
| 267 | (3*Z*)-3-{[(3*S*)-1-azabicyclo[2.2.2]oct-3-ylamino](phenyl)methylene}-2-oxo-*N*-[(1*R*)-1-phenylpropyl]indoline-6-carboxamide |
| 268 | (3*Z*)-3-{[(3*R*)-1-azabicyclo[2.2.2]oct-3-ylamino](pyridin-4-yl)methylene}-2-oxo-*N*-[(1*R*)-1-phenylpropyl]indoline-6-carboxamide and |
| 269 | (3*Z*)-3-{[(3*R*)-1-azabicyclo[2.2.2]oct-3-ylamino](3-thienyl)methylene}-*N-*benzyl-2-oxoindoline-6-carboxamide. |

8. The compound for use according to anyone of claims 1 - 7, wherein the infectious diseases, are virally induced infectious diseases, including opportunistic diseases.

9. The compound for use according to claim 8, wherein the virally induced infectious diseases, including opportunistic diseases, are infections with dsDNA viruses, ssDNA viruses, (+)ssRNA viruses, (-)ssRNA viruses, ssRNA-RT viruses, or dsDNA-RT viruses.

10. The compound for use according to claim 9, wherein the infection with (-)ssRNA viruses are an infection with Orthomyxoviridae, Bornaviridae, Filoviridae, Paramyxoviridae, or Rhabdoviridae.

11. The compound for use according to claim 10, wherein the infection with Orthomyxoviridae is an infection with Influenza virus A, Influenza virus B, or Influenza virus C.

12. The compound for use according to anyone of claims 1 - 7, wherein cancer is selected from the group consisting of adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer, breast cancer, Burkitt's lymphoma, corpus cancer, colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors, brain metastases, testicle cancer, hypophysis tumor, carcinoids, Kaposi's sarcoma, laryngeal cancer, germ cell tumor, bone cancer, colorectal carcinoma, head and neck tumors, colon carcinoma, craniopharyngiomas, oral cancer, cancer of the central nervous system, liver cancer, liver metastases, leukemia, eyelid tumor, lung cancer, lymph node cancer, lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, nasal cancer, neurinoma, neuroblastoma, kidney cancer, renal cell carcinomas, non-Hodgkin's lymphomas, oligodendroglioma, esophageal carcinoma, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, esophageal cancer, spinalioms, T-cell lymphoma, thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer.

13. The compound for use according to claim 12, wherein the cancer is a colorectal cancer.

14. A pharmaceutical compositions comprising at least one compound according to general formula (I) as an active ingredient, together with at least one pharmaceutically acceptable carrier, excipient and/or diluents for the use in the treatment of an infectious diseases or cancer.
